# EUROPEAN PATENT SPECIFICATION

(11) **EP 4 333 985 B1**
(45) Date of publication and mention of the grant of the patent: **30.07.2025**
(21) Application number: 22729327.1
(22) Date of filing: 04.05.2022
(51) Int. Cl.: A61P 35/00, C07D 413/12, A61K 31/513

(54) **MODULATORS OF TREX1**
TREX1-MODULATOREN
MODULATEURS DE TREX1

(30) Priority: 05.05.2021 US 202163184460 P
(43) Date of publication of application: 13.03.2024
(73) Proprietor: Constellation Pharmaceuticals, Inc., Boston, MA 02210 (US)
(72) Inventor: LEVELL, Julian R., Arlington, MA 02474 (US); COFFIN, Aaron, Everett, MA 02149 (US); KHANNA, Avinash, Cambridge, MA 02141 (US); WILSON, Jonathan E., Arlington, MA 02474 (US)
(74) Representative: Potter Clarkson
(86) International application number: PCT/US2022/027571
(87) International publication number: WO 2022/235725

(56) References cited:
- WO-A1-2012/151567
- WO-A1-2014/023691
- WO-A1-2020/118133
- WO-A1-2021/016317

## Description

### RELATED APPLICATIONS

This application claims the benefit of priority to U.S. Provisional Application No 63/184,460, filed May 5, 2021.

### BACKGROUND

A potential immune therapy is needed for cancers related to the innate immune system recognition of non-self, and to detect and protect against potential danger. Cancer cells differ antigenically from their normal counterparts and emit danger signals to alert the immune system similar to viral infection. These signals, which include damage-associated molecular patterns (DAMPs) and pathogen-associated molecular patterns (PAMPs), further activate the innate immune system resulting in the protection of the host from a variety of threats (Front. Cell Infect. Microbiol. 2012, 2, 168).

Ectopically expressed single stranded DNA (ssDNA) and double stranded DNA (dsDNA) are known PAMPs and/or DAMPs, which are being recognized by the cyclic GMP-AMP synthase (cGAS), a nucleic acid sensor (Nature 2011, 478, 515-518). Upon sensing of cytosolic DNA, cGAS catalyzes the generation of the cyclic dinucleotide 2',3'-cGAMP, a potent second messenger and activator of the ER transmembrane adapter protein stimulator of interferon genes (STING) (Cell Rep. 2013, 3, 1355-1361). STING activation triggers phosphorylation of IRF3 via TBK1 which in turn leads to type I interferon production and activation of interferon stimulated genes (ISGs); a pre-requisite to the activation of innate immunity and initiation of adaptive immunity. Production of type I interferons thus constitutes a key bridge between the innate and adaptive immunity (Science 2013, 341, 903-906).

Excess type I IFN can be harmful to the host and induce autoimmunity, therefore, negative feedback mechanisms exist that keep type I IFN-mediated immune activation in check. Three prime repair exonuclease I (TREX1) is a 3'-5' DNA exonuclease responsible for the removal of ectopically expressed ssDNA and dsDNA and is therefore a key repressor of the cGAS/STING pathway (PNAS 2015, 112, 5117-5122).

Type I interferons and downstream pro-inflammatory cytokine responses are critical to the development of immune responses and their effectiveness. Type I interferons enhance both the ability of dendritic cells and macrophages to take up, process, present, and cross-present antigens to T cells, and their potency to stimulate T cells by eliciting the upregulation of the co-stimulatory molecules such as CD40, CD80 and CD86 (J. Exp. Med. 2011, 208, 2005-2016). Type I interferons also bind their own receptors and activate interferon responsive genes that contribute to activation of cells involved in adaptive immunity (EMBO Rep. 2015, 16, 202-212).

From a therapeutic perspective, type I interferons and compounds that can induce type I interferon production have potential for use in the treatment of human cancers (Nat. Rev Immunol. 2015, 15, 405-414). Interferons can inhibit human tumor cell proliferation directly. In addition, type I interferons can enhance anti-tumor immunity by triggering the activation of cells from both the innate and adaptive immune system. Importantly, the anti-tumor activity of PD-1 blockade requires pre-existing intratumoral T cells. By turning cold tumors into hot and thereby eliciting a spontaneous anti-tumor immunity, type I IFN-inducing therapies have the potential to expand the pool of patients responding to anti-PD-1 therapy as well as enhance the effectiveness of anti-PD1 therapy.

Human and mouse genetic studies suggest that TREX1 inhibition might be amenable to a systemic delivery route and therefore TREX1 inhibitory compounds could play an important role in the anti-tumor therapy landscape. TREX1 is a key determinant for the limited immunogenicity of cancer cells responding to radiation treatment [Trends in Cell Biol., 2017, 27 (8), 543-4; Nature Commun., 2017, 8, 15618]. TREX1 is induced by genotoxic stress and involved in protection of glioma and melanoma cells to anticancer drugs [Biochim. Biophys. Acta, 2013, 1833, 1832-43]. STACT-TREX1 therapy shows robust anti-tumor efficacy in multiple murine cancer models [Glickman et al, Poster P235, 33rd Annual Meeting of Society for Immunotherapy of Cancer, Washington DC, Nov. 7-11, 2018**].** (TREX1) expression correlates with cervical cancer cells growth in vitro and disease progression in vivo [*Scientific Reports* 1019, 9, 351]. Beyond oncology there is also support for agonists of the IFN pathway to be useful in antiviral therapy, for example STING agonists induce an innate antiviral immune response against Hepatitis B Virus via stimulation of the IFN pathway and upregulation of ISG's [Antimicrob. Agents Chemother. 2015, 59:1273-1281] and TREX1 inhibits the innate immune response to HIV type 1 [Nature Immunology, 2010, 11(11), 1005]. WO 2020/118133 refers to compounds and pharmaceutically acceptable salts and compositions thereof, which are useful for treating a variety of conditions associated with TREX1.

### SUMMARY

The invention is set out in the appended set of claims. In addition, any reference to methods of treatment of a subject in the subsequent paragraphs of this description is to be interpreted as references to the compounds, pharmaceutical compositions and/or medicaments of the present invention for use in treatment of the subject. Provided herein are compounds having the Formula **I**: 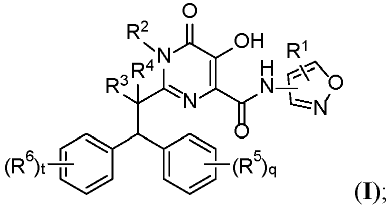 and pharmaceutically acceptable salts and compositions thereof, wherein R¹, R², R³, R⁴, R⁵, R⁶, q and t are as described herein. The disclosed compounds and compositions modulate TREX1, and are useful in a variety of therapeutic applications such as, for example, in treating cancer.

In one aspect, substitution at R³ and/or R⁴ in combination with the introduction of one or more halo or cyano groups at R⁵ and/or R⁶ seemed to have a profound effect on the resulting kinetic properties. See e.g., **Table 9.**

### DETAILED DESCRIPTION

### 1. General Description of Compounds

In a first embodiment, provided herein is a compound of Formula **I**: 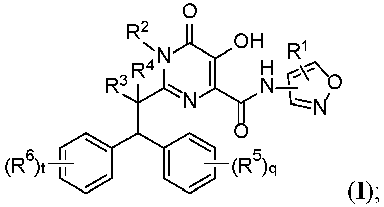 or a pharmaceutically acceptable salt thereof, wherein:
R¹ is halo, hydrogen, (C₁-C₄)alkyl, or halo(C₁-C₄)alkyl;
R² is hydrogen, (C₁-C₄)alkyl, halo(C₁-C₄)alkyl, -(C₁-C₄)alkylOR^{a}, -(C₁-C₄)alkylSR^{a}, -(C₁-C₄)alkylNR^{a}R^{b},
R^{a} and R^{b} are each independently selected from hydrogen, (C₁-C₄)alkyl, halo(C₁-C₄)alkyl, COOR^{c}, and -C(O)NR^{c}R^{d};
R³ and R⁴ are each independently hydrogen, halo, (C₁-C₄)alkyl, or halo(C₁-C₄)alkyl;
R⁵ and R⁶ are each independently (C₁-C₄)alkyl, halo(C₁-C₄)alkyl, halo(C₁-C₄)alkoxy, halo, cyano, or -C(O)NR^{c}R^{d};
R^{c} and R^{d} are each independently hydrogen or (C₁-C₄)alkyl;

t is 1, 2, or 3, and
q is 0, 1, 2, or 3.

### 2. Definitions

When used in connection to describe a chemical group that may have multiple points of attachment, a hyphen (-) designates the point of attachment of that group to the variable to which it is defined. For example, -C(O)NR^{c}R^{d} means that the point of attachment for this group occurs on the carbonyl carbon atom.

The terms "halo" and "halogen" refer to an atom selected from fluorine (fluoro, -F), chlorine (chloro, -Cl), bromine (bromo, -Br), and iodine (iodo, -I).

The term "alkyl" when used alone or as part of a larger moiety, such as "haloalkyl", and the like, means saturated straight-chain or branched monovalent hydrocarbon radical. Unless otherwise specified, an alkyl group typically has 1-4 carbon atoms, *i.e.,* (C₁-C₄)alkyl.

"Alkoxy" means an alkyl radical attached through an oxygen linking atom, represented by -O-alkyl. For example, "(C₁-C₄)alkoxy" includes methoxy, ethoxy, proproxy, and butoxy.

The term "haloalkyl" includes mono, poly, and perhaloalkyl groups where the halogens are independently selected from fluorine, chlorine, bromine, and iodine.

"Haloalkoxy" is a haloalkyl group which is attached to another moiety via an oxygen atom such as, e.g., -OCHF₂ or -OCF₃.

The disclosed compounds exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that contain two or more asymmetrically substituted carbon atoms. "R" and "S" represent the configuration of substituents around one or more chiral carbon atoms.

"Racemate" or "racemic mixture" means a compound of equimolar quantities of two enantiomers, wherein such mixtures exhibit no optical activity, *i.e.,* they do not rotate the plane of polarized light.

When the stereochemistry of a disclosed compound is named or depicted by structure, the named or depicted stereoisomer is at least 60%, 70%, 80%, 90%, 99% or 99.9% by weight pure relative to all of the other stereoisomers. Percent by weight pure relative to all of the other stereoisomers is the ratio of the weight of one stereoisomer over the weight of the other stereoisomers. When a single enantiomer is named or depicted by structure, the depicted or named enantiomer is at least 60%, 70%, 80%, 90%, 99% or 99.9% by weight optically pure. Percent optical purity by weight is the ratio of the weight of the enantiomer over the weight of the enantiomer plus the weight of its optical isomer.

When the stereochemistry of a disclosed compound is named or depicted by structure, and the named or depicted structure encompasses more than one stereoisomer (e.g., as in a diastereomeric pair), it is to be understood that one of the encompassed stereoisomers or any mixture of the encompassed stereoisomers are included. It is to be further understood that the stereoisomeric purity of the named or depicted stereoisomer is at least 60%, 70%, 80%, 90%, 99% or 99.9% by weight pure relative to all of the other stereoisomers. The stereoisomeric purity in this case is determined by dividing the total weight in the mixture of the stereoisomers encompassed by the name or structure by the total weight in the mixture of all of the stereoisomers.

When a disclosed compound is named or depicted by structure without indicating the stereochemistry, and the compound has one chiral center, it is to be understood that the name or structure encompasses one enantiomer of compound free from the corresponding optical isomer, a racemic mixture of the compound, or mixtures enriched in one enantiomer relative to its corresponding optical isomer.

When a disclosed compound is named or depicted by structure without indicating the stereochemistry and *e.g.,* the compound has more than one chiral center (e.g., at least two chiral centers), it is to be understood that the name or structure encompasses one stereoisomer free of other stereoisomers, mixtures of stereoisomers, or mixtures of stereoisomers in which one or more stereoisomers is enriched relative to the other stereoisomer(s). For example, the name or structure may encompass one stereoisomer free of other diastereomers, mixtures of stereoisomers, or mixtures of stereoisomers in which one or more diastereomers is enriched relative to the other diastereomer(s).

The term "TREX1" refers to three prime repair exonuclease 1 or DNA repair exonuclease 1, which is an enzyme that in humans is encoded by the TREX1 gene. Mazur DJ, Perrino FW (Aug 1999). "Identification and expression of the TREX1 and TREX2 cDNA sequences encoding mammalian 3'-->5' exonucleases". J Biol Chem. 274 (28): 19655-60. doi:10.1074/jbc.274.28.19655. PMID 10391904; Hoss M, Robins P, Naven TJ, Pappin DJ, Sgouros J, Lindahl T (Aug 1999). "A human DNA editing enzyme homologous to the Escherichia coli DnaQ/MutD protein". EMBO J. 18 (13): 3868-75. doi:10.1093/emboj/18.13.3868. PMC 1171463. PMID 10393201. This gene encodes the major 3'->5' DNA exonuclease in human cells. The protein is a non-processive exonuclease that may serve a proofreading function for a human DNA polymerase. It is also a component of the SET complex, and acts to rapidly degrade 3' ends of nicked DNA during granzyme A-mediated cell death. Cells lacking functional TREX1 show chronic DNA damage checkpoint activation and extra-nuclear accumulation of an endogenous single-strand DNA substrate. It appears that TREX1 protein normally acts on a single-stranded DNA polynucleotide species generated from processing aberrant replication intermediates. This action of TREX1 attenuates DNA damage checkpoint signaling and prevents pathological immune activation. TREX1 metabolizes reverse-transcribed single-stranded DNA of endogenous retroelements as a function of cell-intrinsic antiviral surveillance, resulting in a potent type I IFN response. TREX1 helps HIV-1 to evade cytosolic sensing by degrading viral cDNA in the cytoplasm.

The term "TREX2" refers to Three prime repair exonuclease 2 is an enzyme that in humans is encoded by the TREX2 gene. This gene encodes a nuclear protein with 3' to 5' exonuclease activity. The encoded protein participates in double-stranded DNA break repair, and may interact with DNA polymerase delta. Enzymes with this activity are involved in DNA replication, repair, and recombination. TREX2 is a 3'-exonuclease which is predominantly expressed in keratinocytes and contributes to the epidermal response to UVB-induced DNA damage. TREX2 biochemical and structural properties are similar to TREX1, although they are not identical. The two proteins share a dimeric structure and can process ssDNA and dsDNA substrates *in vitro* with almost identical k_{cat} values. However, several features related to enzyme kinetics, structural domains, and subcellular distribution distinguish TREX2 from TREX1. TREX2 present a 10-fold lower affinity for DNA substrates *in vitro* compared with TREX1. In contrast with TREX1, TREX2 lacks a COOH-terminal domain that can mediate protein-protein interactions. TREX2 is localized in both the cytoplasm and nucleus , whereas TREX1 is found in the endoplasmic reticulum, and is mobilized to the nucleus during granzyme A-mediated cell death or after DNA damage.

The terms "subject" and "patient" may be used interchangeably, and means a mammal in need of treatment, *e.g.,* companion animals (*e.g.,* dogs, cats, and the like), farm animals (*e.g.,* cows, pigs, horses, sheep, goats and the like) and laboratory animals (*e.g.,* rats, mice, guinea pigs and the like). Typically, the subject is a human in need of treatment.

The term "inhibit," "inhibition" or "inhibiting" includes a decrease in the baseline activity of a biological activity or process.

As used herein, the terms "treatment," "treat," and "treating" refer to reversing, alleviating, delaying the onset of, or inhibiting the progress of a disease or disorder, or one or more symptoms thereof, as described herein. In some aspects, treatment may be administered after one or more symptoms have developed, *i.e.,* therapeutic treatment. In other aspects, treatment may be administered in the absence of symptoms. For example, treatment may be administered to a susceptible individual prior to the onset of symptoms (e.g., in light of a history of symptoms and/or in light of exposure to a particular organism, or other susceptibility factors), *i.e.,* prophylactic treatment. Treatment may also be continued after symptoms have resolved, for example to delay their recurrence.

The term "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

For use in medicines, the salts of the compounds described herein refer to non-toxic "pharmaceutically acceptable salts." Pharmaceutically acceptable salt forms include pharmaceutically acceptable acidic/anionic or basic/cationic salts. Suitable pharmaceutically acceptable acid addition salts of the compounds described herein include e.g., salts of inorganic acids (such as hydrochloric acid, hydrobromic, phosphoric, nitric, and sulfuric acids) and of organic acids (such as, acetic acid, benzenesulfonic, benzoic, methanesulfonic, and p-toluenesulfonic acids). Compounds of the present teachings with acidic groups such as carboxylic acids can form pharmaceutically acceptable salts with pharmaceutically acceptable base(s). Suitable pharmaceutically acceptable basic salts include e.g., ammonium salts, alkali metal salts (such as sodium and potassium salts) and alkaline earth metal salts (such as magnesium and calcium salts). Compounds with a quaternary ammonium group also contain a counteranion such as chloride, bromide, iodide, acetate, perchlorate and the like. Other examples of such salts include hydrochlorides, hydrobromides, sulfates, methanesulfonates, nitrates, benzoates and salts with amino acids such as glutamic acid.

The term "effective amount" or "therapeutically effective amount" refers to an amount of a compound described herein that will elicit a desired or beneficial biological or medical response of a subject e.g., a dosage of between 0.01 - 100 mg/kg body weight/day.

### 3. Compounds

In a second embodiment, provided are compounds of Formula **I,** or a pharmaceutically acceptable salt thereof, wherein when R³ is halo then R⁴ is halo, wherein the remaining variables are as described above for Formula **I.** Alternatively, as part of a second embodiment, provided are compounds of Formula **I,** or a pharmaceutically acceptable salt thereof, wherein when R³ is hydrogen then R⁴ is (C₁-C₄)alkyl or halo(C₁-C₄)alkyl, wherein the remaining variables are as described above for Formula **I.** In another alternative, as part of a second embodiment, provided are compounds of Formula **I,** or a pharmaceutically acceptable salt thereof, wherein when R³ is hydrogen then R⁴ is (C₁-C₄)alkyl or halo(C₁-C₄)alkyl, wherein the remaining variables are as described above for Formula **I.** provided are compounds of Formula **I,** or pharmaceutically acceptable salt thereof, wherein when R³ is halo then R⁴ is halo, and wherein when R³ is hydrogen then R⁴ is (C₁-C₄)alkyl or halo(C₁-C₄)alkyl, wherein the remaining variables are as described above for Formula **I.**

In a third embodiment, the compound of Formula **I** is of the Formula **II**: 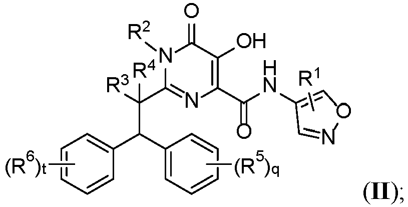 or a pharmaceutically acceptable salt thereof, wherein the remaining variables are as described above for Formula **I** or the second embodiment.

In a fourth embodiment, R¹ in the compound of Formula **I** or **II,** or a pharmaceutically acceptable salt thereof, is H, wherein the remaining variables are as described above for Formula **I** or the second embodiment.

In a fifth embodiment, R² in the compound of Formula **I** or **II,** or a pharmaceutically acceptable salt thereof, is (C₁-C₄)alkyl, wherein the remaining variables are as described above for Formula **I** or the second or fourth embodiment.

In a sixth embodiment, R³ in the compound of Formula **I** or **II,** or a pharmaceutically acceptable salt thereof, is halo, hydrogen or (C₁-C₄)alkyl, wherein the remaining variables are as described above for Formula **I** or the second, fourth, or fifth embodiment. Alternatively, as part of a sixth embodiment, R³ in the compound of Formula **I** or **II,** or a pharmaceutically acceptable salt thereof, is halo, wherein the remaining variables are as described above for Formula **I** or the second, fourth, or fifth embodiment. In another alternative, as part of a sixth embodiment, R³ in the compound of Formula **I** or **II,** or a pharmaceutically acceptable salt thereof, is hydrogen, wherein the remaining variables are as described above for Formula **I** or the second, fourth, or fifth embodiment.

In a seventh embodiment, R⁴ in the compound of Formula **I** or **II,** or a pharmaceutically acceptable salt thereof, is hydrogen, (C₁-C₄)alkyl, or halo(C₁-C₄)alkyl, wherein the remaining variables are as described above for Formula **I** or the second, fourth, fifth, or sixth embodiment. Alternatively, as part of a seventh embodiment, R⁴ in the compound of Formula **I** or **II**, or a pharmaceutically acceptable salt thereof, is (C₁-C₄)alkyl or halo(C₁-C₄)alkyl, wherein the remaining variables are as described above for Formula **I** or the second, fourth, fifth, or sixth embodiment. In another alternative, as part of a seventh embodiment, R⁴ in the compound of Formula **I** or **II**, or a pharmaceutically acceptable salt thereof, is halo, wherein the remaining variables are as described above for Formula **I** or the second, fourth, fifth, or sixth embodiment. In another alternative, as part of a seventh embodiment, R⁴ in the compound of Formula **I** or **II,** or a pharmaceutically acceptable salt thereof, is (C₁-C₄)alkyl, wherein the remaining variables are as described above for Formula **I** or the second, fourth, fifth, or sixth embodiment.

In an eighth embodiment, R⁵ in the compound of Formula **I** or **II**, or a pharmaceutically acceptable salt thereof, is halo, cyano, or -C(O)NR^{a}R^{b}, wherein the remaining variables are as described above for Formula **I** or the second, fourth, fifth, sixth, or seventh embodiment.

In a ninth embodiment, R⁶ in the compound of Formula **I** or **II,** or a pharmaceutically acceptable salt thereof, is halo, cyano, or -C(O)NR^{a}R^{b}, wherein the remaining variables are as described above for Formula **I** or the second, fourth, fifth, sixth, seventh, or eighth embodiment. Alternatively, as part of a ninth embodiment, R⁶ in the compound of Formula **I** or **II,** or a pharmaceutically acceptable salt thereof, is halo or cyano, wherein the remaining variables are as described above for Formula **I** or the second, fourth, fifth, sixth, seventh, or eighth embodiment.

Compounds having the Formula **I** are further disclosed in the Exemplification and are included in the present disclosure. Pharmaceutically acceptable salts thereof as well as the neutral forms are included.

### 4. Uses, Formulation and Administration

Compounds and compositions described herein are generally useful for modulating the activity of TREX1. In some aspects, the compounds and pharmaceutical compositions described herein inhibit the activity TREX1.

In some aspects, compounds and pharmaceutical compositions described herein are useful in treating a disorder associated with TREX1 function. Thus, provided herein are methods of treating a disorder associated with TREX1 function, comprising administering to a subject in need thereof, a therapeutically effective amount of a compound described herein, or a pharmaceutically acceptable salt thereof, or a pharmaceutical composition comprising a disclosed compound or pharmaceutically acceptable salt thereof. Also provided is the use of a compound described herein, or a pharmaceutically acceptable salt thereof, or a pharmaceutical composition comprising a disclosed compound or pharmaceutically acceptable salt thereof, for the manufacture of a medicament for treating a disorder associated with TREX1 function. Also provided is a compound described herein, or a pharmaceutically acceptable salt thereof, or a pharmaceutical composition comprising a disclosed compound or pharmaceutically acceptable salt thereof, for use in treating a disorder associated with TREX1.

In some aspects, the compounds and pharmaceutical compositions described herein are useful in treating cancer.

In some aspects, the cancer treated by the compounds and pharmaceutical compositions described herein is selected from colon cancer, gastric cancer, thyroid cancer, lung cancer, leukemia, pancreatic cancer, melanoma, multiple melanoma, brain cancer, CNS cancer, renal cancer, prostate cancer, ovarian cancer, leukemia, and breast cancer.

In some aspects, the cancer treated by the compounds and pharmaceutical compositions described herein is selected from lung cancer, breast cancer, pancreatic cancer, colorectal cancer, and melanoma.

In certain aspects, a pharmaceutical composition described herein is formulated for administration to a patient in need of such composition. Pharmaceutical compositions described herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. In some embodiments, the compositions are administered orally, intraperitoneally or intravenously. Sterile injectable forms of the pharmaceutical compositions described herein may be aqueous or oleaginous suspension. These suspensions may be formulated according to techniques known in the art using suitable dispersing or wetting agents and suspending agents.

In some aspects, the pharmaceutical compositions are administered orally.

A specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound described herein in the composition will also depend upon the particular compound in the pharmaceutical composition.

### EXEMPLIFICATION

The representative examples that follow are intended to help illustrate the present disclosure, and are not intended to, nor should they be construed to, limit the scope of the invention.

General starting materials used were obtained from commercial sources or prepared in other examples, unless otherwise noted.

The following abbreviations have the indicated meanings:
ACN Acetonitrile
Ag(Phen)₂OTf Silver(1+), bis(1,10-phenanthroline-κN1,κN10)-, (T-4)-, 1,1,1-trifluoromethanesulfonate
AIBN Azobisisobutyronitrile
BOC t-butyloxycarbonyl
CDI Carbonyldiimidazole
Cs₂CO₃ Cesium Carbonate
DBU 1,8-Diazabicycloundec-7-ene
DCC 1,3-dicyclohexylcarbodiimide
DEA Diethylamine
DCE 1,2-dichloroethane
DCM or CH₂Cl₂ Dichloromethane
DIAD Diisopropyl azodicarboxylate
DIBAL Diisobutyl aluminum hydride
DIPEA or DIEA N,N-diisoproylethylamine, also known as Hunig's base.
DMA N,N-dimethylacetamide
DMAD Dimethyl acetylenedicarboxylate
DMAP 4-(dimethylamino)pyridine
DMF N,N-dimethylformamide
DME 1,2-dimethoxyethane
DMP Dess-Martin periodinane
DMSO Dimethylsulfoxide
DPPA Diphenylphosphoryl azide
DPPP 1,3-bis(diphenylphosphino)propane
Dtbbpy 4,4 '-di-/e/7-butyl-2,2 ' -dipyridyl
EDC or EDCI1-(3-dimethylaminopropyl)-3-ethylcarbodiimide hydrochloride
EtOAc Ethyl acetate
EtOH Ethanol
FA Formic Acid
HATU(9-(7-Azabenzotriazol-l-yl)-N, N, N, N
tetramethyluroniumhexafluorophosphate
HCl Hydrochloric acid
HOAt 1-Hydroxy-7-azabenzotriazole or 3H-[1,2,3]triazolo[4,5-b]pyridin-3-ol
HOBt 1-hydroxybenzotriazole
IPA Isopropylamine
iPrMgCl isopropylmagnesium chloride
KHMDS Potassium hexamethyldisilazane
K₂CO₃ Potassium Carbonate
LDA Lithium diisopropylamide
LiBr Lithium Bromide
LiCl Lithium Chloride
LiHMDS or LHMDS Lithium hexamethyldisilazane
LiOH Lithium hydroxide
MCPBA meta-chloroperbenzoic acid
MeI or CH₃I Methyl Iodide
MeOH Methanol
MnO₂ Manganese(IV) oxide
MsO Methanefulfonate mesylate
MTBE Methyl *t*-butyl ether
n-BuLi n-butyllithium
Na₂CO₃ Sodium Carbonate
Na₂SO₄ Sodium Sulphate
NaH Sodium hydride
NaHMDS Sodium hexamethyldisilazane
NaOH Sodium Hydroxide
NBS N-bromosuccinimide
NH₄Cl Ammonium Chloride
NMM 4-methylmorpholine
NMP N-methylpyrroIidinone
PCC Pyridinium chlorochromate
PDC Pyridinium dichromate
Pd(dppf)Cl₂ [1,1'-Bis(diphenylphosphino)ferrocene] dichloropalladium (II)
Pd(dtbpf)Cl₂ [1,1'-Bis(di-tert-butylphosphino)ferrocene] dichloropalladium (II)
Pd(PPh₃)₄ Tetrakis(triphenylphosphine) palladium (0)
SPhos Pd 3G (2-Dicyclohexylphosphino-2',6'-dimethoxybiphenyl) [2-(2'-amino-1,1'biphenyl)]palladium (II) methanesulfonate
T₃P 2,4,6-Tripropyl-1,3,5,2,4,6-trioxatriphosphinane 2,4,6-trioxide
TEA Triethylamine
TFA Trifluoroacetic acid
TFAA Trifluoroacetic anhydride
TfO Trifluoromethanesulfonate triflate
THF Tetrahydrofuran
TMSCl Trimethylsilyl chloride
Togni Reagent II 1-Trifluoromethyl-1,2-benziodoxol-3-(1H)-one

The progress of reactions was often monitored by TLC or LC-MS. The LC-MS was recorded using one of the following methods.

### LCMS METHOD-1:

| | | | | |
|---|---|---|---|---|
| Mobile Phase | (A) | 2 mM Ammonium acetate + 0.1% Formic Acid in Water | | |
| | (B) | 0.1% Formic Acid in Acetonitrile | | |
| Column | : | BEH C18 (50*2.1mm) 1.7 um | | |
| Column Flow | : | 0.55 mL/min | | |
| Gradient | : | Time (min) | % A | % B |
| | | 0.01 | 98 | 2 |
| | | 0.30 | 98 | 2 |
| | | 0.60 | 50 | 50 |
| | | 1.10 | 25 | 75 |
| | | 2.00 | 0 | 100 |
| | | 2.70 | 0 | 100 |
| | | 2.71 | 98 | 2 |
| | | 3.00 | 98 | 2 |

### LCMS Method-2:

| | | | | |
|---|---|---|---|---|
| Mobile Phase | (A) | 5mM Ammonium Acetate + 0.1% Formic Acid in Water | | |
| | (B) | 0.1% Formic Acid in Acetonitrile | | |
| Column | : | BEH C18 (50*2.1mm), 1.7um or Equivalent | | |
| Column Flow | : | 0.45 mL/min | | |
| Gradient | : | Time (min) | % A | % B |
| | | 0.01 | 98 | 2 |
| | | 0.50 | 98 | 2 |
| | | 5.00 | 10 | 90 |
| | | 6.00 | 5 | 95 |
| | | 7.00 | 5 | 95 |
| | | 7.01 | 98 | 2 |
| | | 8.00 | 98 | 2 |

### LCMS Method-3:

| | | | | |
|---|---|---|---|---|
| Mobile Phase | (A) | 5mM Ammonium bicarbonate in water | | |
| | (B) | Acetonitrile | | |
| Column | : | X-Bridge C18 (50*4.6 mm), 3.5 um | | |
| Column Flow | : | 1.0 mL/min | | |
| Gradient | : | Time (min) | % A | % B |
| | | 0.01 | 95 | 5 |
| | | 5.00 | 10 | 90 |
| | | 5.80 | 5 | 95 |
| | | 7.20 | 5 | 95 |
| | | 7.21 | 95 | 5 |
| | | 10.00 | 95 | 5 |

### LCMS Method-4:

| | | | | |
|---|---|---|---|---|
| Mobile Phase | (A) | 10mM Ammonium Acetate in WATER | | |
| | (B) | 100% Acetonitrile | | |
| Column | : | X-Bridge C18 (150*4.6 mm), 5 um or Equivalent | | |
| Column Flow | : | 1.0 mL/min | | |
| Gradient | : | Time (min) | % A | %B |
| | | 0.01 | 90 | 10 |
| | | 5.00 | 10 | 90 |
| | | 7.00 | 0 | 100 |
| | | 11.00 | 0 | 100 |
| | | 11.01 | 90 | 10 |
| | | 12.00 | 90 | 10 |

### LCMS Method-5:

| | | | | |
|---|---|---|---|---|
| Mobile Phase | (A) | 10mM Ammonium Acetate in Water | | |
| | (B) | 100% Acetonitrile | | |
| Column | : | X-Bridge C18 (150*4.6 mm), 5 um or Equivalent | | |
| Column Flow | : | 1.0 mL/min | | |
| Gradient | : | Time (min) | % A | % B |
| | | 0.01 | 100 | 0 |
| | | 7.00 | 50 | 50 |
| | | 9.00 | 0 | 100 |
| | | 11.00 | 0 | 100 |
| | | 11.01 | 100 | 0 |
| | | 12.00 | 100 | 0 |

### LCMS Method-6:

| | | | | |
|---|---|---|---|---|
| Mobile Phase | (A) | 0.1% Formic Acid in Water | | |
| | (B) | 0.1% Formic Acid in Acetonitrile | | |
| Column | : | Zorbax SB-C8 (4.5x75 mm), 3.5 µm | | |
| Column Flow | : | 1.5 mL/min | | |
| Gradient | : | Time (min) | % A | %B |
| | | 0.00 | 95 | 5 |
| | | 3.60 | 5 | 95 |
| | | 4.00 | 5 | 95 |
| | | 4.50 | 95 | 5 |

NMR was recorded at room temperature unless noted otherwise on Varian Inova 400 or 500 MHz spectrometers with the solvent peak used as the reference or on Bruker 300 or 400 MHz spectrometers with the TMS peak used as internal reference.

The compounds described herein may be prepared using the following methods and schemes. Unless specified otherwise, all starting materials used are commercially available.

### Method A.

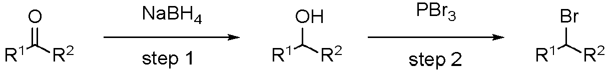

### Method B.

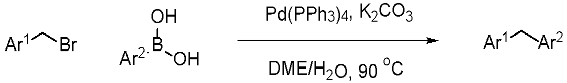

### Method C.

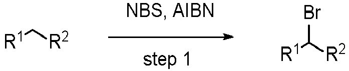

### Method D.

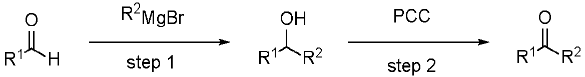

### Method E.

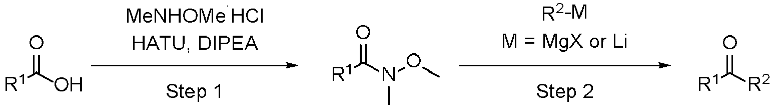

### Method F.

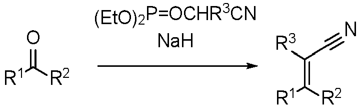

### Method G.

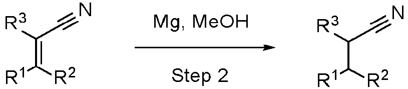

### Method H.

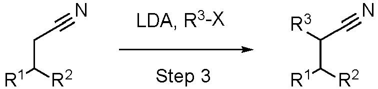

### Method I.

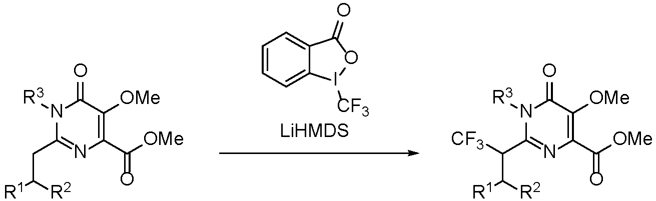

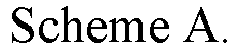

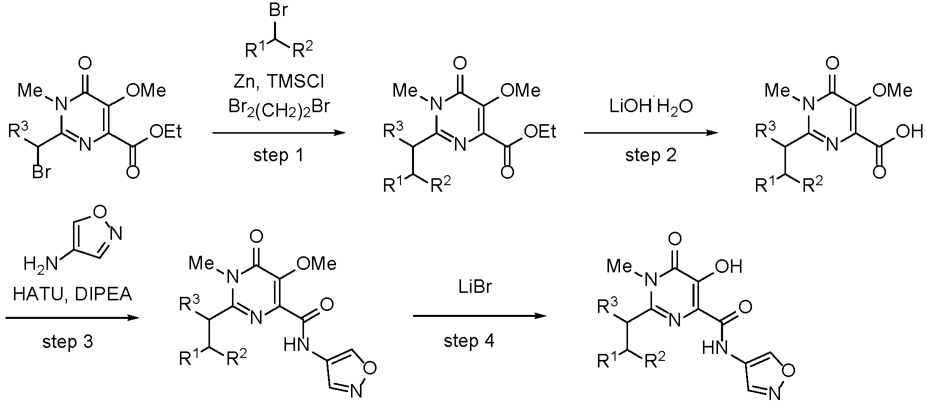

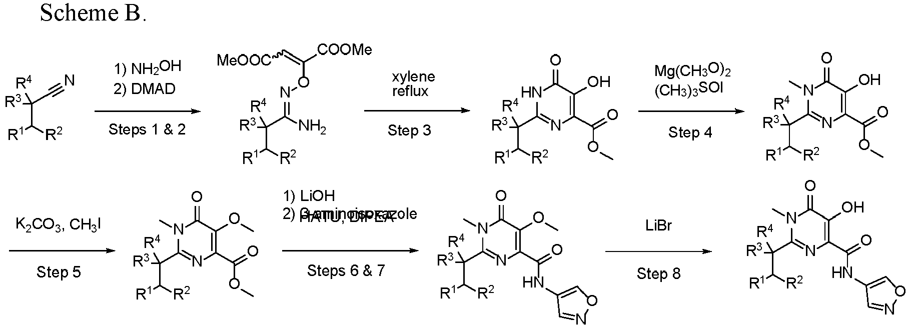

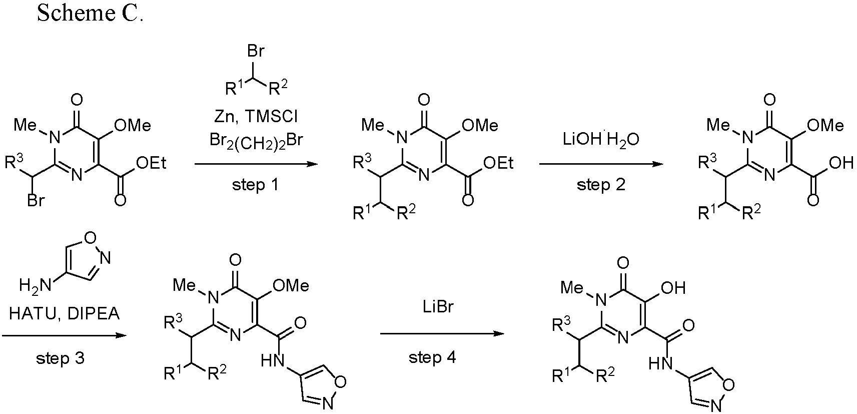

Method A, preparation of: Bromo(phenyl)methyl]-2-chlorobenzene

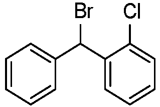

Method A, step 1. 2-chlorobenzhydrol: To a stirred solution of (2-chlorophenyl)(phenyl)-methanone (20.0 g, 92.3 mmol) in MeOH (200 mL) was added sodium borohydride (6.98 g, 184.6 mmol) in portions at 0 °C. The resulting mixture was stirred for 1 h at RT. The reaction was quenched with ice water at 0 °C. The resulting mixture was concentrated under reduced pressure. The resulting mixture was extracted with ethyl acetate (3 x 50 mL). The combined organic layers were washed with water (2 x 50 mL) and dried over anhydrous sodium sulfate. After filtration, the filtrate was concentrated under reduced pressure to afford the title compound (89% yield) as a light yellow solid.

¹H NMR (400 MHz, DMSO-d6) δ 7.69 (dd, J = 8.0, 1.8 Hz, 1H), 7.44 - 7.16 (m, 8H), 6.05 (d, J = 4.4 Hz, 1H), 6.00 (d, *J =* 4.4 Hz, 1H).

Method A, step 2. bromo(phenyl)methyl]-2-chlorobenzene: To a stirred solution of 2-chlorobenzhydrol (10.0 g, 45.7 mmol) in dichloromethane (100 mL) was added phosphorus tribromide (6.19 g, 22.9 mmol) dropwise at 0 °C under an atmosphere of argon. The resulting mixture was stirred for 1 h at RT. The reaction was quenched with ice water at 0 °C. The resulting mixture was diluted with dichloromethane. The resulting mixture was washed with water (3 x 100 mL). The combined organic layers were dried over anhydrous sodium sulfate. After filtration, the filtrate was concentrated under reduced pressure to afford the title compound (69% yield) as a light-yellow oil.

¹H NMR (400 MHz, DMSO-d6) δ 7.70 (dd, J = 7.7, 1.8 Hz, 1H), 7.49 (dq, J = 7.8, 1.7 Hz, 3H), 7.45 - 7.29 (m, 5H), 6.80 (s, 1H).

### Method B, preparation of: 2-[(3,5-difluorophenyl)methyl]benzonitrile

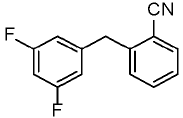

Method B. 2-[(3,5-difluorophenyl)methyl]benzonitrile: Into a 500mL 3-necked round-bottom flask were added 2-(bromomethyl)benzonitrile (8.00 g, 40.8 mmol), 3,5-difluorophenylboronic acid (8.38 g, 53.0 mmol), Pd(PPh₃)₄ (1.41 g, 1.220 mmol), K₂CO₃ (11.3 g, 81.6 mmol), DME (150 mL) and water (30 mL). The resulting mixture was stirred for 1h at 90 °C under an atmosphere of nitrogen. The mixture was cooled to RT. The organic solvent was removed under reduced pressure and the remaining aqueous layer was extracted with ethyl acetate (3x100 mL). The combined organic layers were dried over anhydrous Na₂SO₄. After filtration, the filtrate was concentrated under reduced pressure. The residue was purified by preparative TLC (10:1 petroleum ether:ethyl acetate) to afford the title compound (7.0 g, 75% yield) as a white solid.

¹H NMR (300 MHz, DMSO-d6) δ 7.79 (dd, J = 7.7, 1.4 Hz, 1H), 7.64 (td, J = 7.7, 1.5 Hz, 1H), 7.53 - 7.36 (m, 2H), 7.07 - 6.85 (m, 3H), 4.20 (s, 2H). ESI-MS m/z = 230.1 [M+H].

The compounds in Table 1 were prepared using the appropriate starting materials using a procedure similar to that described above for 2-[(3,5-difluorophenyl)methyl]benzonitrile.

**Table 1.**

| Intermediate | yield | ¹H NMR | LCMS |
|---|---|---|---|
| 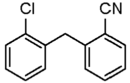 | 89% | (300 MHz, DMSO-d6) δ 7.86 (dd, J = 7.8, 1.4 Hz, 1H), 7.64 (td, J = 7.7, 1.4 Hz, 1H), 7.53 - 7.42 (m, 2H), 7.38 - 7.29 (m, 2H), 7.28 - 7.18 (m, 2H). | |
| 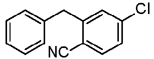 | 67% | | m/z = 228.1 [M+H] |
| 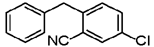 | 85% | | m/z = 270.2 [M+H+ACN] |
| 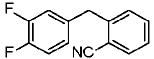 | 73% | (300 MHz, DMSO-*d*₆) δ 7.86-7.82 (m, 1H), 7.70-7.66 (m, 1H), 7.61-7.30 (m, 4H), 7.10-7.06 (m, 1H), 4.18 (s, 2H). | |
| 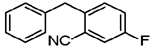 | 45% | | m/z = 212.0 [M+H]. |
| 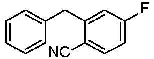 | 63% | (300 MHz, DMSO-*d*₆) δ 7.93 (dd, *J =* 8.6, 5.7 Hz, 1H), 7.45 - 7.14 (m, 7H), 4.17 (s, 2H). | m/z = 212.1 [M+H]. |
| 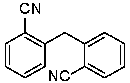 | 71% | (400 MHz, DMSO-d6) δ 7.90-7.87 (m, 2H), 7.71-7.65 (m, 2H), 7.54-7.46 (m, 2H), 7.32-7.28 (m, 2H), 4.42 (s, 2H). | |

### Method C, preparation of: 2-[bromo(phenyl)methyl]benzonitrile

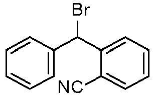

Method C, step 1. 2-[bromo(phenyl)methyl]benzonitrile: Into a 40-mL sealed tube, was placed 2-benzylbenzonitrile (1.70 g, 8.80 mmol), 1-bromopyrrolidine-2,5-dione (2.39 g, 13.2 mmol), and AIBN (433 mg, 2.64 mmol) in DCE (20 mL). The resulting solution was stirred for 1 h at 80 °C under a blue LED lamp. The mixture was cooled to RT and the mixture was concentrated under reduced pressure. The residue was purified by silica gel column chromatography (40:1 petroleum ether:ethyl acetate) to afford the title compound (1.7 g, 71% yield) as an orange oil.

¹H NMR (300 MHz, DMSO-d6) δ 7.89 (dt, J = 7.7, 1.0 Hz, 1H), 7.89 - 7.69 (m, 2H), 7.63 - 7.47 (m, 3H), 7.53 - 7.36 (m, 2H), 7.42 - 7.30 (m, 1H), 6.78 (s, 1H). ESI-MS m/z = 272.1 [M+H].

The compounds in Table 2 were prepared using the appropriate starting materials using a procedure similar to that described above for 2-[bromo(phenyl)methyl]benzonitrile.

**Table 2.**

| Intermediate | yield | ¹H NMR | LCMS |
|---|---|---|---|
| 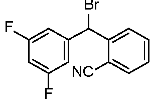 | 56% | | m/z = 323.3, 325.3 [M+16] |
| 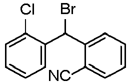 | 85% | (300 MHz, DMSO-d6) δ 7.93 (dd, J = 7.7, 1.4 Hz, 1H), 7.79 (td, J = 7.7, 1.4 Hz, 1H), 7.72 - 7.63 (m, 2H), 7.62 - 7.53 (m, 2H), 7.46 (ddd, J = 6.8, 4.3, 2.0 Hz, 2H), 6.92 (s, 1H). | |
| 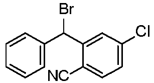 | 74% | (300 MHz, DMSO-*d*₆) δ 7.96 (d, *J =* 8.3 Hz, 1H), 7.79 (d, *J =* 2.1 Hz, 1H), 7.67 (dd, *J =* 8.3, 2.1 Hz, 1H), 7.57 - 7.49 (m, 2H), 7.46 - 7.31 (m, 3H), 6.78 (s, 1H). | m/z = 306.0, 308.0 [M+H] |
| 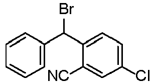 | 84% | | m/z = 306.0 and 308.0 [M+H]. |
| 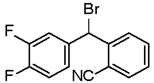 | 62% | (300 MHz, DMSO-*d*₆) δ 7.94-7.80 (m, 1H), 7.80-7.46 (m, 5H), 7.40-7.36 (m, 1H), 6.82 (s, H). | |
| 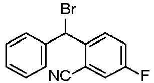 | 32% | | m/z = 289.7, 291.7 [M+H]. |
| 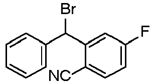 | 90% | (300 MHz, DMSO-*d*₆) δ 8.02 (dd, *J =* 8.6, 5.6 Hz, 1H), 7.62 (dd, *J =* 9.8, 2.6 Hz, 1H), 7.58 - 7.51 (m, 2H), 7.46 (dd, *J =* 8.4, 2.5 Hz, 1H), 7.42 (d, *J* = 2.2 Hz, 1H), 7.41 - 7.38 (m, 1H), 7.38 - 7.23 (m, 1H), 6.77 (s, 1H). | m/z = 290.0/292.0 [M+H]. |

### Method D, preparation of: Bis(2-chlorophenyl)methanone

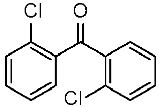

Method D, step 1: Bis(2-chlorophenyl)methanol: To a solution of 2-chlorobenzaldehyde (80.0 g, 569 mmol) in dry THF (800 mL) under an atmosphere of nitrogen was added a solution of (2-chlorophenyl)magnesium bromide (739.0 mL, 1 M in THF, 739 mmol) drop wise at -78 °C. The resulting reaction mixture was warmed to RT and stirred for 1 hour. After completion of reaction, the reaction was quenched by the addition of saturated aqueous ammonium chloride solution (1000 mL) and was extracted with ethyl acetate (2 x 1500 mL). The combined organic layers were washed with brine (1000 mL), dried over anhydrous sodium sulphate and concentrated under reduced pressure. The residue was purified by silica gel column chromatography to afford the title compound (28.0 g, 19% yield) as a yellow oil.

LCMS: m/z = 253.2 [M+1].

Method D, step 2. Bis(2-chlorophenyl)methanone: A solution of bis(2-chlorophenyl)methanol (28.0 g, 110.6 mmol) in dry DCM (560 mL) was cooled to 0 °C. To this, pyridinium chlorochromate (26.0 g, 120.6 mmol) was added portion wise. The resulting reaction mixture was warmed to RT and stirred for 2 hours. After completion of reaction, the reaction mixture was poured into ice-cold water (500 mL) and extracted with DCM (2 x 500 mL). The combined organic layers were washed with brine (200 mL), dried over anhydrous sodium sulphate and concentrated under reduced pressure. The residue was purified by silica gel column chromatography to afford the title compound (20.0 g, 72% yield) as a light yellow oil.

LCMS: m/z = 252.6 [M+1].

The compounds in Table 3 were prepared using the appropriate starting materials using a procedure similar to that described above for bis(2-chlorophenyl)methanone.

**Table 3.**

| Intermediate | yield | ¹H NMR | LCMS |
|---|---|---|---|
| 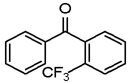 | Step 1 - 78% | (400 MHz, DMSO-d*₆*): δ 7.42-7.44 (m, 1H), 7.50 (t, *J =* 7.6 Hz, 2H), 7.63-7.67 (m, 3H), 7.82 (d, *J =* 7.6 Hz, 3H). | |
| | Step 2 - 70% | | |
| 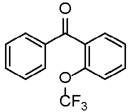 | Step 1 - 55% | (400 MHz, DMSO-d*₆*): 7.58-7.63 (m, 5H), 7.72-7.74 (m, 4H). | |
| | Step 2 - 79% | | |

### Method F, preparation of: 3,3-Bis(2-chlorophenyl)acrylonitrile

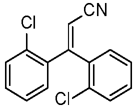

Method F. Step-3: 3,3-Bis(2-chlorophenyl)acrylonitrile: To a stirred solution of diethyl (cyanomethyl)phosphonate (42.0 g, 237 mmol) in dry DME (420 mL) was added sodium hydride (4.8 g, 60% oil dispersion, 119 mmol) at 0 °C. The reaction mixture was heated to 100 °C and stirred for 30 minutes. Bis(2-chlorophenyl)methanone (20.0 g, 79.64mmol) in DME (200 mL) was added and the reaction mixture was heated at 100 °C for 1 hour. After completion of reaction, the reaction mixture was poured into ice-water (1000 mL) and extracted with DCM (2 x 500 mL). The combined organic layers were washed with brine (200 mL), dried over anhydrous sodium sulphate and concentrated under reduced pressure. The crude compound was purified by silica gel chromatography to afford the title compound (9.3 g, 42% yield) as light yellow solid.

¹H NMR (400 MHz, DMSO-d6): δ 5.99 (s, 1H), 7.17 -7.47 (m, 8H).

The compounds in Table 4 were prepared using the appropriate starting materials using a procedure similar to that described above for 3,3-Bis(2-chlorophenyl)acrylonitrile.

**Table 4.**

| Intermediate | yield | ¹H NMR | LCMS |
|---|---|---|---|
| 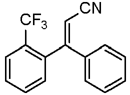 | 96% | (400 MHz, DMSO-d*₆*): δ 6.06 (s, 1H), 7.32-7.38 (d, *J* = 7.2 Hz, 1H), 7.39-7.52 (m, 4H), 7.68-7.89 (m, 4H). | *m*/*z* = 274.17 [M+1]. |
| 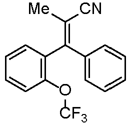 | 92% | (Mixture of E & Z isomer, 400 MHz, CDCl₃): δ 1.97 (s, 3H), 2.17 (s, 3H), 7.16-7.18 (m, 2H), 7.24 (d, *J =* 7.6 Hz, 1H), 7.28-7.30 (m, 2H), 7.33-7.37 (m, 10H), 7.44-7.50 (m, 3H). | *m*/*z* = 304.20 [M+1]. |
| 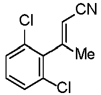 | 36% | (300 MHz, DMSO-d₆) δ 7.69 - 7.56 (m, 2H), 7.48 (dd, J = 8.9, 7.2 Hz, 1H), 6.23 (q, J = 1.6 Hz, 1H), 5.76 (s, 1H), 2.19 (d, J = 1.7 Hz, 3H). | *m*/*z = 253.1 [M+H+ACN].* |

### Method G, preparation of: 3,3-Bis(2-chlorophenyl)propanenitrile

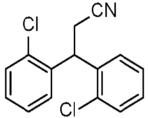

Method G. Step-4: 3,3-Bis(2-chlorophenyl)propanenitrile: To a solution of 3,3-bis(2-chlorophenyl)acrylonitrile (9.3. g, 33.9 mmol) in dry THF:MeOH (186 mL, 1:1) under an atmosphere of nitrogen was added of magnesium metal (24.85 g, 1022 mmol). To it, ammonium chloride (1.8 g, 33.6 mmol) was added in portions at 0 °C. The resulting reaction mixture was warmed to RT and stirred for 3 hours. After completion of reaction, the reaction was quenched with saturated aqueous sodium bicarbonate solution (150 mL) and the mixture was extracted with ethyl acetate (2 x 150 mL). The combined organic layers were washed with brine (50 mL), dried over anhydrous sodium sulphate and concentrated under reduced pressure. The crude compound was triturated with 4:1 ethyl acetate:hexanes (2 x 50 mL) to provide a solid that was filtered and dried under vacuum to afford the title compound (7.6 g, 81% yield).

¹H NMR (400 MHz, DMSO-d6): δ 3.34-3.36 (m, 2H), 5.12 (t, J = 7.6 Hz, 1H), 7.33-7.39 (m, 6H), 7.50 (d, J = 7.6 Hz, 2H).

The compounds in Table 5 were prepared using the appropriate starting materials using a procedure similar to that described above for 3,3-Bis(2-chlorophenyl)propanenitrile.

**Table 5.**

| Intermediate | yield | ¹H NMR | LCMS |
|---|---|---|---|
| 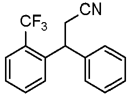 | 89% | (400 MHz, DMSO-d*₆*): δ 3.48 (d, *J =* 17.6 Hz, 2H), 4.71 (t, *J =* 8.0 Hz, 1H), 7.25-7.30 (m, 1H), 7.34-7.37 (m, 4H), 7.52 (t, *J =* 7.6 Hz, 1H), 7.71-7.78 (m, 3H). | |
| 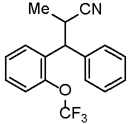 | 84% | (400 MHz, CDCl₃): δ 1.37 (d, J=7.2 Hz, 3H), 3.42-3.50 (m, 1H), 4.49-4.53 (m, 1H), 7.28-7.40 (m, 7H), 7.44-7.46 (m, 1H), 7.61-7.63 (m, 1H). | |
| 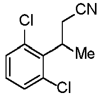 | 48% | (300 MHz, DMSO-d₆) δ 7.50 (d, J = 8.1 Hz, 2H), 7.33 (t, J = 8.0 Hz, 1H), 4.08 (dp, J = 9.5, 7.2 Hz, 1H), 3.28 - 2.99 (m, 2H), 1.42 (d, J = 7.1 Hz, 3H). | |

### Method H, preparation of: 2-Methyl-3, 3-diphenylpropanenitrile

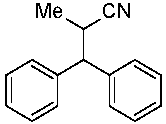

Method H. 2-Methyl-3, 3-diphenylpropanenitrile: To a dried 250 mL round bottom flask under an atmosphere of nitrogen, diisopropylamine (3.66 g, 36.2 mmol) and THF (40 mL) were added. The reaction mixture was cooled to -78 °C and n-BuLi (12.6 mL, 2.5 M in hexanes, 31.4 mmol) was added dropwise. The solution was stirred for 30 minutes at -78 °C before a solution of 3, 3-diphenylpropanenitrile (5.00 g, 24.1 mmol) in THF (25 mL) was added. The resulting yellow solution was stirred for 1 hour and then methyl iodide (3.77 g, 26.53mmol) was added drop wise at -78 °C. The reaction temperature was slowly increased to RT and stirring was continued for 3 hours. After completion of reaction, the reaction was quenched with 10% aqueous solution of ammonium chloride (50 mL) and the mixture was extracted with ethyl acetate (2 x 50 mL). The combined organic layers were washed with brine (50 mL), dried over anhydrous sodium sulphate and concentrated under vacuum. The residue was purified by silica gel column chromatography to afford the title compound (3.2 g, 60% yield) as a white solid.

¹H NMR (400 MHz, CDCl₃): δ 1.34 (d, J = 6.8 Hz, 3H), 3.41-3.48 (m, 1H), 4.04 (d, J = 9.6 Hz, 1H), 7.25-7.29 (m, 5 H) 7.32-7.37 (m, 5H).

### Method H, preparation of: 2,2-Dimethyl-3,3-diphenylpropanenitrile

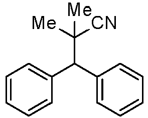

Method H. 2,2-Dimethyl-3,3-diphenylpropanenitrile: To a dried 250 mL round bottom flask under an atmosphere of nitrogen, diisopropylamine (14.65 g, 144.7 mmol) and THF (100 mL) were added. The reaction mixture was cooled to -78 °C and n-BuLi (54.0 mL, 2.5 M in hexanes, 135 mmol) was added dropwise. The solution was stirred for 30 minutes at -78 °C and a solution of 3,3-diphenylpropanenitrile (10 g, 48.2 mmol) in THF was added. The resulting yellow solution was stirred for 1 hour and then methyl iodide (20.54 g, 144.73mmol) was added dropwise at -78 °C. The reaction temperature was slowly increased to RT and stirring was continued for 3 hours. After completion of reaction, the reaction mixture was quenched with 10% aqueous solution of ammonium chloride (100 mL) and extracted with ethyl acetate (2 x 100 mL). The combined organic layers were washed with brine (50 mL), dried over anhydrous sodium sulphate and concentrated under vacuum. The residue was purified by silica gel column chromatography to afford the title compound (8.0 g, 70% yield) as a white solid.

¹H NMR (400 MHz, CDCl₃): δ 1.41 (s, 6H), 3.66 (s, 1H), 7.27-7.29 (m, 2H), 7.35 (t, J = 7.2 Hz, 4H) 7.59 (d, J = 7.2 Hz, 4H).

The compounds in Table 6 were prepared using the appropriate starting materials using a procedure similar to that described above for 2-methyl-3, 3-diphenylpropanenitrile.

**Table 6.**

| Intermediate | yield | ¹H NMR | LCMS |
|---|---|---|---|
| 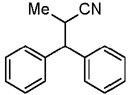 | 52% | (400 MHz, CDCl₃): δ 1.45 (d, *J =* 7.2 Hz, 3H), 3.40-3.48 (m, 1H), 5.14 (d, *J =* 8.4 Hz, 1H), 7.20-7.29 (m, 4H), 7.35 (t, *J =* 8.4 Hz, 1H), 7.41-7.43 (m, 2H), 7.68 (d, *J =* 7.6 Hz, 1H). | |
| 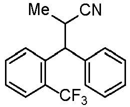 | 52% | (400 MHz, DMSO-d*₆*): δ 1.18 (dd, *J₁* = 7.2 Hz, *J₂ =* 20 Hz, 3H), 3.94-4.04 (m, 1H), 4.40 (d, *J =* 10.8 Hz, 1H), 7.26 (q, *J =* 7.2 Hz, 1H), 7.33-7.43 (m, 3H), 7.46-7.50 (m, 2H), 7.69-7.76 (m, 2H), 7.88 (d, *J =* 7.6 Hz, 0.5H), 8.02 (d, *J =* 8.0 Hz, 0.5H). | |

### Method I, preparation of: Methyl 5-methoxy-1-methyl-6-oxo-2-(1,1,1-trifluoro-3,3-diphenylpropan-2-yl)-1,6-dihydro pyrimidine-4-carboxylate

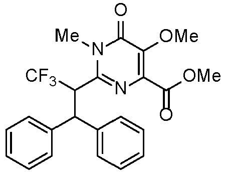

Method I. Methyl 5-methoxy-1-methyl-6-oxo-2-(1,1,1-trifluoro-3,3-diphenylpropan-2-yl)-1,6-dihydro pyrimidine-4-carboxylate: To a stirred solution of methyl 2-(2,2-diphenylethyl)-5-methoxy-1-methyl-6-oxo-1,6-dihydropyrimidine-4-carboxylate (0.500 g, 1.32 mmol) in THF (5 mL) was added LiHMDS (1.98 mL, 1M in THF, 1.98 mmol) at -78 °C. The reaction mixture was stirred at -78 °C for 30 minutes before the addition of 1-trifluoromethyl-1,2-benziodoxol-3-(1H)-one (0.543 g, 1.72 mmol). The reaction mixture was warmed to RT and stirred for 4 hours. After completion of the reaction, the reaction mixture was quenched with saturated aqueous ammonium chloride (20 mL) and extracted with ethyl acetate (3 x 10 mL). The combined organic layers were washed with brine (10 mL), dried over anhydrous sodium sulphate and concentrated. The residue was purified by silica gel column chromatography to afford the title compound (0.08 g, 13% yield).

¹H NMR (400 MHz, CDCl₃): δ1.37 (s, 3H), 3.93 (s, 3H), 3.98 (s, 3H), 4.59-4.64 (m, 1H), 5.04 (d, J = 11.6 Hz, 1H), 7.10 (t, J = 7.6 Hz, 1H), 7.17-7.28 (m, 5H), 7.35 (t, J = 7.6 Hz, 2H), 7.49 (d, J = 7.6 Hz, 2H). ¹⁹F NMR (400 MHz, CDCl3): δ -62.63. LCMS: m/z = 447.40 [M+1].

### Reference Example A.

### 2-(2,2-diphenylethyl)-5-hydroxy-N-(isoxazol-4-yl)-1-methyl-6-oxo-1,6-dihydropyrimidine-4-carboxamide

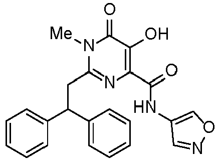

Scheme B, step 1. N-Hydroxy-3,3-diphenylpropanimidamide: To a stirred mixture of 3,3-diphenylpropanenitrile (10.00 g, 48.2 mmol) and hydroxylamine hydrochloride (5.00 g, 71.9 mmol) in ethanol (100 mL), sodium carbonate (10.75 g, 101.42mmol) in water (30 mL) was added at RT. The reaction mixture was heated to reflux at 90 °C for 16 hours. After completion of reaction, the reaction mixture was cooled to RT. The reaction mixture was concentrated, diluted with water (100 mL) and extracted with ethyl acetate (2 x 100 mL). The combined organic layers were dried over anhydrous sodium sulphate and concentrated under reduced pressure to obtain the crude title compound (12.0 g). The crude product was used in the next step without further purification.

LCMS: m/z = 241.18 [M+1].

Scheme B, step 2. Dimethyl 2-((E)-N'-hydroxy-3,3-diphenylpropanimidamido)maleate: A solution of N-hydroxy-3,3-diphenylpropanimidamide (12.00 g, 49.93 mmol) in chloroform (120 mL) was cooled 0 °C. To the cooled solution, dimethyl acetylenedicarboxylate (7.10 g, 50.0 mmol) was added drop wise. The reaction mixture was stirred at RT for 4 hours. After completion of reaction, the reaction mixture was concentrated and the residue was purified by silica gel column chromatography to afford the title compound (4.5 g, 24% yield over 2 steps)).

¹H NMR (400 MHz, DMSO-d6): δ 2.85 (d, J = 8.4 Hz, 2H), 3.58 or 3.62 (s, 3H), 3.68 or 3.73 (s, 3H), 4.48 (t, J = 8.0 Hz, 1H), 5.34 (s, 1H), 6.65 (bs, 2H), 7.17 (t, J = 7.2 Hz, 2H), 7.26-7.34 (m, 8H). LCMS: m/z = 383.30 [M+1].

Scheme B, step 3. Methyl 2-(2,2-diphenylethyl)-5-hydroxy-6-oxo-1,6-dihydropyrimidine-4-carboxylate: Dimethyl 2-((E)-N'-hydroxy-3,3-diphenylpropanimidamido)maleate (4.50 g, 11.77mmol) was dissolved in o-xylene (50 mL) and heated in a microwave reactor at 180 °C for 1 hour. After completion of reaction, the reaction mixture was concentrated and triturated with diethyl ether (50 mL) and hexanes (50 mL) to provide the crude compound which was purified by silica gel column chromatography to afford the title compound (1.8 g, 43% yield).

¹H NMR (400 MHz, DMSO-d6): δ 3.25 (d, J = 8.0 Hz, 2H), 3.79 (s, 3H), 4.62 (t, J = 8.0 Hz, 1H), 7.16-7.28 (m, 10H), 10.16 (bs, 1H), 12.81 (bs, 1H). LCMS: m/z = 351.25 [M+1].

Scheme B, step 4. Methyl 2-(2,2-diphenylethyl)-5-hydroxy-1-methyl-6-oxo-1,6-dihydropyrimidine-4-carboxylate: To a solution of methyl 2-(2,2-diphenylethyl)-5-hydroxy-6-oxo-1,6-dihydropyrimidine-4-carboxylate (2.00 g, 5.71 mmol) in DMSO (20 mL) cooled to 0 °C was added magnesium methoxide (12.4 mL, 6-10% in MeOH (8%), 11.4 mmol) drop wise. The reaction mixture was stirred at RT for 1 hour. The MeOH was removed under reduced pressure and the reaction mixture was cooled to 0 °C before the dropwise addition of methyl iodide (3.24 g, 22.8 mmol). The reaction mixture was stirred at RT for 16 hours. The reaction mixture was cooled to 5-10 °C and quenched by dropwise addition of 1N HCl (15 mL). The mixture was extracted with ethyl acetate (2 x 200 mL) and the combined organic layers were dried over anhydrous sodium sulphate and concentrated under reduced pressure. The residue was purified by reverse phase chromatography (C18 column; acetonitrile in water + 0.1% formic acid) to afford the title compound (1.63 g, 78% yield).

¹H NMR (400 MHz, DMSO-d6): δ 3.46 (s, 3H), 3.54 (d, J = 7.6 Hz, 2H), 3.81 (s, 3H), 4.68 (t, J = 7.2 Hz, 1H), 7.15 (t, J = 7.2 Hz, 2H), 7.25 (t, J = 7.6 Hz, 4H), 7.38 (d, J = 7.6 Hz, 4H), 10.11 (s, 1H). LCMS: m/z = 365.24 [M+1].

Scheme B, step 5. Methyl 2-(2,2-diphenylethyl)-5-methoxy-1-methyl-6-oxo-1,6-dihydropyrimidine-4-carboxylate: To a stirred solution of methyl 2-(2,2-diphenylethyl)-5-hydroxy-1-methyl-6-oxo-1,6-dihydropyrimidine-4-carboxylate (1.50 g, 4.11 mmol) in DMF (7.5 mL) was added cesium carbonate (3.35 g, 10.3 mmol). To this suspension, methyl iodide (0.879 g, 6.17mmol) was added at RT. The reaction mixture was stirred for 2 hours. After completion of reaction, the reaction mixture was diluted with water (20 mL) and extracted with ethyl acetate (2 x 20 mL). The combined organic layers were washed with brine (10 mL), dried over anhydrous sodium sulphate and concentrated under reduced pressure. The crude compound was purified by silica gel column chromatography to afford the title compound (1.5 g, 96% yield).

LCMS: m/z = 379.90 [M+1].

Scheme B, step 6. Sodium 2-(2,2-Diphenylethyl)-5-methoxy-1-methyl-6-oxo-1,6-dihydropyrimidine-4-carboxylate: To a stirred solution of methyl 2-(2,2-diphenylethyl)-5-methoxy-1-methyl-6-oxo-1,6-dihydropyrimidine-4-carboxylate (0.150 g, 0.39 mmol) in 1:1:1 MeOH:THF:water (4.5 mL) sodium hydroxide (0.019 g, 0.47 mmol) was added at RT. The reaction mixture was stirred for 1 hour. After completion of reaction, the reaction mixture was evaporated to dryness. The pH of the residue was adjusted to 5 using 1N HCl (aq.) and extracted with 10% MeOH in DCM (3 x 25 mL). The organic layer was concentrated to afford the title compound (0.12 g, 83% yield) as a solid.

LCMS: m/z = 365.28 [M+1].

Scheme B, step 7: 2-(2,2-Diphenylethyl)-N-(isoxazol-4-yl)-5-methoxy-1-methyl-6-oxo-1,6-dihydropyrimidine-4-carboxamide: To a solution of sodium 2-(2,2-diphenylethyl)-5-methoxy-1-methyl-6-oxo-1,6-dihydropyrimidine-4-carboxylate (0.120 g, 0.33 mmol) in DMF (2.0 mL) was added HATU (0.188 g, 0.49 mmol), isoxazol-4-amine (0.034 g, 0.39 mmol) at RT. The reaction mixture was stirred for 30 minutes and then DIPEA (0.128 g, 0.99mmol) was added. The reaction mixture was stirred for 2 hours. After completion of reaction, the reaction mixture was quenched with water (15 mL) and extracted with ethyl acetate (3 x 10 mL). The organic layers were dried over sodium sulphate and concentrated under reduced pressure. The crude compound was purified by silica gel chromatography to afford the title compound (0.090 g, 63% yield).

¹H NMR (400 MHz, DMSO-d6): δ 3.50 (s, 3H), 3.66 (d, J = 7.2 Hz, 2H), 3.77 (s, 3H), 4.94 (t, J = 7.6 Hz, 1H), 7.14 (t, J = 7.2 Hz, 2H), 7.26 (t, J = 7.6 Hz, 4H), 7.45 (d, J = 7.6 Hz, 4H), 8.83 (s, 1H), 9.30 (s, 1H), 10.26 (s, 1H). LCMS: m/z = 431.81 [M+1].

Scheme B, step 8: 2-(2,2-Diphenylethyl)-5-hydroxy-N-(isoxazol-4-yl)-1-methyl-6-oxo-1,6-dihydropyrimidine-4-carboxamide: To a solution of 2-(2,2-diphenylethyl)-N-(isoxazol-4-yl)-5-methoxy-1-methyl-6-oxo-1,6-dihydropyrimidine-4-carboxamide (0.090 g, 0.21 mmol) in DMF (0.9 mL), lithium bromide (0.182 g, 2.09 mmol) was added at RT. The reaction mixture was irradiated in microwave reactor at 90 °C for 40 minutes. After completion of reaction, the reaction mixture was concentrated and the residue was purified by reverse phase chromatography (C18 column; acetonitrile in in water + 0.1% formic acid) to afford the title compound (0.026 g, 30% yield) as solid.

¹H NMR (400 MHz, DMSO-d6): δ 3.47 (s, 3H), 3.63 (d, J = 7.6 Hz, 2H), 5.01 (t, J = 7.6 Hz, 1H), 7.14 (t, J = 7.2 Hz, 2H), 7.28 (t, J = 7.6 Hz, 4H), 7.46 (d, J = 7.2 Hz, 4H), 8.95 (s, 1H), 9.32 (s, 1H), 10.35 (bs, 1H), 11.17 (bs, 1H). LCMS: m/z = 417.29 [M+1].

### Reference Example B

### 5-hydroxy-N-(isoxazol-4-yl)-1-methyl-2-(2-methyl-1,1-diphenylpropan-2-yl)-6-oxo-1,6-dihydropyrimidine-4-carboxamide

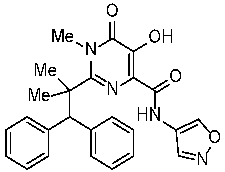

The title compound was prepared in an analogous manner to that described for 2-(2,2-diphenylethyl)-5-hydroxy-N-(isoxazol-4-yl)-1-methyl-6-oxo-1,6-dihydropyrimidine-4-carboxamide (Example A) using scheme B, steps 1 through 8.

¹H NMR (400 MHz, DMSO-d6): δ 1.55 (s, 6H), 3.59 (s, 3H), 4.94 (s, 1H), 7.14-7.18 (m, 2H) 7.24 (t, J = 7.6 Hz, 4H) 7.34 (d, J = 7.6 Hz, 4H), 8.95 (s, 1H), 9.34 (s, 1H), 10.17 (s, 1H), 11.19 (s, 1H). LCMS: m/z = 445.35 [M+1].

### Reference Example 1; Isomer 1, Isomer 2

### (S)-2-(1,1-diphenylpropan-2-yl)-5-hydroxy-N-(isoxazol-4-yl)-1-methyl-6-oxo-1,6-dihydropyrimidine-4-carboxamide

(R)-2-(1,1-diphenylpropan-2-yl)-5-hydroxy-N-(isoxazol-4-yl)-1-methyl-6-oxo-1,6-dihydropyrimidine-4-carboxamide

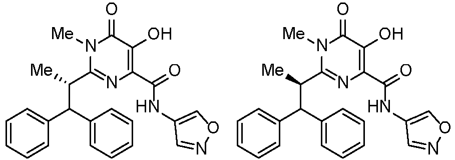

In an analogous manner to that described for Example A, 2-(2,2-diphenylethyl)-5-hydroxy-N-(isoxazol-4-yl)-1-methyl-6-oxo-1,6-dihydropyrimidine-4-carboxamide, Scheme B, steps 1 through 6 were used to prepare the intermediates required for the synthesis of the title compounds.

Scheme B, step 7. 2-(1,1-diphenylpropan-2-yl)-5-hydroxy-N-(isoxazol-4-yl)-1-methyl-6-oxo-1,6-dihydro pyrimidine-4-carboxamide: To a stirred solution of sodium 2-(1,1-diphenylpropan-2-yl)-5-methoxy-1-methyl-6-oxo-1,6-dihydropyrimidine-4-carboxylate (0.450 g, 1.12 mmol) in dry DMF (1.4 mL) was added isoxazol-4-amine (0.116 g, 1.38 mmol) and HATU (0.660 g, 1.73mmol) at RT. The reaction mixture was stirred at RT for 30 minutes. DIPEA (0.451 g, 3.48mmol) was added dropwise and the reaction mixture was allowed to stir at RT for 3 hours. After completion of reaction, the reaction mixture was diluted with water (20 mL) and the aqueous layer was extracted with ethyl acetate (2 x 50 mL). The combined organic layers were washed with brine (20 mL), dried over anhydrous sodium sulphate and concentrated under reduced pressure. The residue was purified by silica gel column chromatography to afford the title compound (0.30 g, 58% yield).

¹H NMR (400 MHz, DMSO-d6): δ 1.19 (d, J = 6.4 Hz, 3H), 3.55 (s, 3H), 3.74 (s, 3H), 4.15-4.23 (m, 1H), 4.60 (d, J = 11.2 Hz, 1H), 7.02 (t, J = 7.6 Hz, 1H), 7.13 (t, J = 7.6 Hz, 2H), 7.22 (t, J = 7.2 Hz 1H), 7.29-7.37 (m, 4H), 7.62 (d, J = 7.2 Hz, 2H), 8.88 (s, 1H), 9.33 (s, 1H), 10.44 (s, 1H). LCMS: m/z = 446.2 [M+2].

The racemic title compound was resolved by Chiral SFC (CHIRALCEL OJ-H; 20% MeOH in liquid CO2+ 0.1% DEA) to furnish the enantiopure intermediates.

Scheme B, step 8: 2-(1,1-diphenylpropan-2-yl)-5-hydroxy-N-(isoxazol-4-yl)-1-methyl-6-oxo-1,6-dihydro pyrimidine-4-carboxamide: To a stirred solution of N-(isoxazol-4-yl)-5-methoxy-1-methyl-2-(methyl(phenyl(2-(trifluoromethyl)phenyl)methyl)amino)-6-oxo-1,6-dihydropyrimidine-4-carboxamide (0.070 g, 0.16 mmol) in DMF (1 mL), lithium bromide (0.082 g, 0.94 mmol) was added at RT. The reaction mixture was heated to 80°C and stirred for 16 hours. After completion of the reaction, the reaction mixture was directly purified by reverse phase chromatography (C18 column; acetonitrile in water + 0.1% formic acid) to afford the title compound (0.020 g, 30% yield). Both stereoisomers were prepared according to this procedure.

Isomer 1 (derived from early-eluting isomer in step 7): ¹H NMR (400 MHz, DMSO-d6): δ 1.22 (d, J = 6.4 Hz, 3H), 3.56 (s, 3H), 4.14-4.18 (m, 1H), 4.81(d, J = 11.2 Hz, 1H) 6.99 (t, J = 7.2 Hz, 1H), 7.11 (t, J = 7.2 Hz, 2H), 7.23 (d, J = 6.8 Hz, 1H), 7.33-7.38 (m, 4H), 7.64 (d, J = 7.2 Hz, 2H) 9.04 (s, 1H), 9.36 (s, 1H), 10.59 (s, 1H), 11.29 (s, 1H). LCMS: m/z = 431.6 [M+1].

Isomer 2 (derived from late-eluting isomer in step 7): ¹H NMR (400 MHz, DMSO-d6): δ 1.21 (d, J = 4.8 Hz, 3H), 3.56 (s, 3H), 4.16 (s, 1H), 4.81 (d, J = 10.4 Hz, 1H), 6.98 (t, J = 6.4 Hz, 1H), 7.09 (t, J = 6.8 Hz, 2H), 7.23 (t, J = 6.0 Hz, 1H), 7.32-7.36 (m, 4H) 7.64 (d, J = 6.4 Hz, 2H) 9.03 (s, 1H), 9.36 (s, 1H), 10.58 (s, 1H), 11.28 (s, 1H). LCMS: m/z = 431.4 [M+1].

### Example 2; Isomer 1, Isomer 2 ,Isomer 3 and Isomer 4

### 2-((1R,2S)-1-(2-chlorophenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-chlorophenyl)-1-phenylpropan-2-yl)-5-hydroxy-N-(isoxazol-4-yl)-1-methyl-6-oxo-1,6-dihydropyrimidine-4-carboxamide

### 2-((1S,2R)-1-(2-chlorophenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(2-chlorophenyl)-1-phenylpropan-2-yl)-5-hydroxy-N-(isoxazol-4-yl)-1-methyl-6-oxo-1,6-dihydropyrimidine-4-carboxamide

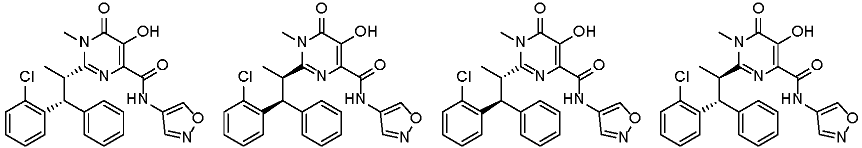

Scheme A, step 1. Ethyl 2-[1-(2-chlorophenyl)-1-phenylpropan-2-yl]-5-methoxy-1-methyl-6-oxopyrimidine-4-carboxylate: Into a 25 mL 3-necked round-bottom flask were added zinc powder (950 mg, 14.5 mmol) and DMA (3.0 mL) at RT. The resulting mixture was stirred for 20 minutes at 65 °C under an atmosphere of argon. To the above mixture was added dibromoethane (0.19 mL) and chlorotrimethylsilane (0.32 mg, 0.003 mmol) dropwise over 5 minutes at 65 °C. The resulting mixture was stirred for additional 30 minutes at 65 °C. The mixture was cooled to 50 °C. To the above mixture was added a solution of 1-[bromo(phenyl)methyl]-2-chlorobenzene (1.76 g, 6.267 mmol, 4.00 equiv) in DMA (2.00 mL) dropwise over 20 minutes at 50 °C. The resulting mixture was stirred for an additional 1 hour at 50 °C, cooled to RT, and then added dropwise to a stirred solution of ethyl 2-(1-bromoethyl)-5-methoxy-1-methyl-6-oxopyrimidine-4-carboxylate (500 mg, 1.57 mmol) in DMA (5.0 mL) cooled to 0 °C. This mixture was then stirred overnight at RT. The reaction was quenched with ice water. The resulting mixture was filtered and the filter cake was washed with ethyl acetate (4x50 mL). The filtrate was washed with brine (3 x 100 mL), dried over anhydrous sodium sulfate, and concentrated under reduced pressure. The residue was purified by silica gel column chromatography (1: 1 hexanes:ethyl acetate) to afford the title compound (380 mg, 55% yield; mixture of diastereomers) as a light yellow solid.

ESI-MS m/z = 441.2 [M+H].

Scheme A, step 2. Lithium 2-[1-(2-chlorophenyl)-1-phenylpropan-2-yl]-5-methoxy-1-methyl-6-oxopyrimidine-4-carboxylate: To a stirred solution of ethyl 2-[1-(2-chlorophenyl)-1-phenylpropan-2-yl]-5-methoxy-1-methyl-6-oxopyrimidine-4-carboxylate (380 mg, 0.86 mmol) in THF (4.0 mL) and water (0.80 mL) was added lithium hydroxide monohydrate (47 mg, 1.12 mmol) in portions at 0 °C. The resulting mixture was stirred for 2 h at RT. The resulting mixture was concentrated under vacuum to afford the title compound (430 mg) which was used in the next step directly without further purification.

ESI-MS m/z = 413.2 [M+H].

Scheme A, step 3. 2-[1-(2-chlorophenyl)-1-phenylpropan-2-yl]-5-methoxy-1-methyl-N-(1,2-oxazol-4-yl)-6-oxopyrimidine-4-carboxamide: To a stirred mixture of lithium (2-[1-(2-chlorophenyl)-1-phenylpropan-2-yl]-5-methoxy-1-methyl-6-oxopyrimidine-4-carboxylate (380 mg, 0.92 mmol), 1,2-oxazol-4-amine (155 mg, 1.84 mmol) and DIPEA (475 mg, 3.68 mmol) in DMF (4.00 mL) was added HATU (700 mg, 1.84 mmol) in portions at 0 °C and the mixture was stirred for 1 h at RT. The reaction mixture was directly purified by reverse phase chromatography (C18 silica gel; 0 to 100% acetonitrile in water) to afford the title compound (250 mg, 56% yield; mixture of diastereomers) as a light yellow solid.

ESI-MS m/z = 479.0 [M+H].

The mixture was separated into the four enantiomeric intermediates by preparative chiral HPLC (CHIRAL ART Cellulose-SB; 20% EtOH in 1: 1 hexanes:MTBE + 0.5% 2M NH3 in MeOH) to afford 2-((1S,2R)-1-(2-chlorophenyl)-1-phenylpropan-2-yl)-N-(isoxazol-4-yl)-5-methoxy-1-methyl-6-oxo-1,6-dihydropyrimidine-4-carboxamide, 2-((1S,2S)-1-(2-chlorophenyl)-1-phenylpropan-2-yl)-N-(isoxazol-4-yl)-5-methoxy-1-methyl-6-oxo-1,6-dihydropyrimidine-4-carboxamide, 2-((1R,2R)-1-(2-chlorophenyl)-1-phenylpropan-2-yl)-N-(isoxazol-4-yl)-5-methoxy-1-methyl-6-oxo-1,6-dihydropyrimidine-4-carboxamide, and 2-((1R,2S)-1-(2-chlorophenyl)-1-phenylpropan-2-yl)-N-(isoxazol-4-yl)-5-methoxy-1-methyl-6-oxo-1,6-dihydropyrimidine-4-carboxamide.

1st-eluting peak, 50 mg; 2nd-eluting peak, 63 mg); 3rd-eluting peak, 56 mg); 4th-eluting peak, 52 mg isolated as white solids. ESI-MS m/z = (1st-eluting peak) 478.9 [M+H], (2nd-eluting peak) 478.9 [M+H], (3rd-eluting peak) 478.9 [M+H], and (4th-eluting peak) 478.9 [M+H].

Scheme A, step 4. 2-[1-(2-chlorophenyl)-1-phenylpropan-2-yl]-5-hydroxy-1-methyl-N-(1,2-oxazol-4-yl)-6-oxopyrimidine-4-carboxamide, Isomer 1 (Example 2, Isomer 1): A mixture of 2-[1-(2-chlorophenyl)-1-phenylpropan-2-yl]-5-methoxy-1-methyl-N-(1,2-oxazol-4-yl)-6-oxopyrimidine-4-carboxamide (1st eluting peak) (50 mg, 0.10 mmol) and lithium bromide (136 mg, 1.56 mmol) in DMF (2.5 mL) was stirred for 3 h at 95 °C. The mixture was cooled to RT. The crude material was purified by reverse phase chromatography (Column: Gemini-NX C18 AXAI Packed; Mobile Phase A: water + 0.1% formic acid, Mobile Phase B: acetonitrile; Gradient: 38 to 70% B) to afford the title compound (20 mg, 40% yield) as an off-white solid.

¹H NMR (400 MHz, DMSO-d6) δ 11.34 (s, 1H), 10.58 (s, 1H), 9.35 (s, 1H), 8.98 (s, 1H), 8.06 (d, J = 7.8 Hz, 1H), 7.44 (m, J = 14.8, 7.7, 1.4 Hz, 2H), 7.29 (m, 1H), 7.16 - 7.09 (m, 4H), 7.03 (m, 1H), 4.96 (d, J = 11.2 Hz, 1H), 4.20 (s, 1H), 1.32 (d, J = 6.4 Hz, 3H). ESI-MS m/z = 465.0 [M+H].

Scheme A, step 4. 2-(1-(2-chlorophenyl)-1-phenylpropan-2-yl)-5-hydroxy-N-(isoxazol-4-yl)-1-methyl-6-oxo-1,6-dihydropyrimidine-4-carboxamide, Isomer 2 (Example 2, Isomer 2): Into a 40-mL sealed tube, was placed 2-(1-(2-chlorophenyl)-1-phenylpropan-2-yl)-N-(isoxazol-4-yl)-5-methoxy-1-methyl-6-oxo-1,6-dihydropyrimidine-4-carboxamide (2nd eluting peak) (56 mg, 0.12 mmol), lithium bromide (151 mg, 1.76 mmol), and DMF (2.80 mL). The resulting solution was stirred for 3 hours at 95 °C. The mixture was cooled to room temperature and the solids were filtered off. The crude product was purified by reverse phase HPLC (Column: Gemini-NX C18 AXAI Packed; Mobile Phase A: water + 0.1% formic acid, Mobile Phase B: acetonitrile; Gradient: 38 to 70% B) to afford the title compound (30 mg, 49% yield) as a white solid.

¹H NMR (400 MHz, DMSO-d6) δ 11.06 (d, J = 2.5 Hz, 1H), 10.37 (s, 1H), 9.34 (s, 1H), 8.96 (s, 1H), 7.74 (dd, J = 7.9, 1.7 Hz, 1H), 7.71 - 7.64 (m, 2H), 7.36 (t, J = 7.6 Hz, 2H), 7.29 - 7.16 (m, 3H), 7.02 (td, J = 7.6, 1.5 Hz, 1H), 5.32 (d, J = 11.3 Hz, 1H), 4.21 (dq, J = 13.1, 6.8 Hz, 1H), 3.69 (s, 3H), 1.20 (d, J = 6.5 Hz, 2H). ESI-MS m/z = 464.9 [M+H].

Scheme A, step 4. 2-(1-(2-chlorophenyl)-1-phenylpropan-2-yl)-5-hydroxy-N-(isoxazol-4-yl)-1-methyl-6-oxo-1,6-dihydropyrimidine-4-carboxamide Isomer 3 (Example 2, Isomer 3): Into a 40-mL sealed tube, was placed 2-(1-(2-chlorophenyl)-1-phenylpropan-2-yl)-N-(isoxazol-4-yl)-5-methoxy-1-methyl-6-oxo-1,6-dihydropyrimidine-4-carboxamide (3rd eluting peak) (56 mg, 0.10 mmol), lithium bromide (151 mg, 1.76 mmol), DMF (2.8 mL). The resulting solution was stirred for 3 h at 95 °C. The mixture was cooled to RT and the solids were filtered off. The crude product was purified by reverse phase HPLC (Column: Gemini-NX C18 AXAI Packed; Mobile Phase A: water + 0.1% formic acid, Mobile Phase B: acetonitrile; Gradient: 38 to 70% B) to afford the title compound (29 mg, 53% yield) as a white solid.

¹H NMR (400 MHz, DMSO-d6) δ 11.06 (d, J = 2.5 Hz, 1H), 10.37 (s, 1H), 9.34 (s, 1H), 8.96 (s, 1H), 7.74 (dd, J = 7.9, 1.7 Hz, 1H), 7.71 - 7.64 (m, 2H), 7.36 (t, J = 7.6 Hz, 2H), 7.29 - 7.16 (m, 3H), 7.02 (td, J = 7.6, 1.5 Hz, 1H), 5.32 (d, J = 11.3 Hz, 1H), 4.21 (dq, J = 13.1, 6.8 Hz, 1H), 3.69 (s, 3H), 1.20 (d, J = 6.5 Hz, 2H). ESI-MS m/z = 464.9 [M+H].

Scheme A, step 4. 2-(1-(2-chlorophenyl)-1-phenylpropan-2-yl)-5-hydroxy-N-(isoxazol-4-yl)-1-methyl-6-oxo-1,6-dihydropyrimidine-4-carboxamide Isomer 4 (Example 2, Isomer 4): Into a 40-mL sealed tube, was placed 2-(1-(2-chlorophenyl)-1-phenylpropan-2-yl)-N-(isoxazol-4-yl)-5-methoxy-1-methyl-6-oxo-1,6-dihydropyrimidine-4-carboxamide (4th eluting peak) (52 mg, 0.11 mmol), lithium bromide (141 mg, 1.64 mmol), and DMF (2.60 mL). The resulting solution was stirred for 3 h at 95 °C. The mixture was cooled to RT and the solids were filtered off. The crude product was purified by reverse phase HPLC (Column: Gemini-NX C18 AXAI Packed; Mobile Phase A: water + 0.1% formic acid, Mobile Phase B: acetonitrile; Gradient: 38 to 70% B) to afford the title compound (21 mg, 41% yield) as a white solid.

¹H NMR (400 MHz, DMSO-d6) δ 11.34 (s, 1H), 10.58 (s, 1H), 9.35 (s, 1H), 8.98 (s, 1H), 8.06 (d, J = 7.7 Hz, 1H), 7.49 - 7.41 (m, 2H), 7.33 - 7.25 (m, 1H), 7.19 - 7.07 (m, 4H), 7.06 - 6.99 (m, 1H), 4.97 (d, J = 11.1 Hz, 1H), 4.20 (s, 1H), 1.32 (d, J = 6.4 Hz, 3H). ESI-MS m/z = 464.9 [M+H].

### Example 3; Isomer 1, Isomer 2 ,Isomer 3 and Isomer 4

### 2-((1R,2S)-1-(2-cyanophenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-cyanophenyl)-1-phenylpropan-2-yl)-5-hydroxy-N-(isoxazol-4-yl)-1-methyl-6-oxo-1,6-dihydropyrimidine-4-carboxamide

### 2-((1S,2R)-1-(2-cyanophenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(2-cyanophenyl)-1-phenylpropan-2-yl)-5-hydroxy-N-(isoxazol-4-yl)-1-methyl-6-oxo-1,6-dihydropyrimidine-4-carboxamide

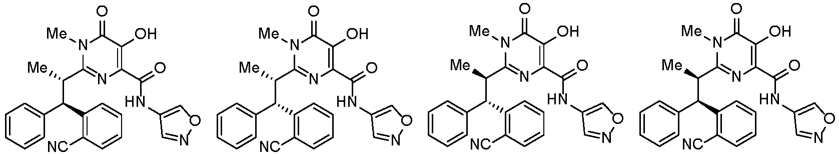

Scheme A, step 1. Ethyl 2-[1-(2-cyanophenyl)-1-phenylpropan-2-yl]-5-methoxy-1-methyl-6-oxopyrimidine-4-carboxylate: Into a 100-mL 3-necked round-bottom flask under an atmosphere of argon, was placed zinc dust (828 mg, 12.6 mmol) and DMA (5.0 mL). The mixture was stirred for 15 minutes at 60 °C. To this mixture was added a solution of dibromoethane (352 mg, 1.87 mmol) and chlorotrimethylsilane (102 mg, 0.937 mmol) in DMA (2.0 mL) dropwise over 15 minutes at 50 °C. The mixture was stirred for 20 minutes before a solution of (2-[bromo(phenyl)methyl]benzonitrile) (1.70 g, 6.25 mmol) in DMA (3.0 mL) was added dropwise at RT. The resulting solution was stirred for 1 hour at RT. The supernatant containing (bromo[(2-cyanophenyl)(phenyl)methyl]zinc) was used in the next phase of step 1.

Into a 40-mL sealed tube under an atmosphere of argon (ethyl 2-(1-bromoethyl)-5-methoxy-1-methyl-6-oxopyrimidine-4-carboxylate) (500 mg, 1.57 mmol) was dissolved in DMA (5.0 mL). A solution of (bromo[(2-cyanophenyl)(phenyl)methyl]zinc) in DMA (10.0 mL) (prepared as described above) was then added dropwise with stirring at 0 °C. The resulting solution was stirred for 2 hours at RT. The reaction was then quenched by the addition of water. The resulting solution was extracted with ethyl acetate (3 × 20 mL) and the organic layers were combined and concentrated. The crude residue was purified by reversed-phase column chromatography (C18 column; 1: 1 acetonitrile:water) to afford the title compound (460 mg, 68% yield) as a white solid.

ESI-MS m/z = 432.2 [M+H].

Scheme A, step 2. Lithium 2-[1-(2-cyanophenyl)-1-phenylpropan-2-yl]-5-methoxy-1-methyl-6-oxopyrimidine-4-carboxylate: Into a 40-mL sealed tube, was placed ethyl 2-[1-(2-cyanophenyl)-1-phenylpropan-2-yl]-5-methoxy-1-methyl-6-oxopyrimidine-4-carboxylate (460 mg, 1.07 mmol), tetrahydrofuran (6.0 mL) and water (4.0 mL). This was followed by the addition of lithium hydroxide hydrate (90 mg, 2.13 mmol) in portions at 0 °C. The resulting solution was stirred for 1 hour at RT. The resulting mixture was concentrated under vacuum to afford the crude title compound (460 mg) as a light-yellow solid which was used in the next step without further purification.

ESI-MS: m/z = 404.2 [M+H], M+H for the parent acid observed.

Scheme A, step 3. 2-[1-(2-Cyanophenyl)-1-phenylpropan-2-yl]-5-methoxy-1-methyl-N-(1,2-oxazol-4-yl)-6-oxopyrimidine-4-carboxamide: Into a 40-mL sealed tube, was placed lithium 2-[1-(2-cyanophenyl)-1-phenylpropan-2-yl]-5-methoxy-1-methyl-6-oxopyrimidine-4-carboxylate (460 mg, 1.12 mmol), 1,2-oxazol-4-amine (189 mg, 2.25 mmol), HATU (641 mg, 1.69 mmol), and DMA (5.0 mL). This was followed by the addition of DIPEA (0.59 mL, 4.54 mmol) dropwise at 0 °C. The resulting solution was stirred for 2 hours at RT. The reaction mixture was directly purified by reverse phase column chromatography (C18 column; 3:2 acetonitrile: water) to afford the title compound (370 mg, 70% yield) as a white solid.

ESI-MS m/z = 452.2 [M+H].

This mixture (370 mg) was resolved into the four stereoisomeric intermediates by two rounds of preparative chiral chromatography. The first separation provided pure isomer 1 and pure isomer 4. Isomers 2 and 3 were obtained as mixture and were separated in a second chiral separation.

First separation: Purification by chiral chromatography (Column: Regis (R, R)-Whelk-01; Mobile Phase A: hexanes (10mM NH3 in MeOH), Mobile Phase B: IPA; Gradient: 20% B to 20% B) afforded two pure isomers. Isomer 1 (1st-eluting peak, 60 mg) and Isomer 4 (3rd-eluting peak, 65 mg) as white solids. The other two isomers were obtained as a mixture (2nd eluting peak, 140 mg) and were separated in a second chiral separation.

Second separation: The 2nd-peak from the first separation (140 mg) was purified by chiral HPLC (Column: GreenSep Ethyl Pyridine II 120A; mobile phase: 70:30 hexanes (0.1% DEA):IPA) to afford Isomer 2 (1st eluting peak, 55 mg) and Isomer 3 (second eluting peak, 60 mg) as white solids.

Example 3 Isomer 1 : Into a 40-mL sealed tube, was placed 2-[1-(2-cyanophenyl)-1-phenylpropan-2-yl]-5-methoxy-1-methyl-N-(1,2-oxazol-4-yl)-6-oxopyrimidine-4-carboxamide (isomer 1) (60 mg, 0.13 mmol), lithium bromide (222 mg, 2.56 mmol), and DMF (3.0 mL). The resulting solution was stirred for 3 hours at 95 °C. The mixture was cooled to RT and the solids were filtered off. The crude product was purified by reverse phase HPLC (Column: Gemini-NX C18 AXAI Packed; Mobile Phase A: Water + 0.1% formic acid, Mobile Phase B: acetonitrile; Gradient: 32 to 70% B) to afford the title compound (25 mg, 43% yield) as a white solid.

¹H NMR (300 MHz, DMSO-d6) δ 11.17 (s, 1H), 10.54 (s, 1H), 9.34 (s, 1H), 8.92 (s, 1H), 8.19 - 8.17 (m, 1H), 7.84 - 7.79 (m, 2H), 7.50 - 7.44 (m, 1H), 7.23 - 7.07 (m, 5H), 4.97 (d, J = 11.0 Hz, 1H), 4.33 (dt, J = 11.0, 6.1 Hz, 1H), 3.42 (s, 3H), 1.31 (d, J = 6.4 Hz, 3H). ESI-MS m/z = 456.2 [M+H].

Example 3 Isomer 2 : Into a 40-mL sealed tube, was placed 2-[1-(2-cyanophenyl)-1-phenylpropan-2-yl]-5-methoxy-1-methyl-N-(1,2-oxazol-4-yl)-6-oxopyrimidine-4-carboxamide isomer 2 (55 mg, 0.12 mmol), lithium bromide (204 mg, 2.34 mmol), DMF (3.0 mL). The resulting solution was stirred for 3 hours at 95 °C. The mixture was cooled to RT and the solids were filtered off. The crude product was purified by reverse phase chromatography (Column: Gemini-NX C18 AXAI Packed; Mobile Phase A: Water + 0.1% formic acid, Mobile Phase B: acetonitrile; Gradient: 32 to 70% B) to afford the title compound (23 mg, 43% yield) as a white solid.

¹H NMR (300 MHz, DMSO-d6) δ 11.17 (s, 1H), 10.53 (s, 1H), 9.34 (s, 1H), 8.92 (s, 1H), 8.18 (d, J = 8.1 Hz, 1H), 7.82 (td, J = 7.1, 6.5, 1.5 Hz, 2H), 7.47 (t, J = 7.4 Hz, 1H), 7.23 - 7.14 (m, 4H), 7.09 - 7.04 (m, 1H), 4.96 (d, J = 11.0 Hz, 1H), 4.34 - 4.29 (m, 1H), 1.31 (d, J = 6.4 Hz, 3H). ESI-MS m/z = 456.2 [M+H].

Example 3, Isomer 3: Into a 40-mL sealed tube, was placed 2-[1-(2-cyanophenyl)-1-phenylpropan-2-yl]-5-methoxy-1-methyl-N-(1,2-oxazol-4-yl)-6-oxopyrimidine-4-carboxamide isomer 3 (60 mg, 0.13 mmol), lithium bromide (222 mg, 2.56 mmol), N,N dimethyl formamide (3.0 mL). The resulting solution was stirred for 3 hours at 95 °C. The mixture was cooled to RT and the solids were filtered off. The crude product was purified by reverse phase chromatography (Column: Gemini-NX C18 AXAI Packed; Mobile Phase A: Water + 0.1% formic acid, Mobile Phase B: acetonitrile; Gradient: 38 to 75% B) to afford the title compound (30 mg, 52% yield) as a white solid.

¹H NMR (300 MHz, DMSO-d6) δ 11.12 (s, 1H), 10.44 (s, 1H), 9.31 (s, 1H), 8.89 (s, 1H), 7.92 (d, J = 8.0 Hz, 1H), 7.72 - 7.54 (m, 4H), 7.40 (t, J = 7.5 Hz, 2H), 7.30 - 7.19 (m, 2H), 5.21 (d, J = 11.2 Hz, 1H), 4.33 (dt, J = 12.9, 6.5 Hz, 1H), 3.69 (s, 3H), 1.24 (d, J = 6.4 Hz, 3H). ESI-MS m/z = 456.2 [M+H].

Example 3, isomer 4: Into a 40-mL sealed tube, was placed 2-[1-(2-cyanophenyl)-1-phenylpropan-2-yl]-5-methoxy-1-methyl-N-(1,2-oxazol-4-yl)-6-oxopyrimidine-4-carboxamide isomer 4 (70 mg, 0.15 mmol), lithium bromide (259 mg, 2.98 mmol), DMF (3.5 mL). The resulting solution was stirred for 3 hours at 95 °C. The mixture was cooled to RT and the solids were filtered off. The crude product was purified by reverse phase chromatography (Column: Gemini-NX C18 AXAI Packed; Mobile Phase A: Water + 0.1% formic acid, Mobile Phase B: acetonitrile; Gradient: 35 to 73% B) to afford the title compound (35 mg, 51% yield) as a white solid.

¹H NMR (300 MHz, DMSO-d6) δ 11.11 (s, 1H), 10.44 (s, 1H), 9.31 (s, 1H), 8.89 (s, 1H), 7.92 (d, J = 8.1 Hz, 1H), 7.72 (d, J = 7.6 Hz, 2H), 7.61 - 7.56 (m, 2H), 7.40 (t, J = 7.5 Hz, 2H), 7.27 - 7.21 (m, 2H), 5.21 (d, J = 11.2 Hz, 1H), 4.30 (dd, J = 11.4, 6.5 Hz, 1H), 3.68 (s, 3H), 1.25 (d, J = 6.4 Hz, 3H). ESI-MS m/z = 456.2 [M+H].

### Example 4; Isomer 1, Isomer 2

### (S)-2-(1,1-bis(2-cyanophenyl) propan-2-yl)-N-(isoxazol-4-yl)-5-methoxy-1-methyl-6-oxo-1,6-dihydropyrimidine-4-carboxamide

### (R)-2-(1,1-bis(2-cyanophenyl) propan-2-yl)-N-(isoxazol-4-yl)-5-methoxy-1-methyl-6-oxo-1,6-dihydropyrimidine-4-carboxamide

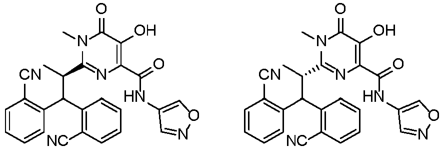

The intermediates necessary to prepare the title compounds were prepared following scheme A, steps 1, 2, and 3 in a manner similar to that described for Example 2 & 3.

The enantiomeric intermediates were then separated by preparative chiral HPLC ((Column: CHIRALPAK IG; Mobile Phase A: 5:1 hexanes:DCM + 0.1% DEA, Mobile Phase B: Ethanol; Gradient: 30% B isocratic) to afford the early-eluting enantiomer, (R)-2-(1,1-bis(2-cyanophenyl) propan-2-yl)-N-(isoxazol-4-yl)-5-methoxy-1-methyl-6-oxo-1,6-dihydropyrimidine-4-carboxamide (100 mg), and the late-eluting enantiomer, (S)-2-(1,1-bis(2-cyanophenyl) propan-2-yl)-N-(isoxazol-4-yl)-5-methoxy-1-methyl-6-oxo-1,6-dihydropyrimidine-4-carboxamide as white solids.

Example 4, Isomer 1: Into a 40-mL sealed tube, was placed (R)-2-(1,1-bis(2-cyanophenyl) propan-2-yl)-N-(isoxazol-4-yl)-5-methoxy-1-methyl-6-oxo-1,6-dihydropyrimidine-4-carboxamide (100 mg, 0.202 mmol), lithium bromide (350 mg, 4.00 mol), DMF (5.0 mL). The resulting solution was stirred for 3 hours at 95 °C. The mixture was cooled to RT and the solids were filtered off. The crude product was purified by preparative HPLC (Column: XB ridge Shield RP18 OBD Column; Mobile Phase A: water 0.05% formic acid, Mobile Phase B: ACN; Gradient: 45 60% B) to afford the title compound (25 mg, 25% yield) as an off-white solid.

¹H NMR (400 MHz, DMSO-d6) δ= 11.11 (s, 1H), 10.21 (s, 1H), 9.30 (s, 1H), 8.79 (s, 1H), 7.98 (d, J = 8.0 Hz, 1H), 7.92 (dd, J = 7.7, 1.6 Hz, 2H), 7.79 (td, J = 7.7, 1.5 Hz, 1H), 7.69 (dd, J = 8.0, 6.7 Hz, 2H), 7.53 (td, J = 7.6, 1.0 Hz, 1H), 7.36 (t, J = 7.6 Hz, 1H), 5.58 (d, J = 10.8 Hz, 1H), 4.35 (dq, J = 13.0, 6.6 Hz, 1H), 3.55 (s, 3H), 1.23 (d, J = 6.4 Hz, 3H). ESI-MS: m/z = 481.1 [M+H].

Example 4, Isomer 2: Into a 40-mL sealed tube, was placed (S)-2-(1,1-bis(2-cyanophenyl) propan-2-yl)-N-(isoxazol-4-yl)-5-methoxy-1-methyl-6-oxo-1,6-dihydropyrimidine-4-carboxamide (100 mg, 0.202 mmol), lithium bromide (350 mg, 4.03 mmol), DMF (5.0 mL). The resulting solution was stirred for 3 hours at 95 °C. The mixture was cooled to RT and solids were filtered off. The crude product was purified by preparative HPLC (Column: XB ridge Shield RP18 OBD Column; Mobile Phase A: water 0.05% formic acid, Mobile Phase B: ACN; Gradient: 45 60% B) to afford the title compound as an off-white solid (33 mg, 34% yield).

¹H NMR (400 MHz, DMSO-d6) δ = 11.11 (s, 1H), 10.21 (s, 1H), 9.30 (s, 1H), 8.79 (s, 1H), 7.98 (d, J = 8.0 Hz, 1H), 7.92 (dd, J = 7.7, 1.6 Hz, 2H), 7.79 (td, J = 7.7, 1.5 Hz, 1H), 7.69 (dd, J = 8.0, 6.7 Hz, 2H), 7.53 (td, J = 7.6, 1.0 Hz, 1H), 7.36 (t, J = 7.6 Hz, 1H), 5.58 (d, J = 10.8 Hz, 1H), 4.35 (dq, J = 13.0, 6.6 Hz, 1H), 3.53 (s, 3H), 1.21 (d, J = 6.4 Hz, 3H). ESI-MS m/z = 481.2 [M+ H].

### Example 5; Isomer 1, Isomer 2

### (S)-2-(1,1-bis(2-cyanophenyl)propan-2-yl)-5-hydroxy-N-(isoxazol-4-yl)-6-oxo-1,6-dihydropyrimidine-4-carboxamide

### (R)-2-(1,1-bis(2-cyanophenyl)propan-2-yl)-5-hydroxy-N-(isoxazol-4-yl)-6-oxo-1,6-dihydropyrimidine-4-carboxamide

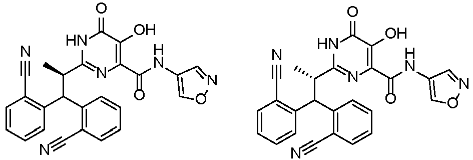

### Step 1

To a stirred solution of (2-[(2-cyanophenyl)methyl]benzonitrile) (9.07 g, 41.56 mmol, 1 equiv) in tetrahydrofuran was added potassium bis(trimethylsilyl)amide (1M in tetrahydrofuran) (45.72 mL, 45.72 mmol, 1.10 equiv) at -60 °C. The mixture was stirred for another 0.5 hour. The above solution was added to a solution of 2-bromopropanenitrile (11.14 g, 83.121 mmol, 2 equiv) in tetrahydrofuran (10 mL) at -78 °C under argon atmosphere. The resulting mixture was stirred overnight at room temperature. The reaction was quenched with saturated ammonium chloride (aq.) at 0°C. The aqueous layer was extracted with dichloromethane. The combined organic phase was washed with brine, dried over anhydrous sodium sulfate. After filtration, the filtrate was concentrated under reduced pressure. The residue was purified by silica gel column chromatography, eluted with dichloromethane / petroleum ether / Ethyl acetate (5:10:1) to afford 5.66 g of 2-[2-cyano-1-(2-cyanophenyl)-2-methylethyl]benzonitrile (yield 50%) as a light yellow solid.

¹H NMR (400 MHz, DMSO-d6) δ 7.91 - 7.87 (m, 2H), 7.82 - 7.75 (m, 2H), 7.73 (d, 1H), 7.67 (d, 1H), 7.56- 7.51 (m, 2H), 4.81 (d, 1H), 4.15-4.07 (m, 1H), 1.30 (d, 3H).

### Step 2

A mixture of 2-[2-cyano-1-(2-cyanophenyl)-2-methylethyl]benzonitrile (4.70 g, 17.32 mmol, 1 equiv) and hydroxylammonium (50% in water)(1.26 g, 19.05 mmol, 1.1 equiv) in methanol (70 mL) was stirred overnight at 50 °C. The mixture was concentrated under reduced pressure. The residue was dissolved with methanol (70 mL). To the above solution was added dimethyl acetylenedicarboxylate (9.85 g, 69.29 mmol, 4 equiv) dropwise at 0 °C. The resulting mixture was stirred for additional 1 hour at room temperature. The resulting mixture was concentrated under reduced pressure. The residue was purified by silica gel column chromatography, eluted with dichloromethane / petroleum ether / Ethyl acetate (5:10:3) to afford 2 g of (1,4-dimethyl 2-[[(Z)-[1-amino-2-[bis(2-cyanophenyl)methyl]propylidene]amino]oxy]but-2-enedioate) (yield 20%) as a brown yellow solid.

ESI-MS m/z = 447.3 [M+H] +. Calculated MW: 446.2

### Step 3

A solution of 1,4-dimethyl 2-[[(Z)-[1-amino-2-[bis(2-cyanophenyl)methyl]propylidene]amino]oxy]but-2-enedioate(1.54 g, 3.449 mmol, 1 equiv) in xylene(80 mL) stirred overnight at 140 °C under argon atmosphere. The mixture was allowed to cool down to room temperature. The resulting mixture was concentrated under reduced pressure. The residue was purified by Prep-HPLC with the following conditions (Column: SunFire Prep C18 OBD Column, 19*150 mm, 5µm 10nm; Mobile Phase A: Water(0.05%FA), Mobile Phase B: ACN; Flow rate: 25 mL/min; Gradient: 28% B to 57% B in 10 min, 57% B; Wave Length: 254/220 nm; RT1(min): 8.33;) to afford 275 mg of methyl 2-(1,1-bis(2-cyanophenyl)propan-2-yl)-5-hydroxy-6-oxo-1,6-dihydropyrimidine-4-carboxylate (yield 20%) as a yellow solid. ESI-MS m/z = 415.1 [M+H] +. Calculated MW: 414.1

### Chiral separation

methyl 2-(1,1-bis(2-cyanophenyl)propan-2-yl)-5-hydroxy-6-oxo-1,6-dihydropyrimidine-4-carboxylate (255 mg) was separated by Prep-chiral-HPLC with the following conditions: Column: CHIRALCEL OD-H, 2*25mm,5um; Mobile Phase A:Hex(0.1%TFA)--HPLC, Mobile Phase B:EtOH--HPLC; Flow rate:25 mL/min; Gradient:40 B to 40 B in 8 min; 205/308 nm; RT1:2.52; RT2:4.92; Injection Volume:0.3 mL;

The first peak (isomer1) (methyl 2-(1,1-bis(2-cyanophenyl)propan-2-yl)-5-hydroxy-6-oxo-1,6-dihydropyrimidine-4-carboxylate) was obtained 95 mg as an off-white solid.

The second peak (isomer2) methyl 2-(1,1-bis(2-cyanophenyl)propan-2-yl)-5-hydroxy-6-oxo-1,6-dihydropyrimidine-4-carboxylate was obtained 91 mg as an off-white solid.

### Example 5, Isomer 1

To a stirred mixture of methyl 2-[1,1-bis(2-cyanophenyl)propan-2-yl]-5-hydroxy-6-oxo-1H-pyrimidine-4-carboxylate isomer 1 (88 mg, 0.212 mmol, 1equiv) and 1,2-oxazol-4-amine(53.56 mg, 0.637 mmol, 3.equiv) in tetrahydrofuran (1.30 mL) was added isopropylmagnesium chloride (2M in tetrahydrofuran ) (0.53 mL, 1.06 mmol, 5.00 equiv) dropwise at -78 °C under argon atmosphere. The resulting mixture was stirred for 1h at 0°C under argon atmosphere. The mixture was acidified to pH=3 with 2M hydrochloric acid. The mixture was extracted with dichloromethane, washed with brine. The combined organic phase was concentrated under reduced pressure. The crude product was purified by Prep-HPLC with the following conditions Column: (Gemini-NX C18 AXAI Packed, 21.2*150mm 5um; Mobile Phase A:Water(0.05%FA), Mobile Phase B:ACN; Flow rate:25 mL/min; Gradient:31 B to 69 B in 7 min, 220/254 nm; RT1:6.28) to afford 30.2 mg of 2-(1,1-bis(2-cyanophenyl)propan-2-yl)-5-hydroxy-N-(isoxazol-4-yl)-6-oxo-1,6-dihydropyrimidine-4-carboxamide (yield 30%) as an off-white solid .

ESI-MS m/z = 467.3 [M+H] +. Calculated MW: 466.1

¹H NMR (400 MHz, DMSO-d6) δ 12.79 (s, 1H), 11.42 (s, 1H), 10.50 (s, 1H), 9.30 (s, 1H), 8.87 (s, 1H), 7.91 - 7.79 (m, 2H), 7.74 (d, 1H), 7.71 - 7.62 (m, 3H), 7.55-7.51 (m, 1H), 7.35 (s, 1H), 5.24 (d, 1H), 3.85-3.81 (m, 1H), 1.31 (d, 3H). ee value: 100%

### Example 5, Isomer 2

To a stirred mixture of methyl 2-[1,1-bis(2-cyanophenyl)propan-2-yl]-5-hydroxy-6-oxo-1H-pyrimidine-4-carboxylate isomer 2 (89.00 mg, 0.215 mmol, 1 equiv) and 1,2-oxazol-4-amine(54.17 mg, 0.645 mmol, 3 equiv) in tetrahydrofuran (1.30 mL) was added isopropylmagnesium chloride (2M in tetrahydrofuran ) (0.54 mL, 1.08 mmol, 5.00 equiv) dropwise at -78 °C under argon atmosphere. The resulting mixture was stirred for 1h at 0 °C under argon atmosphere. The mixture was acidified to pH=3 with 2M hydrochloric acid. The mixture was extracted with dichloromethane, washed with brine. The combined organic phase was concentrated under reduced pressure. The crude product was purified by Prep-HPLC with the following conditions Column: ( Gemini-NX C18 AXAI Packed, 21.2*150mm 5um; Mobile Phase A:Water(0.05%FA), Mobile Phase B:ACN; Flow rate:25 mL/min; Gradient:31 B to 69 B in 7 min; 220/254 nm; RT1:6.28) to afford 36.0 mg of 2-(1,1-bis(2-cyanophenyl)propan-2-yl)-5-hydroxy-N-(isoxazol-4-yl)-6-oxo-1,6-dihydropyrimidine-4-carboxamide (yield 36%) as an off-white solid .

ESI-MS m/z = 467.3 [M+H] +. Calculated MW: 466.1

¹H NMR (400 MHz, DMSO-d6) δ 12.79 (s, 1H), 11.42 (s, 1H), 10.50 (s, 1H), 9.30 (s, 1H), 8.87 (s, 1H), 7.89 - 7.79 (m, 2H), 7.74 (d, 1H), 7.71 - 7.62 (m, 3H), 7.55-7.51 (m, 1H), 7.35 (s, 1H), 5.24 (d, 1H), 3.85-3.81 (m, 1H), 1.31 (d, 3H). ee value: 98.4%

### Example 6; Isomer 1, Isomer 2, Isomer 3, Isomer 4

### 2-((1R,2R)-1-(2-cyano-5-fluorophenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-cyano-5-fluorophenyl)-1-phenylpropan-2-yl)-5-hydroxy-N-(isoxazol-4-yl)-1-methyl-6-oxo-1,6-dihydropyrimidine-4-carboxamide

### 2-((1S,2R)-1-(2-cyano-5-fluorophenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(2-cyano-5-fluorophenyl)-1-phenylpropan-2-yl)-5-hydroxy-N-(isoxazol-4-yl)-1-methyl-6-oxo-1,6-dihydropyrimidine-4-carboxamide

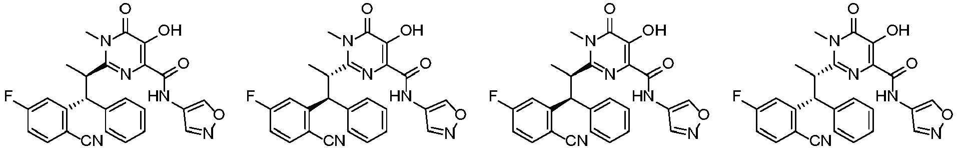

The intermediates necessary to prepare the title compounds were prepared following scheme A, steps 1, 2, and 3 in a manner similar to that described for Example 2 & 3.

The intermediate, 2-(1-(2-cyano-5-fluorophenyl)-1-phenylpropan-2-yl)-N-(isoxazol-4-yl)-5-methoxy-1-methyl-6-oxo-1,6-dihydropyrimidine-4-carboxamide (524 mg) was separated into its four stereoisomers by preparative chiral HPLC (Column: CHIRAL ART Cellulose-SB; Mobile Phase A: 5:1 hexanes: DCM + 0.1% DEA), Mobile Phase B: EtOH; Gradient: 10% B isocratic) to afford the 1st-eluting stereoisomer (73 mg), the 2nd-eluting stereoisomer (63 mg), the 3rd-eluting stereoisomer (91 mg), and the 4th-eluting stereoisomer (85 mg).

Example 6, Isomer 1: Into a 40 mL vial were added (2-(1-(2-cyano-5-fluorophenyl)-1-phenylpropan-2-yl)-N-(isoxazol-4-yl)-5-methoxy-1-methyl-6-oxo-1,6-dihydropyrimidine-4-carboxamide isomer 1 (73 mg, 0.150 mmol), lithium bromide (260 mg, 2.99 mmol) and DMF (4.0 mL) at 25 °C. The resulting mixture was stirred for 1h at 95°C. The reaction was cooled to RT and directly purified by preparative HPLC (Column: SunFire Prep C18 OBD Column; Mobile Phase A: water + 0.1% formic acid, Mobile Phase B: ACN; Gradient: 49 to 61% B) to afford the title compound (31 mg, 43% yield) as a white solid.

¹H NMR (400 MHz, Chloroform-d) δ 11.25 (s, 1H), 9.59 (s, 1H), 9.13 (s, 1H), 8.74 (s, 1H), 7.76 (dd, J = 8.6, 5.4 Hz, 1H), 7.29 (dd, J = 6.9, 1.5 Hz, 4H), 7.26 - 7.17 (m, 2H), 7.11 (ddd, J = 8.6, 7.7, 2.5 Hz, 1H), 5.60 - 5.49 (m, 1H), 3.93 - 3.80 (m, 1H), 3.66 (s, 3H), 1.16 (d, J = 6.8 Hz, 3H). ESI-MS: m/z = 473.8 [M+H].

Example 6, Isomer 2: Into a 40 mL vial were added 2-(1-(2-cyano-5-fluorophenyl)-1-phenylpropan-2-yl)-N-(isoxazol-4-yl)-5-methoxy-1-methyl-6-oxo-1,6-dihydropyrimidine-4-carboxamide isomer 2 (63 mg, 0.129 mmol), lithium bromide (224 mg, 2.59 mmol) and DMF (4.0 mL) at 25 °C. The resulting mixture was stirred for 1h at 95°C. The reaction was cooled to RT and directly purified by preparative HPLC (Column: SunFire Prep C18 OBD Column; Mobile Phase A: water + 0.1% formic acid, Mobile Phase B: ACN; Gradient: 49 to 69% B) to afford the title compound (19 mg, 31% yield) as a white solid.

¹H NMR (400 MHz, Chloroform-d) δ 11.25 (s, 1H), 9.59 (s, 1H), 9.13 (s, 1H), 8.74 (s, 1H), 7.76 (dd, J = 8.6, 5.4 Hz, 1H), 7.33 - 7.26 (m, 4H), 7.25 - 7.17 (m, 2H), 7.11 (ddd, J = 8.5, 7.6, 2.5 Hz, 1H), 5.56 (d, J = 10.6 Hz, 1H), 3.88 (dq, J = 13.7, 7.0 Hz, 1H), 3.66 (s, 3H), 1.16 (d, J = 6.8 Hz, 3H). ESI-MS: m/z = 473.8 [M+H].

Example 6, Isomer 3: Into a 40 mL vial were added 2-(1-(2-cyano-5-fluorophenyl)-1-phenylpropan-2-yl)-N-(isoxazol-4-yl)-5-methoxy-1-methyl-6-oxo-1,6-dihydropyrimidine-4-carboxamide isomer 3 (91 mg, 0.187 mmol), lithium bromide (324 mg, 3.73 mmol) and DMF (4.0 mL) at 25 °C. The resulting mixture was stirred for 1h at 95°C. The reaction was cooled to RT and directly purified by preparative HPLC (Column: SunFire Prep C18 OBD Column; Mobile Phase A: water + 0.1% formic acid, Mobile Phase B: ACN; Gradient: 49 to 69% B) to afford the title compound (23 mg, 26% yield) as a white solid.

¹H NMR (400 MHz, DMSO-d6) δ 11.13 (s, 1H), 10.44 (s, 1H), 9.31 (s, 1H), 8.89 (s, 1H), 7.91 (dd, J = 10.5, 2.5 Hz, 1H), 7.73 (t, J = 7.8 Hz, 3H), 7.42 (t, J = 7.5 Hz, 2H), 7.29 (t, J = 7.3 Hz, 1H), 7.09 (td, J = 8.4, 2.5 Hz, 1H), 5.26 (d, J = 11.1 Hz, 1H), 4.29 (dq, J = 13.2, 6.7 Hz, 1H), 3.71 (s, 3H), 1.21 (d, J = 6.6 Hz, 3H). ESI-MS: m/z = 473.8 [M+H].

Example 6, Isomer 4: Into a 40 mL vial were added 2-(1-(2-cyano-5-fluorophenyl)-1-phenylpropan-2-yl)-N-(isoxazol-4-yl)-5-methoxy-1-methyl-6-oxo-1,6-dihydropyrimidine-4-carboxamide isomer 4 (85 mg, 0.174 mmol), lithium bromide (303 mg, 3.49 mmol) and DMF (4.00 mL) at 25 °C. The resulting mixture was stirred for 1h at 95°C. The reaction was cooled to RT and directly purified by preparative HPLC (Column: SunFire Prep C18 OBD Column; Mobile Phase A: water + 0.1% formic acid, Mobile Phase B: ACN; Gradient: 49 to 69% B) to afford the title compound (29 mg, 35% yield) as a white solid.

¹H NMR (400 MHz, DMSO-d6) δ 11.06 (s, 1H), 10.38 (s, 1H), 9.24 (s, 1H), 8.82 (s, 1H), 7.84 (dd, J = 10.4, 2.5 Hz, 1H), 7.67 (d, J = 7.5 Hz, 3H), 7.35 (t, J = 7.5 Hz, 2H), 7.22 (t, J = 7.3 Hz, 1H), 7.02 (td, J = 8.4, 2.4 Hz, 1H), 5.19 (d, J = 11.1 Hz, 1H), 4.22 (dq, J = 12.9, 6.5 Hz, 1H), 3.64 (s, 3H), 1.14 (d, J = 6.5 Hz, 3H). ESI-MS: m/z = 473.8 [M+H].

### Example 7; Isomer 1, Isomer 2, Isomer 3, Isomer 4

### 2-((1S,2R)-1-(2-cyano-4-fluorophenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(2-cyano-4-fluorophenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(2-cyano-4-fluorophenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-cyano-4-fluorophenyl)-1-phenylpropan-2-yl)-5-hydroxy-N-(isoxazol-4-yl)-1-methyl-6-oxo-1,6-dihydropyrimidine-4-carboxamide

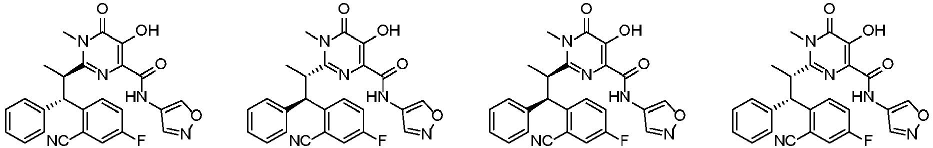

The intermediates necessary to prepare the title compounds were prepared following scheme A, steps 1, 2, and 3 in a manner similar to that described for Example 2 & 3.

2-[1-(2-Cyano-4-fluorophenyl)-1-phenylpropan-2-yl]-5-methoxy-1-methyl-N-(1,2-oxazol-4-yl)-6-oxopyrimidine-4-carboxamide (750 mg) was separated into its four stereoisomers via two rounds of preparative chiral HPLC with the following conditions. The first separation yielded the 1st-eluting stereoisomer and the 4th-eluting stereoisomer as pure compounds and the 2nd-eluting and 3rd-eluting stereoisomers were obtained as mixture, which was separated in the second round of chiral purification.

The first chiral separation. (Column: CHIRAL ART Cellulose-SB; Mobile Phase A: 1:1 hexanes: MTBE=1:1 + 0.5% 2M NH3-MeOH, Mobile Phase B: EtOH; Gradient: 10% B isocratic) afforded the 1st-eluting stereoisomer (150 mg), a mixture of the 2nd-eluting and 3rd-eluting stereoisomers (330 mg), and the 4th-eluting stereoisomer (130 mg) as white solids.

The second chiral separation. The mixture of the 2nd-eluting and 3rd-eluting stereoisomers (330 mg) was separated by preparative chiral-HPLC (Column: CHIRAL ART Cellulose-SC; Mobile Phase A: hexanes (10mM NH₃-MeOH), Mobile Phase B: EtOH; Gradient: 50% B isocratic) to afford the 2nd-eluting stereoisomer (140 mg) and the 3rd-eluting stereoisomer (100 mg) as white solids.

Example 7, Isomer 1: A solution of 2-[1-(2-cyano-4-fluorophenyl)-1-phenylpropan-2-yl]-5-methoxy-1-methyl-N-(1,2-oxazol-4-yl)-6-oxopyrimidine-4-carboxamide isomer 1 (120.0 mg, 0.246 mmol) and lithium bromide (321 mg, 3.69 mmol) in DMF (6.0 mL) was stirred for 3 hours at 95 °C under an atmosphere of nitrogen. The mixture was cooled to RT and directly purified by preparative HPLC (Column: XSelect CSH Prep C18 OBD; Mobile Phase A: water + 0.1% formic acid, Mobile Phase B: acetonitrile; Gradient: 44 to 61% B) to afford the title compound (62 mg, 52% yield) as a white solid.

¹H NMR (400 MHz, DMSO-d6) δ 11.17 (s, 1H), 10.61 (s, 1H), 9.34 (s, 1H), 8.91 (s, 1H), 8.23 (dd, J = 9.0, 5.3 Hz, 1H), 7.84 (dd, J = 8.6, 2.9 Hz, 1H), 7.73 (td, J = 8.7, 2.9 Hz, 1H), 7.24 - 7.13 (m, 4H), 7.07 (t, J = 7.0 Hz, 1H), 4.95 (d, J = 10.9 Hz, 1H), 4.29 (dd, J = 11.2, 6.4 Hz, 1H), 3.42 (s, 3H), 1.30 (d, J = 6.5 Hz, 3H). ESI-MS: m/z = 471.8 [M-H].

Example 7, Isomer 2: A solution of 2-[1-(2-cyano-4-fluorophenyl)-1-phenylpropan-2-yl]-5-methoxy-1-methyl-N-(1,2-oxazol-4-yl)-6-oxopyrimidine-4-carboxamide isomer 2 (100 mg, 0.205 mmol) and lithium bromide (267 mg, 3.08 mmol) in DMF (5.0 mL) was stirred for 4 hours at 95 °C under an atmosphere of nitrogen. The mixture was cooled to RT and directly purified by preparative HPLC (Column: XSelect CSH Prep C18 OBD; Mobile Phase A: water + 0.1% formic acid, Mobile Phase B: acetonitrile; Gradient: 39 to 68% B) to afford the title compound (36.8 mg, 37% yield) as a white solid.

¹H NMR (400 MHz, DMSO-d6) δ 11.17 (s, 1H), 10.57 (s, 1H), 9.34 (s, 1H), 8.91 (s, 1H), 8.23 (dd, J = 8.9, 5.3 Hz, 1H), 7.84 (dd, J = 8.6, 2.8 Hz, 1H), 7.73 (td, J = 8.6, 2.9 Hz, 1H), 7.24 - 7.13 (m, 4H), 7.08 (t, J = 7.0 Hz, 1H), 4.95 (d, J = 10.9 Hz, 1H), 4.29 (dd, J = 11.1, 6.4 Hz, 1H), 3.42 (s, 3H), 1.30 (d, J = 6.5 Hz, 3H). ESI-MS: m/z = 474.1 [M+H].

Example 7, Isomer 3: A solution of 2-[1-(2-cyano-4-fluorophenyl)-1-phenylpropan-2-yl]-5-methoxy-1-methyl-N-(1,2-oxazol-4-yl)-6-oxopyrimidine-4-carboxamide isomer 3 (100 mg, 0.205 mmol) and lithium bromide (267 mg, 3.08 mmol) in DMF (5.0 mL) was stirred for 4 hours at 95 °C under an atmosphere of nitrogen. The mixture was cooled to RT and directly purified by preparative HPLC (Column: XSelect CSH Prep C18 OBD; Mobile Phase A: water + 0.1% formic acid, Mobile Phase B: acetonitrile; Gradient: 39 to 68% B) to afford the title compound (56 mg, 57% yield) as a white solid.

¹H NMR (400 MHz, DMSO-d6) δ 11.14 (s, 1H), 10.57 (s, 1H), 9.30 (s, 1H), 8.87 (s, 1H), 7.97 (dd, J = 8.9, 5.2 Hz, 1H), 7.69 (d, J = 7.6 Hz, 2H), 7.61 (dd, J = 8.6, 2.9 Hz, 1H), 7.50 - 7.41 (m, 1H), 7.40 (t, J = 7.6 Hz, 2H), 7.28 (t, J = 7.3 Hz, 1H), 5.20 (d, J = 11.2 Hz, 1H), 4.26 (dd, J = 11.3, 6.4 Hz, 1H), 3.68 (s, 3H), 1.22 (d, J = 6.5 Hz, 3H). ESI-MS: m/z = 474.1 [M+H].

Example 7, Isomer 4: A solution of 2-[1-(2-cyano-4-fluorophenyl)-1-phenylpropan-2-yl]-5-methoxy-1-methyl-N-(1,2-oxazol-4-yl)-6-oxopyrimidine-4-carboxamide isomer 4 (120 mg, 0.246 mmol) and lithium bromide (321 mg, 3.69 mmol) in DMF (6.0 mL) was stirred for 3 hours at 95 °C under an atmosphere of nitrogen. The mixture was cooled to RT and directly purified by preparative HPLC (Column: XSelect CSH Prep C18 OBD; Mobile Phase A: water + 0.1% formic acid, Mobile Phase B: acetonitrile; Gradient: 43 to 63% B) to afford the title compound (64 mg, 55% yield) as a white solid.

¹H NMR (400 MHz, DMSO-d6) δ 11.14 (s, 1H), 10.54 (s, 1H), 9.31 (s, 1H), 8.87 (s, 1H), 7.97 (dd, J = 8.9, 5.2 Hz, 1H), 7.70 (d, J = 7.6 Hz, 2H), 7.61 (dd, J = 8.6, 2.8 Hz, 1H), 7.46 (td, J = 8.6, 2.9 Hz, 1H), 7.40 (t, J = 7.6 Hz, 2H), 7.28 (t, J = 7.4 Hz, 1H), 5.20 (d, J = 11.2 Hz, 1H), 4.27 (dd, J = 11.3, 6.5 Hz, 1H), 3.68 (s, 3H), 1.22 (d, J = 6.5 Hz, 3H). ESI-MS: m/z = 471.9 [M-H].

### Example 8; Isomer 1, Isomer 2, Isomer 3, Isomer 4

### 2-((1S,2S)-1-(5-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(5-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1S,2R)-1-(5-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(5-chloro-2-cyanophenyl)-1-phenylpropan-2-yl)-5-hydroxy-N-(isoxazol-4-yl)-1-methyl-6-oxo-1,6-dihydropyrimidine-4-carboxamide

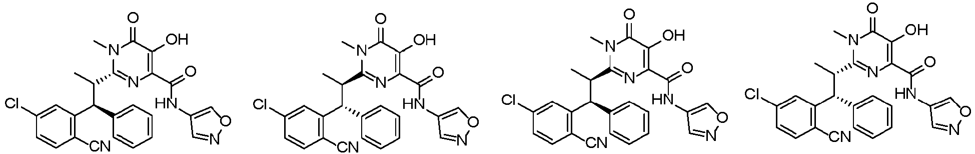

The intermediates necessary to prepare the title compounds were prepared following scheme A, steps 1, 2, and 3 in a manner similar to that described for Example 2 & 3.

The diastereomers were then separated by preparative HPLC (Column: XSelect CSH Prep Fluoro-phenyl OBD Column; Mobile Phase A: water + 0.1% formic acid, Mobile Phase B: MeOH; Gradient: 20% MeOH isocratic). The early-eluting diastereomer was a mixture of 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 1 and 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 2 (240 mg). ESI-MS: m/z = 504.1 [M+H]. The late-eluting diastereomer was a mixture of 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 3 and 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 4 (210 mg). ESI-MS: m/z = 504.1 [M+H].

The early-eluting diastereomer was then resolved by preparative chiral HPLC (Column: CHIRAL ART Cellulose-SB; Mobile Phase A: hexanes (10mM NH3-MeOH), Mobile Phase B:EtOH; Gradient: 15% B isocratic) to afford the early-eluting enantiomer, isomer 1 (79 mg) and the late-eluting enantiomer, isomer 2 (55 mg) as white solids.

The late-eluting diastereomer was then resolved by preparative SFC chiral HPLC (Column: CHIRAL ART; Mobile Phase A: hexanes (10mM NH3-MeOH), Mobile Phase B:EtOH; Gradient: 25% B isocratic) to afford the early-eluting enantiomer, isomer 3 (72 mg) and the late-eluting enantiomer, isomer 4 (80 mg) as white solids.

Example 8, Isomer 1: Into a 40 mL vial were added 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 1 (79 mg, 0.157 mmol), lithium bromide (272 mg, 3.14 mmol) and DMF (4.0 mL) at 95 °C. The resulting mixture was stirred for 2 hours at 95 °C. The crude product was purified by preparative HPLC (Column: SunFire Prep C18 OBD Column; Mobile Phase A: water + 0.1% formic acid, Mobile Phase B: ACN; Gradient: 50 to 68% B) to afford the title compound (36 mg, 47% yield) as a white solid.

¹H NMR (300 MHz, DMSO-d6) δ 11.14 (s, 1H), 10.50 (s, 1H), 9.31 (s, 1H), 8.88 (s, 1H), 8.09 (d, J = 2.0 Hz, 1H), 7.75 (d, J = 7.6 Hz, 2H), 7.67 (d, J = 8.3 Hz, 1H), 7.43 (t, J = 7.6 Hz, 2H), 7.33 - 7.19 (m, 2H), 5.23 (d, J = 11.1 Hz, 1H), 4.35 (dt, J = 11.4, 6.4 Hz, 1H), 3.70 (s, 3H), 1.23 (d, J = 6.5 Hz, 3H). ESI-MS: m/z = 489.9 [M+H].

Example 8, Isomer 2: Into a 40 mL vial were added 2-(1-(5-chloro-2-cyanophenyl)-1-phenylpropan-2-yl)-5-methoxy-N-(isoxazol-4-yl)-1-methyl-6-oxo-1,6-dihydropyrimidine-4-carboxamide isomer 2 (55 mg, 0.109 mmol), lithium bromide (190 mg, 2.18 mmol) and DMF (3.00 mL) at 95 °C. The resulting mixture was stirred for 2 hours at 95 °C. The crude product was purified by preparative HPLC (Column: SunFire Prep C18 OBD Column; Mobile Phase A: water + 0.1% formic acid, Mobile Phase B: ACN; Gradient: 50 to 68% B) to afford the title compound (41 mg, 76% yield) as a white solid.

¹H NMR (300 MHz, DMSO-d6) δ 11.13 (s, 1H), 10.63 (s, 1H), 9.30 (s, 1H), 8.87 (s, 1H), 8.08 (d, J = 1.9 Hz, 1H), 7.75 (d, J = 7.6 Hz, 2H), 7.66 (d, J = 8.3 Hz, 1H), 7.43 (t, J = 7.5 Hz, 2H), 7.36 - 7.20 (m, 2H), 5.21 (d, J = 11.1 Hz, 1H), 4.33 (dd, J = 11.3, 6.5 Hz, 1H), 3.69 (s, 3H), 1.22 (d, J = 6.5 Hz, 3H). ESI-MS: m/z = 489.9 [M+H].

Example 8, Isomer 3: Into a 40 mL vial were added 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 3 (72 mg, 0.143 mmol), lithium bromide (248.16 mg, 2.858 mmol, 20.00 equiv) and DMF (4.0 mL) at 95 °C. The resulting mixture was stirred for 2 hours at 95 °C. The crude product was purified by preparative HPLC (Column: XSelect CSH Prep C18 OBD Column; Mobile Phase A: water + 0.1% formic acid, Mobile Phase B: CAN; Gradient: 42 to 63% B) to afford the title compound (39 mg, 55% yield) as a white solid.

¹H NMR (300 MHz, DMSO-d6) δ 11.14 (s, 1H), 10.61 (s, 1H), 9.34 (s, 1H), 8.92 (s, 1H), 8.37 (d, J = 2.0 Hz, 1H), 7.88 (d, J = 8.3 Hz, 1H), 7.57 (dd, J = 8.3, 2.0 Hz, 1H), 7.28 - 7.12 (m, 4H), 7.12 - 7.03 (m, 1H), 4.98 (d, J = 11.0 Hz, 1H), 4.37 (dq, J = 12.6, 6.3 Hz, 1H), 3.45 (s, 3H), 1.36 - 1.27 (m, 3H), 1.25 (d, J = 4.3 Hz, 1H). ESI-MS: m/z = 489.9 [M+H].

Example 8, Isomer 4: Into a 40 mL vial were added 2-(1-(5-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 4 (80 mg, 0.159 mmol), lithium bromide (276 mg, 3.18 mmol) and DMF (4.0 mL) at 95 °C. The resulting mixture was stirred for 2 hours at 95 °C. The crude product was purified by preparative HPLC (Column: XSelect CSH Prep C18 OBD Column; Mobile Phase A: water + 0.1% formic acid, Mobile Phase B: ACN; Gradient:42 to 63% B) to afford the title compound (27 mg, 35% yield) as a white solid.

¹H NMR (300 MHz, DMSO-d6) δ 11.18 (s, 1H), 10.60 (s, 1H), 9.34 (s, 1H), 8.92 (s, 1H), 8.36 (d, J = 2.0 Hz, 1H), 7.88 (d, J = 8.3 Hz, 1H), 7.57 (dd, J = 8.3, 1.9 Hz, 1H), 7.28 - 7.14 (m, 4H), 7.09 (t, J = 7.1 Hz, 1H), 4.98 (d, J = 11.0 Hz, 1H), 4.37 (dq, J = 12.7, 6.4 Hz, 1H), 3.45 (s, 3H), 1.32 (d, J = 6.3 Hz, 3H). ESI-MS: m/z = 489.9 [M+H].

### Example 9; Isomer 1, Isomer 2, Isomer 3, Isomer 4

### 2-((1S,2S)-1-(4-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(4-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1S,2R)-1-(4-chloro-2-cyanophenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(4-chloro-2-cyanophenyl)-1-phenylpropan-2-yl)-5-hydroxy-N-(isoxazol-4-yl)-1-methyl-6-oxo-1,6-dihydropyrimidine-4-carboxamide

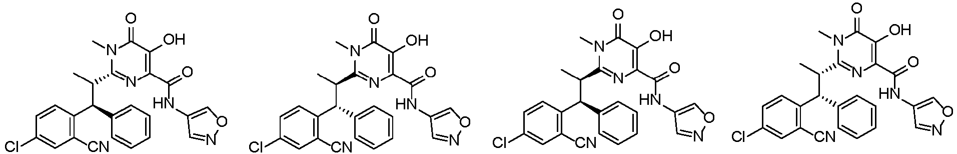

The intermediates necessary to prepare the title compounds were prepared following scheme A, steps 1, 2, and 3 in a manner similar to that described for Example 2 & 3.

The mixture of four stereoisomers (600 mg) was resolved into the four pure enantiomers by two rounds of chiral chromatography. The first round (Column: CHIRAL ART Cellulose-SB; Mobile Phase A: hex anes (10mM NH3-MeOH), Mobile Phase B: EtOH; Gradient:30% B isocratic) afforded 2-(1-(4-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 1 (1st-eluting isomer; 126 mg) and (2-(1-(4-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 4 (4th-eluting isomer; 110mg) as off-white solids. The middle peak containing the other two stereoisomers (260 mg) was purified again by chiral chromatograph (Column: CHIRALPAK IC; Mobile Phase A: (1: 1 hexanes: MTBE + 0.1% DEA), Mobile Phase B: EtOH; Gradient: 30% B isocratic) to afford 2-(1-(4-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 2 (1st eluting isomer; 84 mg) and 2-((1S,2S)-1-(4-chloro-2-cyanophenyl)-1-phenylpropan-2-yl)-N-(isoxazol-4-yl)-5-methoxy-1-methyl-6-oxo-1,6-dihydropyrimidine-4-carboxamide isomer 3 2nd-eluting isomer; 65 mg) as off-white solids. ESI-MS: m/z = 504.2 [M+H].

Example 9, Isomer 1: To a stirred mixture of 2-(1-(4-chloro-2-cyanophenyl)-1-phenylpropan-2-yl)-5-methoxy-N-(isoxazol-4-yl)-1-methyl-6-oxo-1,6-dihydropyrimidine-4-carboxamide isomer 1 (120 mg, 0.238 mmol) and DMF (6.00 mL) was added LiBr (414 mg, 4.762 mmol). The resulting mixture was stirred for 2 hours at 95 °C. The crude product was purified by preparative HPLC (Column: Gemini-NX C18 AXAI Packed; Mobile Phase A: water + 0.1% formic acid), Mobile Phase B: ACN; Gradient: 5 to 52% B) to afford the title compound (82 mg) as an off-white solid. ESI-MS: m/z = 488.0 [M-H].

Example 9, Isomer 2: To a stirred mixture of 2-(1-(4-chloro-2-cyanophenyl)-1-phenylpropan-2-yl)-5-methoxy-N-(isoxazol-4-yl)-1-methyl-6-oxo-1,6-dihydropyrimidine-4-carboxamide isomer 2 (84 mg, 0.167 mmol) and DMF (4.50 mL) was added LiBr (290 mg, 3.34 mmol). The resulting mixture was stirred for 2 hours at 95 °C. The crude product was purified by preparative HPLC (Column: XBridge Shield RP18 OBD Column; Mobile Phase A: water + 0.1% formic acid, Mobile Phase B: ACN; Gradient: 45 to 65% B) to afford the title compound (54 mg) as an off-white solid. ESI-MS: m/z = 488.0 [M-H].

Example 9, Isomer 3: To a stirred mixture of 2-(1-(4-chloro-2-cyanophenyl)-1-phenylpropan-2-yl)-5-methoxy-N-(isoxazol-4-yl)-1-methyl-6-oxo-1,6-dihydropyrimidine-4-carboxamide isomer 3 (65 mg, 0.129 mmol) and DMF (3.48 mL) was added LiBr (224 mg, 2.58 mmol). The resulting mixture was stirred for 2 hours at 95 °C. The crude product was purified by preparative HPLC with the following conditions (Column: XBridge Shield RP18 OBD Column; Mobile Phase A: Water + 0.1% formic acid, Mobile Phase B: ACN; Gradient: 45 to 70% B) to afford the title compound (44 mg) as an off-white solid. ESI-MS: m/z = 488.0 [M-H].

Example 9, Isomer 4: To a stirred mixture of 2-(1-(4-chloro-2-cyanophenyl)-1-phenylpropan-2-yl)-5-methoxy-N-(isoxazol-4-yl)-1-methyl-6-oxo-1,6-dihydropyrimidine-4-carboxamide isomer 4 (110 mg, 0.218 mmol) and DMF (5.50 mL) was added LiBr (379 mg, 4.36 mmol). The resulting mixture was stirred for 2 hours at 95 °C. The crude product was purified by preparative HPLC (Column: YMC-Actus Triart C18; Mobile Phase A: water + 0.1% formic acid), Mobile Phase B: ACN; Gradient: 5 to 50% B) to afford the title compound (57 mg) as an off-white solid. ESI-MS: m/z = 488.0 [M-H].

### Example 10; Isomer 1, Isomer 2, Isomer 3, Isomer 4

### 2-((1R,2R)-1-(2-chlorophenyl)-1-(2-cyanophenyl)propan-2-yl)-5-hydroxy-N-(isoxazol-4-yl)-1-methyl-6-oxo-1,6-dihydropyrimidine-4-carboxamide

### 2-((1S,2S)-1-(2-chlorophenyl)-1-(2-cyanophenyl)propan-2-yl)-5-hydroxy-N-(isoxazol-4-yl)-1-methyl-6-oxo-1,6-dihydropyrimidine-4-carboxamide

### 2-((1S,2R)-1-(2-chlorophenyl)-1-(2-cyanophenyl)propan-2-yl)-5-hydroxy-N-(isoxazol-4-yl)-1-methyl-6-oxo-1,6-dihydropyrimidine-4-carboxamide

### 2-((1R,2S)-1-(2-chlorophenyl)-1-(2-cyanophenyl)propan-2-yl)-5-hydroxy-N-(isoxazol-4-yl)-1-methyl-6-oxo-1,6-dihydropyrimidine-4-carboxamide

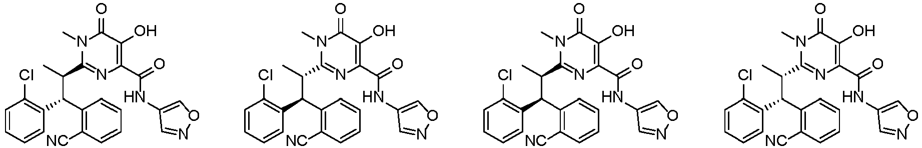

A mixture of the four stereoisomers of 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide was prepared following scheme A, steps 1, 2, and 3 in a manner similar to that described for Example 2 & 3.

The diastereomers were then separated by preparative HPLC (Column: XSelect CSH Prep Fluoro-phenyl OBD Column; Mobile Phase A: Water + 0.1% formic acid, Mobile Phase B: MeOH; Gradient: 5% to 59% MeOH). The early-eluting peak was a mixture of 2-(1-(2-chlorophenyl)-1-(2-cyanophenyl)propan-2-yl)-N-(isoxazol-4-yl)-5-methoxy-1-methyl-6-oxo-1,6-dihydropyrimidine-4-carboxamide isomer 1 and 2-(1-(2-chlorophenyl)-1-(2-cyanophenyl)propan-2-yl)-N-(isoxazol-4-yl)-5-methoxy-1-methyl-6-oxo-1,6-dihydropyrimidine-4-carboxamide isomer 2 (190 mg). ESI-MS m/z = 503.9 [M+H]. The late-eluting peak was a mixture of 2-(1-(2-chlorophenyl)-1-(2-cyanophenyl)propan-2-yl)-N-(isoxazol-4-yl)-5-methoxy-1-methyl-6-oxo-1,6-dihydropyrimidine-4-carboxamide isomer 3 and 2-(1-(2-chlorophenyl)-1-(2-cyanophenyl)propan-2-yl)-N-(isoxazol-4-yl)-5-methoxy-1-methyl-6-oxo-1,6-dihydropyrimidine-4-carboxamideisomer 4 (220 mg). ESI-MS m/z = 503.9 [M+H].

The early-eluting diastereomer was then resolved by preparative chiral HPLC (Column: CHIRALPAK IC-3; Mobile Phase: 70:30 (1:1 Hexane:MTBE + 0.1% diethylamine):EtOH) to afford the enantiomeric intermediates, isomer 1 (early-eluting enantiomer, 73 mg) and isomer 2 (late-eluting enantiomer, 68 mg) as white solids.

The late-eluting diastereomer was then resolved by preparative chiral HPLC (Column: CHIRALPAK ID-3; Mobile Phase A: 90:10 (1:1 Hexane:MTBE + 0.1%DEA):EtOH) to afford the enantiomeric intermediates, isomer 3 (early-eluting enantiomer, 70 mg) and isomer 4 (late-eluting enantiomer, 60 mg) as white solids.

Example 10, Isomer 1: Into a 40-mL sealed tube were added (2-[1-(2-chlorophenyl)-1-(2-cyanophenyl)propan-2-yl]-5-methoxy-1-methyl-N-(1,2-oxazol-4-yl)-6-oxopyrimidine-4-carboxamide isomer 1 (73 mg, 0.145 mmol), lithium bromide (189 mg, 2.17 mmol) and DMF (3.0 mL) at RT and stirred for 2 h at 95 °C under and atmosphere of argon. The mixture was cooled to RT. The crude product was purified by preparative HPLC (Column: XSelect CSH Prep C18 OBD Column; Mobile Phase A: Water + 0.1% formic acid), Mobile Phase B: acetonitrile; Gradient: 47 to 63% B) to afford the title compound (11 mg; 16% yield) as a white solid.

¹H NMR (400 MHz, DMSO-d6) δ 11.39 (s, 1H), 10.37 (s, 1H), 9.31 (s, 1H), 8.88 (s, 1H), 8.14 - 8.08 (m, 1H), 7.71 (d, J = 8.0 Hz, 1H), 7.64 - 7.54 (m, 2H), 7.51 - 7.45 (m, 2H), 7.31 (dtd, J = 23.0, 7.5, 1.3 Hz, 2H), 5.39 (d, J = 11.0 Hz, 1H), 4.29 (d, J = 10.0 Hz, 1H), 1.38 (d, J = 6.4 Hz, 3H). ESI-MS: m/z = 490.1 [M+H].

Example 10, Isomer 2: Into a 40-mL sealed tube were added 2-[1-(2-chlorophenyl)-1-(2-cyanophenyl)propan-2-yl]-5-methoxy-1-methyl-N-(1,2-oxazol-4-yl)-6-oxopyrimidine-4-carboxamide isomer 2 (73 mg, 0.145 mmol), lithium bromide (189 mg, 2.17 mmol) and DMF (3.0 mL) at RT and stirred for 2 hours at 95 °C under an atmosphere of argon. The mixture was cooled to RT. The crude product was purified by Prep-HPLC with the following conditions (Column: XSelect CSH Prep C18 OBD Column; Mobile Phase A: Water 0.1% formic acid, Mobile Phase B: ACN; Gradient: 47 to 63% B) to afford the title compound (8 mg, 11% yield) as a white solid.

¹H NMR (300 MHz, DMSO-d6) δ 11.39 (s, 1H), 10.37 (s, 1H), 9.31 (s, 1H), 8.89 (s, 1H), 8.15 - 8.06 (m, 1H), 7.72 (d, J = 7.9 Hz, 1H), 7.66 - 7.53 (m, 2H), 7.49 (ddd, J = 7.2, 3.9, 2.6 Hz, 2H), 7.40 - 7.23 (m, 2H), 5.39 (d, J = 11.0 Hz, 1H), 4.35 - 4.25 (m, 1H), 3.47 (s, 3H), 1.38 (d, J = 6.3 Hz, 3H). ESI-MS: m/z = 490.1 [M+H].

Example 10, Isomer 3: Into a 40-mL sealed tube vial were added 2-[1-(2-chlorophenyl)-1-(2-cyanophenyl)propan-2-yl]-5-methoxy-1-methyl-N-(1,2-oxazol-4-yl)-6-oxopyrimidine-4-carboxamide isomer 3 (70 mg, 0.139 mmol), lithium bromide (181 mg, 2.08 mmol) and DMF (3.00 mL) at RT and stirred for 2 hours at 95 °C under an atmosphere of argon. The mixture was cooled to RT. The crude product was purified by Prep-HPLC with the following conditions (Column: SunFire Prep C18 OBD Column; Mobile Phase A: Water 0.1% formic acid, Mobile Phase B: ACN; Gradient: 47 to 70% B) to afford the title compound (13 mg; 19% yield) as a white solid.

¹H NMR (300 MHz, DMSO-d6) δ 10.88 (s, 1H), 9.85 (s, 1H), 9.32 (s, 1H), 8.82 (s, 1H), 7.95 - 7.82 (m, 2H), 7.82 - 7.68 (m, 2H), 7.66 - 7.44 (m, 1H), 7.33 (t, J = 7.3 Hz, 2H), 7.17 (td, J = 7.6, 1.5 Hz, 1H), 5.61 (d, J = 10.8 Hz, 1H), 4.36 - 4.20 (m, 1H), 3.64 (s, 3H), 1.17 (d, J = 6.6 Hz, 3H). ESI-MS: m/z = 490.1 [M+H].

Example 10, Isomer 4: Into a 40-mL sealed tube vial were added 2-[1-(2-chlorophenyl)-1-(2-cyanophenyl)propan-2-yl]-5-methoxy-1-methyl-N-(1,2-oxazol-4-yl)-6-oxopyrimidine-4-carboxamide isomer 4 (60.0 mg, 0.119 mmol, 1.00 equiv), lithium bromide (155.1 mg, 1.79 mmol, 15.00 equiv) and N,N dimethyl formamide (3.00 mL) at room temperature and stirred for 2 h at 95 °C under argon atmosphere. The mixture was allowed to cool down to room temperature. The crude product was purified by Prep-HPLC with the following conditions (Column: SunFire Prep C18 OBD Column; Mobile Phase A: Water 0.1% formic acid, Mobile Phase B: ACN; Gradient: 47 to 70% B) to afford the title compound as a white solid (16 mg, 12% yield).

¹H NMR (400 MHz, DMSO-d6) δ 10.88 (s, 1H), 9.85 (s, 1H), 9.32 (s, 1H), 8.81 (s, 1H), 7.93 - 7.83 (m, 2H), 7.81 - 7.75 (m, 1H), 7.73 (td, J = 7.7, 1.4 Hz, 1H), 7.49 (td, J = 7.5, 1.4 Hz, 1H), 7.37 - 7.28 (m, 2H), 7.16 (td, J = 7.7, 1.5 Hz, 1H), 5.61 (d, J = 10.8 Hz, 1H), 4.34 - 4.21 (m, 1H), 3.64 (s, 3H), 1.17 (d, J = 6.6 Hz, 3H). ESI-MS: m/z = 490.1 [M+H].

### Example 11; Isomer 1, Isomer 2, Isomer 3, Isomer 4

### 2-((1S,2R)-1-(2-cyanophenyl)-1-(3,5-difluorophenyl)propan-2-yl)-5-hydroxy-N-(isoxazol-4-yl)-1-methyl-6-oxo-1,6-dihydropyrimidine-4-carboxamide

### 2-((1R,2S)-1-(2-cyanophenyl)-1-(3,5-difluorophenyl)propan-2-yl)-5-hydroxy-N-(isoxazol-4-yl)-1-methyl-6-oxo-1,6-dihydropyrimidine-4-carboxamide

### 2-((1S,2S)-1-(2-cyanophenyl)-1-(3,5-difluorophenyl)propan-2-yl)-5-hydroxy-N-(isoxazol-4-yl)-1-methyl-6-oxo-1,6-dihydropyrimidine-4-carboxamide

### 2-((1R,2R)-1-(2-cyanophenyl)-1-(3,5-difluorophenyl)propan-2-yl)-5-hydroxy-N-(isoxazol-4-yl)-1-methyl-6-oxo-1,6-dihydropyrimidine-4-carboxamide

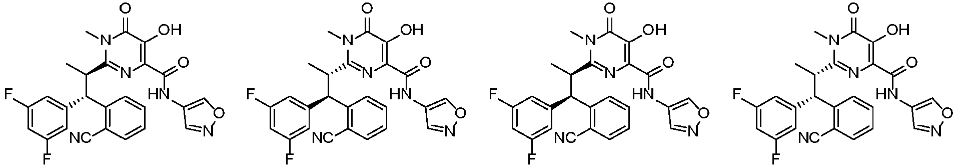

A mixture of the four stereoisomers of 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide was prepared following scheme A, steps 1, 2, and 3 in a manner similar to that described for Example 2 & 3.

The diastereomers were then separated by preparative HPLC (Column: XSelect CSH Prep Fluoro-phenyl OBD Column; Mobile Phase A: Water + 0.1% formic acid, Mobile Phase B: MeOH; Gradient: 52 to 71% MeOH). The early-eluting peak was a mixture of 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 1 and 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 2 (80 mg). ESI-MS m/z = 506.2 [M+H]. The late-eluting peak was a mixture of 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 3 and 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 4 (120 mg). ESI-MS m/z = 506.2 [M+H].

The early-eluting diastereomer was then resolved by preparative chiral HPLC (Column: CHIRAL ART Cellulose-SB; Mobile Phase: 80:20 (1:1 Hexane:MTBE + 0.1% diethylamine):EtOH) to afford the enantiomeric intermediates, isomer 1 (early-eluting enantiomer of the early-eluting diastereomer, 20 mg) and isomer 2 (late-eluting enantiomer of the early-eluting diastereomer, 20 mg) as white solids.

The late-eluting diastereomer was then resolved by preparative chiral HPLC (Column: CHIRALPAK IC-3; Mobile Phase: 90:10 (1:1 Hexane:MTBE + 0.1%DEA):EtOH) to afford the enantiomeric intermediates, isomer 3 (early-eluting enantiomer of the late-eluting diastereomer, 40 mg) and isomer 4 (late-eluting enantiomer of the late-eluting diastereomer, 50 mg) as white solids.

Example 11, Isomer 1: Into a 40 mL vial were added 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 1 (20 mg, 0.044 mmol), lithium bromide (57 mg, 0.65 mmol) and DMF (1.00 mL) at 95 °C. The resulting mixture was stirred for 2h at 95 °C. The crude product was purified by preparative HPLC (Column: XBridge Shield RP18 OBD Column; 50-70% acetonitrile in water + 0.1% formic acid) to afford the title compound (11 mg, 51% yield) as a white solid. ESI-MS m/z = 492.2 [M+H].

Example 11, Isomer 2: Into a 40 mL vial were added 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 2 (20 mg, 0.044 mmol), lithium bromide (57 mg, 0.65 mmol) and DMF (1.0 mL) at 95 °C. The resulting mixture was stirred for 2 hours at 95 °C. The crude product was purified by preparative HPLC (Column: XBridge Shield RP18 OBD Column; 50-70% acetonitrile in water + 0.1% formic acid) to afford the title compound (7 mg, 37% yield) as a white solid.

¹H NMR (400 MHz, DMSO-d6) δ 11.19 (s, 1H), 10.69 (s, 1H), 9.33 (s, 1H), 8.87 (s, 1H), 8.18 (d, J = 8.0 Hz, 1H), 7.91 - 7.78 (m, 2H), 7.50 (t, J = 7.6 Hz, 1H), 7.14 - 7.03 (m, 2H), 6.95 (t, J = 9.4 Hz, 1H), 5.09 (d, J = 10.9 Hz, 1H), 4.28 (dd, J = 11.1, 6.5 Hz, 1H), 3.58 (s, 3H), 1.25 (d, J = 6.5 Hz, 3H). ESI-MS m/z = 492.2 [M+H].

Example 11, Isomer 3: Into a 40 mL vial were added 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 3 (150 mg, 0.297 mmol), lithium bromide (387 mg, 4.45 mmol) and DMF (5.0 mL) at 95 °C. The resulting mixture was stirred for 2 hours at 95 °C. The crude product was purified by preparative HPLC (Column: XBridge Shield RP18 OBD Column; 50-70% acetonitrile in water + 0.1% formic acid) to afford the title compound (93 mg, 68% yield) as a white solid.

¹H NMR (DMSO-d6, 400 MHz) δ 11.07 (s, 1H), 10.46 (s, 1H), 9.30 (s, 1H), 8.88 (s, 1H), 7.95 (d, J = 8.1 Hz, 1H), 7.65 - 7.59 (m, 2H), 7.47 (d, J = 8.0 Hz, 2H), 7.26 (t, J =7.5 Hz, 1H), 7.18 (t, J = 9.4 Hz, 1H), 5.29 (d, J = 11.0 Hz, 1H), 4.33 (s, 1H), 3.67 (s, 3H), 1.24 (d, J = 6.4 Hz, 3H). ESI-MS m/z = 492.2 [M+H].

Example 11, Isomer 4: Into a 40 mL vial were added 2-[1-(2-cyanophenyl)-1-(3,5-difluorophenyl)propan-2-yl]-5-methoxy-1-methyl-N-(1,2-oxazol-4-yl)-6-oxopyrimidine-4-carboxamide isomer 4 (200 mg, 0.396 mmol), lithium bromide (515 mg, 5.94 mmol) and DMF (6.0 mL) at 95 °C. The resulting mixture was stirred for 2 hours at 95 °C. The crude product was purified by preparative HPLC (Column: XBridge Shield RP18 OBD Column; 50-70% acetonitrile in water + 0.1% formic acid) to afford the title compound (86 mg, 74% yield) as a white solid.

¹H NMR (400 MHz, DMSO-d6) δ 11.07 (s, 1H), 10.48 (s, 1H), 9.30 (s, 1H), 8.88 (s, 1H), 7.95 (d, J = 8.1 Hz, 1H), 7.66 - 7.57 (m, 2H), 7.47 (d, J = 7.8 Hz, 2H), 7.29 - 7.22 (m, 1H), 7.18 (t, J = 9.4 Hz, 1H), 5.29 (d, J = 11.1 Hz, 1H), 4.36 - 4.30 (m, 1H), 3.67 (s, 3H), 1.24 (d, J = 6.5 Hz, 3H). ESI-MS m/z = 492.2 [M+H].

### Example 12; Isomer 1, Isomer 2, Isomer 3, Isomer 4

### 2-((1S,2R)-1-(2-cyanophenyl)-1-(3,4-difluorophenyl)propan-2-yl)-5-hydroxy-N-(isoxazol-4-yl)-1-methyl-6-oxo-1,6-dihydropyrimidine-4-carboxamide

### 2-((1R,2S)-1-(2-cyanophenyl)-1-(3,4-difluorophenyl)propan-2-yl)-5-hydroxy-N-(isoxazol-4-yl)-1-methyl-6-oxo-1,6-dihydropyrimidine-4-carboxamide

### 2-((1S,2S)-1-(2-cyanophenyl)-1-(3,4-difluorophenyl)propan-2-yl)-5-hydroxy-N-(isoxazol-4-yl)-1-methyl-6-oxo-1,6-dihydropyrimidine-4-carboxamide

### 2-((1R,2R)-1-(2-cyanophenyl)-1-(3,4-difluorophenyl)propan-2-yl)-5-hydroxy-N-(isoxazol-4-yl)-1-methyl-6-oxo-1,6-dihydropyrimidine-4-carboxamide

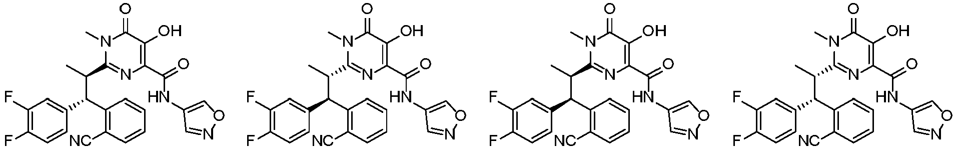

The intermediates necessary to prepare the title compounds were prepared following scheme A, steps 1, 2, and 3 in a manner similar to that described for Example 2 & 3.

The diastereomers of 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide (1.21 g) were separated by preparative HPLC (Column: Xselect CSH F-Phenyl OBD: Mobile Phase A: Water (0.1%FA), Mobile Phase B: MeOH; Gradient: 70 to 75% B) to afford the early-eluting diastereomer (mixture of isomer 1 and isomer 2) (180 mg) and the late-eluting diastereomer (mixture of isomer 3 and isomer 4) (680 mg) .

The early-eluting diastereomer (180 mg) was separated into the enantiomeric intermediates by preparative chiral HPLC (Column: CHIRALPAK IC; Mobile Phase A: hexanes (10mM NH3-MeOH), Mobile Phase B: EtOH; Gradient: 30% B isocratic) to afford the early-eluting enantiomer, 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 1 (52 mg) and the late-eluting enantiomer, 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 2 (59 mg) as off-white solids.

The late-eluting diastereomer (320 mg) was separated into the enantiomeric intermediates by preparative chiral HPLC (Column: CHIRAL ART Cellulose-SC; Mobile Phase A: 1:1 hexanes:MTBE + 0.5% 2M NH3-MeOH, Mobile Phase B: EtOH; Gradient: 10% B isocratic) to afford the early-eluting enantiomer, 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 3 (135mg) and late-eluting enantiomer, 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 4 (117 mg) as off-white solids.

### ESI-MS: m/z = 506.2 [M+H].

Example 12, Isomer 1: To a stirred solution of 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 1 (52 mg, 0.103 mmol) in DMF (2.60 mL) was added LiBr (179 mg, 2.06 mmol). The resulting mixture was stirred for 2 hour at 95 °C. The reaction was cooled to room temperature and directly purified by preparative HPLC (Column: Kinetex EVO C18; Mobile Phase A: Water + 0.1% formic acid, Mobile Phase B: ACN; Gradient: 45 to 58% B) to afford the title compound (22.8 mg) as an off-white solid. ESI-MS: m/z = 492.2 [M+H].

Example 12, Isomer 2. To a stirred solution of 2-[1-(2-cyanophenyl)-1-(3,4-difluorophenyl)propan-2-yl]-5-hydroxy-1-methyl-N-(1,2-oxazol-4-yl)-6-oxopyrimidine-4-carboxamide isomer 2 (59 mg, 0.117 mmol) in DMF (5.0 mL) was added LiBr (202.73 mg, 2.340 mmol, 20.00 equiv). The resulting mixture was stirred for 2 hour at 95 °C. The reaction was cooled to room temperature and directly purified by preparative HPLC (Column: Kinetex EVO C18; Mobile Phase A: Water + 0.1% formic acid, Mobile Phase B: ACN; Gradient: 45 to 58% B) to afford the title compound (28.2 mg) as an off-white solid. ESI-MS: m/z = 492.1 [M+H].

Example 12, Isomer 3: To a stirred solution of 2-[1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 3 (135.00 mg, 0.267 mmol) in DMF (6.7 mL) was added LiBr (464 mg, 5.340 mmol). The resulting mixture was stirred for 2 hour at 95 °C. The reaction was cooled to room temperature and directly purified by preparative HPLC (Column: Kinetex EVO C18; Mobile Phase A: Water + 0.1% formic acid, Mobile Phase B: ACN; Gradient: 45 to 58% B) to afford the title compound (85.6 mg) as an off-white solid. ESI-MS: m/z = 492.1 [M+H].

Example 12, Isomer 4: To a stirred solution of 2-[(1R,2R)-1-(2-cyanophenyl)-1-(3,4-difluorophenyl)propan-2-yl]-5-methoxy-1-methyl-N-(1,2-oxazol-4-yl)-6-oxopyrimidine-4-carboxamide isomer 4 (117 mg, 0.231 mmol) in DMF (5.80 mL) was added LiBr (402 mg, 4.620 mmol The resulting mixture was stirred for 2 hour at 95 °C. The reaction was cooled to room temperature and directly purified by preparative HPLC (Column: Kinetex EVO C18; Mobile Phase A: Water + 0.1% formic acid, Mobile Phase B: ACN; Gradient: 45 to 58% B) to afford the title compound (77.4 mg) as an off-white solid. ESI-MS: m/z = 492.1 [M+H].

### Example 13

### 2-((1R,2R)-1-(2-cyanophenyl)-1-(2,4-difluorophenyl)propan-2-yl)-5-hydroxy-N-(isoxazol-4-yl)-1-methyl-6-oxo-1,6-dihydropyrimidine-4-carboxamide

### 2-((1S,2S)-1-(2-cyanophenyl)-1-(2,4-difluorophenyl)propan-2-yl)-5-hydroxy-N-(isoxazol-4-yl)-1-methyl-6-oxo-1,6-dihydropyrimidine-4-carboxamide

### 2-((1S,2R)-1-(2-cyanophenyl)-1-(2,4-difluorophenyl)propan-2-yl)-5-hydroxy-N-(isoxazol-4-yl)-1-methyl-6-oxo-1,6-dihydropyrimidine-4-carboxamide

### 2-((1R,2S)-1-(2-cyanophenyl)-1-(2,4-difluorophenyl)propan-2-yl)-5-hydroxy-N-(isoxazol-4-yl)-1-methyl-6-oxo-1,6-dihydropyrimidine-4-carboxamide

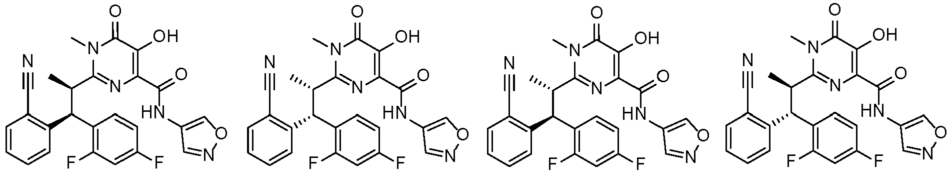

The title compounds were prepared by procedures substantially similar to those reported above for Examples 11 and 12.

Intermediate(2-(1-(2-cyanophenyl)-1-(2,4-difluorophenyl)propan-2-yl)-N-(isoxazol-4-yl)-5-methoxy-1-methyl-6-oxo-1,6-dihydropyrimidine-4-carboxamide) initial separation via PREP-HPLC, Column: Xselect CSH F-Phenyl OBD column, 19*250, 5um; Mobile Phase A:Water(0.1%FA), Mobile Phase B:MeOH-----Preparative; Flow rate:25 mL/min; Gradient:2 B to 74 B in 2 min, 74 B to 74 B in 10 min; 254 nm; RT1:8.48,10.21. Early eluting peak containing isomers 1 & 2; late eluting peak containing isomers 3 & 4.

Early eluting peak from the Prep-HPLC was separated by Prep-chiral-HPLC with the following conditions: Column: CHIRALPAK IC, 2*25cm,5um; Mobile Phase A:HEX:MTBE=1:1(1:1(0.5% 2M NH₃-MEOH), Mobile Phase B:EtOH--HPLC; Flow rate:18 mL/min; Gradient:30 B to 30 B in 9 min; 210/300 nm; RT1:5.95; RT2:7.09; Injection Volumn:0.7 mL; to give individual Isomer 1 & Isomer 2

Late eluting peak from the Prep-HPLC was separated by Prep-chiral-HPLC with the following conditions: Column: CHIRALPAK IC, 2*25cm,5um; Mobile Phase A:HEX:MTBE=1:1(1:1(0.5% 2M NH₃-MEOH), Mobile Phase B:EtOH--HPLC; Flow rate:15 mL/min; Gradient:50 B to 50 B in 14 min; 210/300 nm; RT1:6.2; RT2:10.8125; Injection Volumn:2.5 mL; to give individual Isomer 3 & Isomer 4

Subsequent deprotection yielded the title compounds as white solids :
Example 13, Isomer 1 :
   ESI-MS m/z = 492.1 [M+H]
   ¹H NMR (300 MHz, DMSO-d6) δ 11.25 (s, 1H), 10.40 (s, 1H), 9.30 (s, 1H), 8.86 (s, 1H), 8.06 - 7.96 (m, 1H), 7.77 (d, J = 8.0 Hz, 1H), 7.65 - 7.53 (m, 2H), 7.31 - 7.16 (m, 3H), 5.36 (d, J = 11.2 Hz, 1H), 4.29 (dd, J = 11.4, 6.4 Hz, 1H), 3.60 (s, 3H), 1.32 (d, J = 6.5 Hz, 3H).
Example 13, Isomer 2 :
   ESI-MS m/z = 492.1 [M+H]
   ¹H NMR (300 MHz, DMSO-d6) δ 11.26 (s, 1H), 10.41 (s, 1H), 9.30 (s, 1H), 8.87 (s, 1H), 8.02 (q, J = 8.4 Hz, 1H), 7.78 (d, J = 8.1 Hz, 1H), 7.64 - 7.58 (m, 2H), 7.31 - 7.14 (m, 3H), 5.37 (d, J = 11.3 Hz, 1H), 4.29 (dd, J = 11.4, 6.2 Hz, 1H), 3.60 (s, 3H), 1.33 (d, J = 6.4 Hz, 3H).
Example 13, Isomer 3 :
   ESI-MS m/z = 492.1 [M+H]
   ¹H NMR (300 MHz, DMSO-d6) δ 11.01 (s, 1H), 10.29 (s, 1H), 9.32 (s, 1H), 8.83 (s, 1H), 8.05 (d, J = 8.0 Hz, 1H), 7.86 (dd, J = 7.7, 1.4 Hz, 1H), 7.79 (td, J = 7.8, 1.4 Hz, 1H), 7.64 (q, J = 8.0 Hz, 1H), 7.49 (td, J = 7.6, 1.0 Hz, 1H), 7.08 (td, J = 10.0, 2.6 Hz, 1H), 6.99 (m, J = 9.4, 6.8, 2.7 Hz, 1H), 5.36 (d, J = 11.0 Hz, 1H), 4.33 (dq, J = 13.0, 6.5 Hz, 1H), 3.57 (s, 3H), 1.22 (d, J = 6.5 Hz, 3H).
Example 13, Isomer 4 :
   ESI-MS m/z = 492.1 [M+H]
   ¹H NMR (300 MHz, DMSO-d6) δ 11.00 (s, 1H), 10.23 (s, 1H), 9.32 (s, 1H), 8.83 (s, 1H), 8.06 (d, J = 8.0 Hz, 1H), 7.87 (dd, J = 7.8, 1.4 Hz, 1H), 7.80 (t, J = 7.5 Hz, 1H), 7.64 (q, J = 7.9 Hz, 1H), 7.50 (t, J = 7.5 Hz, 1H), 7.09 (td, J = 10.0, 2.6 Hz, 1H), 7.03 - 6.95 (m, 1H), 5.36 (d, J = 11.0 Hz, 1H), 4.34 (dd, J = 11.0, 6.4 Hz, 1H), 3.57 (s, 3H), 1.23 (d, J = 6.5 Hz, 3H).

### Example 14; Isomer 1, Isomer 2, Isomer 3, Isomer 4

### 5-hydroxy-N-(isoxazol-4-yl)-1-methyl-6-oxo-2-((1R,2S)-1-phenyl-1-(2-(trifluoromethyl)phenyl)propan-2-yl)-1,6-dihydropyrimidine-4-carboxamide

### 5-hydroxy-N-(isoxazol-4-yl)-1-methyl-6-oxo-2-((1S,2S)-1-phenyl-1-(2-(trifluoromethyl)phenyl)propan-2-yl)-1,6-dihydropyrimidine-4-carboxamide

### 5-hydroxy-N-(isoxazol-4-yl)-1-methyl-6-oxo-2-((1S,2R)-1-phenyl-1-(2-(trifluoromethyl)phenyl)propan-2-yl)-1,6-dihydropyrimidine-4-carboxamide

### 5-hydroxy-N-(isoxazol-4-yl)-1-methyl-6-oxo-2-((1R,2R)-1-phenyl-1-(2-(trifluoromethyl)phenyl)propan-2-yl)-1,6-dihydropyrimidine-4-carboxamide

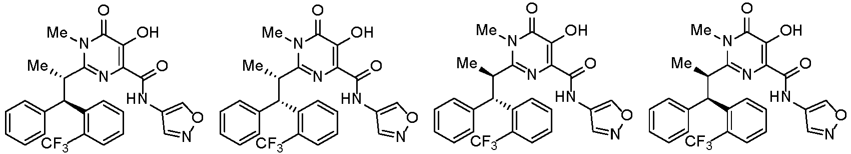

In an analogous manner to that described for 2-(2,2-diphenylethyl)-5-hydroxy-N-(isoxazol-4-yl)-1-methyl-6-oxo-1,6-dihydropyrimidine-4-carboxamide (Example A), Scheme B, steps 1 through 6 were used to prepare the intermediates required for the synthesis of the title compounds.

Method B, steps 6 and 7: N-(Isoxazol-4-yl)-5-methoxy-1-methyl-6-oxo-2-(1-phenyl-1-(2-(trifluoromethyl)phenyl) propan-2-yl)-1,6-dihydropyrimidine-4-carboxamide:

To a solution of methyl 5-methoxy-1-methyl-6-oxo-2-(1-phenyl-1-(2-(trifluoromethyl)phenyl)propan-2-yl)-1,6-dihydropyrimidine-4-carboxylate (2.80 g, 6.08 mmol) in 1: 1: 1 MeOH:THF:water (60 mL) was added sodium hydroxide (0.267 g, 6.68 mmol) at 0 °C. The reaction mixture was stirred for 2 hours at RT. After completion of reaction, the reaction mixture was concentrated under reduced pressure and co-distilled with methanol (3 x 50 mL). The residue was washed with ethyl acetate (2 x 30 mL) to remove the non-polar impurities and concentrated to afford the sodium salt.

The sodium salt from the previous step was dissolved in DMF (25 mL) and HATU (4.62 g, 12.2 mmol) and isoxazol-4-amine (0.664 g, 7.90 mmol) were added. The reaction mixture was stirred for 45 minutes at RT. Then DIPEA (2.360 g, 18.26mmol) was added and the reaction mixture was stirred for 5 hours at RT. After completion of reaction, the reaction mixture was diluted with water (100 mL) and extracted with ethyl acetate (3 x 100 mL). The combined organic layers were washed with brine (200 mL), dried over anhydrous sodium sulphate and concentrated under reduced pressure. The residue was purified by silica gel column chromatography to afford the title compound (2.20 g, 70% yield) as a white solid. The diastereomers (0.9 g) were separated by preparative HPLC to afford the early-eluting diastereomer (0.370 g) and the late-eluting diastereomer (0.380 g).

Each of the two diastereomers were resolved by preparative chiral SFC (CHIRALCEL OJ-H; 10% MeOH in liquid CO₂+ 0.1% DEA) to furnish the enantiopure intermediates. Isomer 1 (early-eluting enantiomer of early-eluting diastereomer tR = 3.71); isomer 2 (late-eluting enantiomer of early-eluting diastereomer: tR = 4.21); isomer 3 (early-eluting enantiomer of late-eluting diastereomer: tR = 3.28); isomer 4 (late-eluting enantiomer of late-eluting diastereomer: tR = 3.67).

Method B, Step 8. 5-Hydroxy-N-(isoxazol-4-yl)-1-methyl-6-oxo-2-(1-phenyl-1-(2-(trifluoromethyl)phenyl) propan-2-yl)-1,6-dihydropyrimidine-4-carboxamide (same procedure for all four stereoisomers): To the solution of N-(isoxazol-4-yl)-5-methoxy-1-methyl-6-oxo-2-(1-phenyl-1-(2-(trifluoromethyl)phenyl)propan-2-yl)-1,6-dihydropyrimidine-4-carboxamide (0.090 g, 0.17 mmol) in dry DMF (4 mL) was added lithium bromide (0.213 g, 2.45mmol) at RT. The reaction mixture was heated and stirred at 80 °C for 40 hours. After completion of reaction, the reaction mixture was concentrated under reduced pressure to provide the crude compound. The residue was filtered through celite and purified by reverse phase chromatography (C18 column; acetonitrile in water + 0.1% formic acid) to afford the title compound (0.053 g, 60% yield) as solid.

Example 14, isomer 1 (derived from early-eluting enantiomer of early-eluting diastereomer from the previous step): ¹H NMR (400 MHz, DMSO-d6): δ 1.22 (d, J = 6.4 Hz, 3H), 3.38 (s, 3H), 4.15 -4.25 (m, 1H), 4.82 (d, J = 10.8 Hz, 1H), 7.05 (t, J = 7.2 Hz, 1H), 7.13 (t, J = 7.2 Hz, 2H), 7.21 (d, J = 7.2 Hz, 2H), 7.50 (t, J = 7.6 Hz, 1H), 7.73-7.81 (m, 2H), 8.31 (d, J = 7.6 Hz, 1H), 8.90 (s, 1H), 9.36 (s, 1H), 10.30 (bs, 1H), 11.09 (bs, 1H). LCMS: m/z = 499.01 [M+1].

Example 14, isomer 2 (derived from late-eluting enantiomer of early-eluting diastereomer from the previous step): ¹H NMR (400 MHz, DMSO-d6): δ 1.20 (d, J = 6.4 Hz, 3H), 3.69 (s, 3H), 4.09 -4.14 (m, 1H), 5.03 (d, J = 10.8 Hz, 1H), 7.22-7.37 (m, 4H), 7.49-7.63 (m, 4H), 8.04 (d, J = 8.0 Hz, 1H), 8.90 (s, 1H), 9.31 (s, 1H), 10.39 (bs, 1H), 11.09 (bs, 1H). LCMS: m/z = 499.00 [M+1].

Example 14, isomer 3 (derived from early-eluting enantiomer of late-eluting diastereomer from the previous step): ¹H NMR (400 MHz, DMSO-d6): δ 1.20 (d, J = 6.0 Hz, 3H), 3.69 (s, 3H), 4.05 -4.15 (m, 1H), 5.03 (d, J = 10.4 Hz, 1H), 7.22-7.37 (m, 4H), 7.49-7.63 (m, 4H), 8.04 (d, J = 7.6 Hz, 1H), 8.90 (s, 1H), 9.31 (s, 1H), 10.38 (bs, 1H), 11.09 (bs, 1H). LCMS: m/z = 499.00 [M+1].

Example 14, isomer 4 (derived from late-eluting enantiomer of late-eluting diastereomer from the previous step): ¹H NMR (400 MHz, DMSO-d6): δ 1.22 (d, J = 6.0 Hz, 3H), 3.38 (s, 3H), 4.19 -4.23 (m, 1H), 4.82 (d, J = 10.8 Hz, 1H), 7.04-7.22 (m, 5H), 7.50 (t, J = 7.6 Hz, 1H), 7.74 (d, J = 7.6 Hz, 1H), 7.79 (t, J = 7.6 Hz, 1H), 8.31 (d, J = 7.6 Hz, 1H), 8.90 (s, 1H), 9.36 (s, 1H), 10.32 (bs, 1H), 11.10 (bs, 1H). LCMS: m/z = 499.00 [M+1].

### Example 15; Isomer 1, Isomer 2, Isomer 3, Isomer 4

### (S)-2-(1,1-bis(2-chlorophenyl)propan-2-yl)-5-hydroxy-N-(isoxazol-4-yl)-1-methyl-6-oxo-1,6-dihydropyrimidine-4-carboxamide

### (R)-2-(1,1-bis(2-chlorophenyl)propan-2-yl)-5-hydroxy-N-(isoxazol-4-yl)-1-methyl-6-oxo-1,6-dihydropyrimidine-4-carboxamide

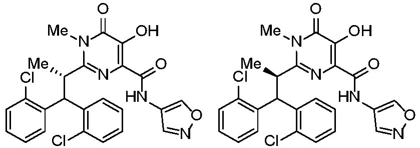

In an analogous manner to that described for 2-(2,2-diphenylethyl)-5-hydroxy-N-(isoxazol-4-yl)-1-methyl-6-oxo-1,6-dihydropyrimidine-4-carboxamide Example A, Scheme B, steps 1 through 6 were used to prepare the intermediates required for the synthesis of the title compounds.

Scheme B, Step 7: 2-(1,1-Bis(2-chlorophenyl)propan-2-yl)-N-(isoxazol-4-yl)-5-methoxy-1-methyl-6-oxo-1,6-dihydropyrimidine-4-carboxamide: To a stirred solution of sodium 2-(1,1-bis(2-chlorophenyl)propan-2-yl)-5-methoxy-1-methyl-6-oxo-1,6-dihydropyrimidine-4-carboxylate (0.315 g, 0.67 mmol) in dry DMF (3.1 mL) was added HATU (0.382 g, 1.00 mmol), isoxazole-4-amine (0.067 g, 0.80 mmol) at RT. The reaction mixture was stirred at RT for 30 minutes. Then DIPEA (0.261 g, 2.01 mmol) was added and reaction mixture was stirred for 3 hours. After completion of reaction, the reaction mixture was diluted with water (15 mL) and the aqueous layer was extracted with ethyl acetate (2 x 20 mL). The combined organic layers were washed with brine (10 mL), dried over anhydrous sodium sulphate and concentrated under reduced pressure. The residue was purified by silica gel column chromatography to afford the title compound (0.21 g, 60% yield).

¹H NMR (400 MHz, DMSO-d6): δ 1.20 (d, J = 6.4 Hz, 3H), 3.62 (s, 3H), 3.75 (s, 3H), 4.10-4.20 (m, 1H), 5.57 (d, J = 10.8 Hz, 1H), 7.13 (t, J = 7.2 Hz, 1H), 7.25-7.28 (m, 3H), 7.34 (t, J = 7.6 Hz, 1H), 7.47 (d, J = 7.6 Hz, 1H), 7.62-7.67 (m, 2H), , 8.74 (s, 1H), 9.28 (s, 1H), 10.25 (bs, 1H). LCMS: m/z = 513.3 [M+ 1].

The racemic title compound was resolved by preparative chiral HPLC (CHIRALPAK IC; 40% (MeOH: IPA = 50:50) in Hexanes + 0.1% DEA) to furnish the enantiopure intermediates as isomer 1 (1st eluting isomer) and isomer 2 (2nd eluting isomer).

Scheme B, step 8: (S)- and (R)-2-(1,1-bis(2-chlorophenyl)propan-2-yl)-5-hydroxy-N-(isooxazol-4-yl)-1-methyl-6-oxo-1,6-dihydropyrimidine-4-carboxamide: To a solution of 2-(1,1-bis(2-chlorophenyl)propan-2-yl)-N-(isoxazol-4-yl)-5-methoxy-1-methyl-6-oxo-1,6-dihydropyrimidine-4-carboxamide isomer 1 (0.05 g, 0.10 mmol) in DMF (0.5 mL) was added lithium bromide (0.085 g, 0.97 mmol) at RT. The reaction mixture was heated in microwave reactor at 180 °C for 1 hour. After completion of reaction, the reaction mixture was concentrated. The residue was filtered through celite and purified by reverse phase chromatography (C18 column; acetonitrile in water + 0.1% formic acid) to afford the title compound (0.023 g, 47% yield) as a solid. 2-(1,1-Bis(2-chlorophenyl)propan-2-yl)-N-(isoxazol-4-yl)-5-methoxy-1-methyl-6-oxo-1,6-dihydropyrimidine-4-carboxamide isomer 2, was prepared in a similar manner.

Example 15, isomer 1 (derived from early-eluting isomer in step 7): ¹H NMR (400 MHz, DMSO-d6): δ 1.30 (d, J = 6.0 Hz, 3H), 3.55 (s, 3H), 4.10-4.20 (m, 1H), 5.57 (d, J = 11.2 Hz, 1H), 7.12 (t, J = 7.6 Hz, 1H), 7.22-7.32 (m, 3H), 7.39 (t, J = 7.2 Hz, 1H), 7.49 (d, J = 7.6 Hz, 1H), 7.64 (d, J = 7.2 Hz, 1H), 7.80 (d, J = 6.8 Hz, 1H), 8.93 (s, 1H), 9.34 (s, 1H), 10.12 (bs, 1H), 11.13 (bs, 1H). LCMS: m/z = 499.1 [M+1].

Example 15, isomer 2 (derived from late-eluting isomer in step 7): ¹H NMR (400 MHz, DMSO-d6): δ 1.29 (d, J = 6.4 Hz, 3H), 3.55 (s, 3H), 4.10-4.20 (m, 1H), 5.57 (d, J = 11.2 Hz, 1H), 7.12 (t, J = 7.6 Hz, 1H), 7.22-7.31 (m, 3H), 7.39 (t, J = 7.2 Hz, 1H), 7.49 (d, J = 7.6 Hz, 1H), 7.63 (d, J = 7.6 Hz, 1H), 7.79 (d, J = 6.0 Hz, 1H), 8.92 (s, 1H), 9.33 (s, 1H), 10.18 (bs, 1H), 11.13 (bs, 1H). LCMS: m/z = 499.1 [M+1].

### Example 16; Isomer 1, Isomer 2, Isomer 3, Isomer 4

### 2-((1S,2R)-1-(2-cyano-4-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(2-cyano-4-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-cyano-4-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(2-cyano-4-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

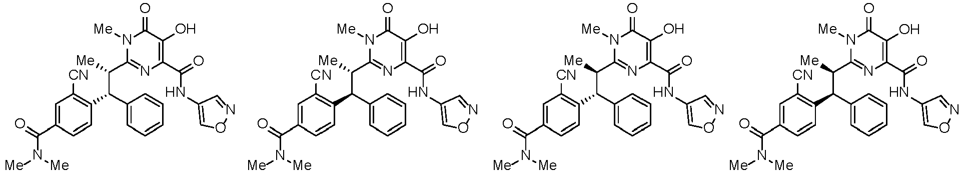

### Step-1: Methyl 3-bromo-4-(bromomethyl)benzoate:

To a 500 mL 3-necked round-bottom flask containing methyl 3-bromo-4-methylbenzoate (10.00 g, 44 mmol), N-bromosuccinimide (7.77 g, 44 mmol) and 1,2-dichloroethane (200 mL) at rt was added 2,2'-azobis(2-methylpropionitrile) (2.15 g, 13 mmol). After complete addition the resulting mixture was heated to 80 °C and subjected to blue LED light with stirring for 4 h. The mixture was then cooled to room temperature and concentrated under reduced pressure. The resulting residue was purified by silica gel column chromatography (0% to 10% EtOAc/pet. ether). Fractions containing product were collected and concentrated in vacuo giving the desired product as a white solid (8.20 g, 61% yield).

¹H NMR (400 MHz, DMSO-d6): δ 8.12 (d, J = 1.7 Hz, 1H), 7.95 (dd, J = 8.0, 1.7 Hz, 1H), 7.78 (d, J = 8.0 Hz, 1H), 4.78 (s, 2H), 3.87 (s, 3H).

### Step-2: Methyl 4-benzyl-3-bromobenzoate:

To a 250 mL 3-necked round-bottom flask containing methyl 3-bromo-4-(bromomethyl)benzoate (5.00 g, 16.24 mmol, 1.00 equiv), phenyl boronic acid (1.98 g, 16 mmol), K2CO3 (4.49 g, 33 mmol) dissolved in dimethoxyethane (100 mL) and H2O (20 mL) was added Pd(PPh3)4 (0.56 g, 0.49 mmol). This reaction mixture was then heated to 90 °C and stirred for 4 h at which point it was cooled to rt and concentrated in vacuo. The resulting mixture was diluted with brine (100 mL) and the product was extracted with EtOAc, the organic layer was washed with brine, dried over Na2SO4 then concentrated in vacuo. The crude reaction mixture was then purified by C18 flash chromatography (60% to 70% acetonitrile/H2O) fractions containing product were collected and the solvent was removed in vacuo affording the product as a yellow oil (2.0 g, 40% yield).

¹H NMR (300 MHz, DMSO-d6): δ 8.11 (d, J = 1.8 Hz, 1H), 7.91 (dd, J = 8.0, 1.8 Hz, 1H), 7.46 (d, J = 8.0 Hz, 1H), 7.36 - 7.26 (m, 2H), 7.29-7.19 (m, 3H), 4.16 (s, 2H), 3.86 (s, 3H).

### Step 3: 4-benzyl-3-bromobenzoic acid:

To a 100 mL round-bottom flask containing methyl 4-benzyl-3-bromobenzoate (5.02 g, 16 mmol) dissolved in MeOH (50 mL) and H2O (10 mL) was added LiOH·H2O (2.07 g, 49 mmol). The resulting mixture was stirred at rt for 2h at which point it was acidified to pH 6~7 with 1M HCl. The product was then extracted with EtOAc, the combined organic layer was washed with brine, dried over Na2SO4 then concentrated in vacuo. The product was collected without further purification as a yellow solid (4.70 g, 98% yield).

ESI-MS m/z: Calc'd 290.0, 292.0 found 291.0, 293.0 [M+H]

¹H NMR (400 MHz, DMSO-d6): δ 13.20 (s, 1H), 8.09 (d, J = 1.7 Hz, 1H), 7.88 (dd, J = 8.0, 1.8 Hz, 1H), 7.44 (d, J = 8.0 Hz, 1H), 7.31 (dd, J = 8.5, 6.4 Hz, 2H), 7.26 - 7.16 (m, 3H), 4.15 (s, 2H).

### Step 4: 4-benzyl-3-bromo-N,N-dimethylbenzamide:

To a 250 mL round-bottom flask containing 4-benzyl-3-bromobenzoic acid (4.70 g, 16 mmol), dimethylamine hydrochloride (1.97 g, 24 mmol) and HATU (6.75 g, 18 mmol) dissolved in N,N-dimethylformamide (60 mL) was added N,N-diisopropylethylamine (10.41 g, 81 mmol) dropwise at 0 °C. The resulting mixture was warmed to rt and stirred for 2h at which point it was diluted with brine and the product was extracted with EtOAc. The organic layers were collected and combined then washed with brine, dried over Na2SO4 and concentrated in vacuo. The resulting crude reaction material was purified by silica gel column chromatography (20% to 30% EtOAc/pet. ether) fractions containing product were combined and concentrated to afford the product as a light yellow oil (5.02 g, 98% yield).

ESI-MS m/z: Calc'd 317.0, 319.0 found 318.0, 320.0 [M+H]

### Step 5: 4-benzyl-3-cyano-N,N-dimethylbenzamide:

To a 250 mL 3-necked round-bottom flask containing 4-benzyl-3-bromo-N,N-dimethylbenzamide (5.00 g, 16 mmol) and Zn(CN)2 (2.40 g, 20.44 mmol) dissolved in N,N-dimethylaniline (60 mL) under N₂ was added Pd₂(dba)₃ (4.32 g, 4.71 mmol) followed by X-Phos (2.25 g, 4.71 mmol). The resulting mixture was then heated to 110 °C and stirred for 2h under N₂ atmosphere. The mixture was allowed to cool to rt at which point it was quenched by the addition of brine. The resulting solution was extracted with EtOAc and the organic layers were collected and combined then washed with brine, dried over Na₂SO₄ and concentrated in vacuo. The resulting crude reaction mixture was purified by silica gel column chromatography (EtOAc/Pet ether 40%-50%) fractions containing product were combined and concentrated affording the product as a brown oil (98.72% yield).

ESI-MS m/z: Calc'd 264.1 found 265.2 [M+H]

### Step-6: Ethyl 2-1-(2-cyano-4-(dimethylcarbamoyl)phenyl)-1-phenylpropan-2-yl)-5-methoxy-1-methyl-6-oxo-1,6-dihydropyrimidine-4-carboxylate:

To a -78 °C stirred solution of 4-benzyl-3-cyano-N,N-dimethylbenzamide (1.70 g, 6.43 mmol) dissolved in THF (10 mL) was added KHMDS (1.28 g, 6.43 mmol) in THF (6.4 mL) dropwise. The resulting mixture was then allowed to warm to -50 °C and stirred for additional 1h at which point the mixture was cooled to -78 °C and a solution of ethyl 2-(1-bromoethyl)-5-methoxy-1-methyl-6-oxo-1,6-dihydropyrimidine-4-carboxylate (1.58 g, 4.95 mmol) in THF (10 mL) was added dropwise. Upon complete addition the reaction was stirred for 2h during which time it was allowed to warm to rt and then quenched with water. The resulting solution was extracted with EtOAc (50 mL) and the organic layer was dried over Na₂SO₄ and concentrated in vacuo. The resulting crude material was purified by reverse phase C18 chromatography (30% to 40% acetonitrile/water (0.1% FA) fractions containing product were combined and concentrated to afford the product as a white solid.

Peak-1_D1 (early eluting peak, containing two separate enantiomers: isomer 1 and isomer2) : 404.3 mg of a white solid.

Peak-2_D2 (late eluting peak, containing two separate enantiomers: isomer 3 and isomer4) : 152.1 mg of a white solid.

ESI-MS m/z: Calc'd 502.2 found 503.3 [M+H]

ESI-MS m/z: Calc'd 502.2 found 503.3 [M+H]

### Step-7: lithium 2-(1-(2-cyano-4-(dimethylcarbamoyl)phenyl)-1-phenylpropan-2-yl)-5-methoxy-1-methyl-6-oxo-1,6-dihydropyrimidine-4-carboxylate:

To a 40 mL vial containing ethyl 2-(1-(2-cyano-4-(dimethylcarbamoyl)phenyl)-1-phenylpropan-2-yl)-5-methoxy-1-methyl-6-oxo-1,6-dihydropyrimidine-4-carboxylate (0.220 g, 0.44 mmol) dissolved in MeOH (4.00 mL) and H2O (1.00 mL) at 0 °C was added LiOH·H2O (0.055 g, 1.31 mmol). The resulting mixture was stirred at rt for 2h and then concentrated in vacuo giving the product as a white solid (0.171 g, 81% yield). This was carried out on both diastereomeric mixtures from Step 6.

ESI-MS m/z: Calc'd 474.2 found 475.2 [M+H]

ESI-MS m/z: Calc'd 474.2 found 475.2 [M+H]

### Step-8: 2-(1-(2-cyano-4-(dimethylcarbamoyl)phenyl)-1-phenylpropan-2-yl)-N-(isoxazol-4-yl)-5-methoxy-1-methyl-6-oxo-1,6-dihydropyrimidine-4-carboxamide:

To a 40 mL vial containing lithium 2-(1-(2-cyano-4-(dimethylcarbamoyl)phenyl)-1-phenylpropan-2-yl)-5-methoxy-1-methyl-6-oxo-1,6-dihydropyrimidine-4-carboxylate (0.171 g, 0.36 mmol), isoxazol-4-amine hydrochloride (0.064 g, 0.53 mmol) and HATU (0.149 mg, 0.39 mmol) dissolved in DMF (3 mL) was added N,N-Diisopropylethylamine (0.230 g, 1.78 mmol) dropwise at 0 °C. The resulting mixture was warmed to rt and stirred for 2h at which point it was diluted with brine and the product was extracted with EtOAc. The organic layers were collected and combined then washed with brine, dried over Na2SO4 and concentrated in vacuo. The resulting crude reaction material was purified by reverse phase C18 chromatography (20% to 60% acetonitrile/water (0.1% FA) fractions containing product were combined and concentrated to afford the product as a white solid (0.100 g, 52% yield).

ESI-MS m/z: Calc'd 540.2 found 541.3 [M+H]

This was carried out on both diastereomeric mixtures from Step 7.

Each mixed product was separated by Prep-chiral-HPLC:

D1 - derived from early eluting peak from step 6 : Column: CHIRAL ART Cellulose-SB, 2*25cm,5um; Mobile Phase A:Hex:MtBE=1:1(0.5% 2M NH3-MEOH), Mobile Phase B:EtOH--HPLC; Flow rate:20 mL/min; Gradient:30 B to 30 B in 10 min; 220/300 nm; RT1:6.56; RT2:7.9625; Injection Volumn:0.3 mL

Peak 1 (Isomer-1): RT 6.56 min; afforded a white solid (85.1 mg)

Peak 2 (Isomer-2): RT 7.96 min; afforded a white solid (87.0 mg)

D2: derived from late eluting peak from step 6. Column: CHIRAL ART Cellulose-SB, 2*25cm,5um; Mobile Phase A:Hex:MtBE=1:1(0.5% 2M NH3-MEOH), Mobile Phase B:EtOH--HPLC; Flow rate:20 mL/min; Gradient:20 B to 20 B in 16 min; 220/300 nm; Injection Volumn:0.15 mL

Peak 1 (Isomer-3): RT 10.06 min; afforded a white solid (75.4 mg)

Peak 2 (Isomer-4): RT 11.99 min; afforded a white solid (64.6 mg)

Step-9: 2-(1-(2-cyano-4-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide: To a solution of 2-(1-(2-cyano-4-(dimethylcarbamoyl)phenyl)-1-phenylpropan-2-yl)-N-(isoxazol-4-yl)-5-methoxy-1-methyl-6-oxo-1,6-dihydropyrimidine-4-carboxamide (0.085 mg, 0.16 mmol) in DMF (3 mL), lithium bromide (0.275 mg, 3.15 mmol) was added at RT. The reaction mixture was then heated to 95 °C for 1h. After completion of reaction, the reaction mixture was concentrated and the residue was purified by reverse phase chromatography (C18 column; acetonitrile in in water + 0.1% formic acid) to afford the title compound (0.035 g, 42% yield) as white solid. Each isomer from step 8 was processed in the same fashion.

Example 16, Isomer-1: Isolated product as a white solid (0.035 g, 42% yield); >98% ee

ESI-MS m/z: Calc'd 526.2 found 526.9 [M+H]

¹H NMR (300 MHz, DMSO-d6): δ 11.18 (s, 1H), 10.54 (s, 1H), 9.35 (s, 1H), 8.93 (s, 1H), 8.26 (d, J = 8.2 Hz, 1H), 7.91 - 7.81 (m, 2H), 7.28 - 7.14 (m, 4H), 7.13 - 7.04 (m, 1H), 5.00 (d, J = 10.9 Hz, 1H), 4.34 (dd, J = 11.1, 6.4 Hz, 1H), 3.44 (s, 3H), 2.99 (s, 3H), 2.90 (s, 3H), 1.33 (d, J = 6.4 Hz, 3H).

Example 16, Isomer-2: Isolated product as a white solid (0.030 g, 35% yield); >98% ee

ESI-MS m/z: Calc'd 526.2 found 526.9 [M+H]

1H NMR (300 MHz, DMSO-d6): δ 11.18 (s, 1H), 10.54 (s, 1H), 9.35 (s, 1H), 8.93 (s, 1H), 8.26 (d, J = 8.2 Hz, 1H), 7.89 (d, J = 1.8 Hz, 1H), 7.85 (dd, J = 8.1, 1.9 Hz, 1H), 7.27 - 7.14 (m, 4H), 7.13 - 7.03 (m, 1H), 5.00 (d, J = 10.9 Hz, 1H), 4.34 (dq, J = 12.9, 6.3 Hz, 1H), 3.44 (s, 3H), 2.99 (s, 3H), 2.90 (s, 3H), 1.33 (d, J = 6.4 Hz, 3H).

Example 16, Isomer-3: Isolated product as a white solid (0.027 g, 37% yield); >98% ee

ESI-MS m/z: Calc'd 526.2 found 526.9 [M+H]

¹H NMR (300 MHz, DMSO-d6): δ 11.13 (s, 1H), 10.45 (s, 1H), 9.31 (s, 1H), 8.89 (s, 1H), 7.97 (d, J = 8.3 Hz, 1H), 7.77 - 7.70 (m, 2H), 7.68 (d, J = 1.8 Hz, 1H), 7.59 (dd, J = 8.2, 1.9 Hz, 1H), 7.42 (t, J = 7.6 Hz, 2H), 7.29 (t, J = 7.3 Hz, 1H), 5.23 (d, J = 11.2 Hz, 1H), 4.32 (dt, J = 11.3, 6.3 Hz, 1H), 3.69 (s, 3H), 2.89 (s, 3H), 2.71 (s, 3H), 1.25 (d, J = 6.4 Hz, 3H).

Example 16, Isomer-4: Isolated product as a white solid (0.021 g, 34% yield); >98% ee

ESI-MS m/z: Calc'd 526.2 found 526.9

¹H NMR (300 MHz, DMSO-d6): δ 11.13 (s, 1H), 10.45 (s, 1H), 9.31 (s, 1H), 8.89 (s, 1H), 7.97 (d, J = 8.3 Hz, 1H), 7.76 - 7.70 (m, 2H), 7.68 (d, J = 1.8 Hz, 1H), 7.59 (dd, J = 8.2, 1.9 Hz, 1H), 7.42 (t, J = 7.6 Hz, 2H), 7.33 - 7.25 (m, 1H), 5.23 (d, J = 11.2 Hz, 1H), 4.31 (dd, J = 11.3, 6.6 Hz, 1H), 3.69 (s, 3H), 2.89 (s, 3H), 2.71 (s, 3H), 1.25 (d, J = 6.5 Hz, 3H).

### Example 17; Isomer 1, Isomer 2, Isomer 3, Isomer 4

### 2-((1S,2R)-1-(2-cyano-5-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1R,2S)-1-(2-cyano-5-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1S,2S)-1-(2-cyano-5-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

### 2-((1R,2R)-1-(2-cyano-5-(dimethylcarbamoyl)phenyl)-1-phenylpropan-2-yl)-5-hydroxy-N-(isoxazol-4-yl)-1-methyl-6-oxo-1,6-dihydropyrimidine-4-carboxamide

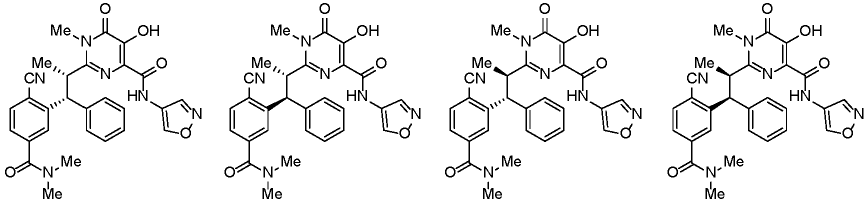

### Step-1: Methyl 3-(bromomethyl)-4-cyanobenzoate:

To a 250-mL round-bottom flask containing methyl 4-cyano-3-methylbenzoate (10.00 g, 57.1 mmol), N-bromosuccinimide (20.32 g, 114 mmol) and 1,2-dichloroethane (100 mL) at room temperature was added 2,2'-azobis(2-methylpropionitrile) (2.81 g, 17.1 mmol) After complete addition the resulting mixture was heated to 80 °C and subjected to blue LED light with stirring for 2 h. The mixture was then cooled to room temperature and concentrated under reduced pressure. The resulting residue was purified by silica gel column chromatography (0% to 20% EtOAc/pet. ether). Fractions containing product were collected and concentrated in vacuo giving the desired product as a light yellow solid (12.2 g, 84% yield).

¹H NMR (400 MHz, DMSO-d6): δ 8.34 - 8.26 (m, 1H), 8.09 - 8.00 (m, 2H), 4.92 (s, 2H), 3.92 (s, 3H).

### Step-2: Methyl 3-benzyl-4-cyanobenzoate:

To a 250-mL round-bottom flask containing methyl 3-(bromomethyl)-4-cyanobenzoate (6.10 g, 24.0 mmol), phenyl boronic acid (2.93 g, 24.0 mmol) and K2CO3 (6.64 g, 48.0 mmol) dissolved in dimethoxyethane (50 mL) and H2O (10 mL) was added Pd(PPh3)4 (0.83 g, 0.720 mmol). This reaction mixture was then heated to 90 °C and stirred for 1 h at which point it was cooled to rt and concentrated in vacuo. The resulting crude reaction material was subsequently purified by silica gel chromatography (0% to 20% EtOAc/pet. Ether), fractions containing product were collected and concentrated in vacuo giving the desired product as a white sold (3.9g, 64% yield).

¹H NMR (400 MHz, DMSO-d6): δ 8.03 - 7.91 (m, 3H), 7.37 - 7.31 (m, 2H), 7.29 - 7.22 (m, 3H), 4.26 (s, 2H), 3.88 (s, 3H).

### Step-3: 3-benzyl-4-cyanobenzoic acid:

To a 250 mL round-bottom flask containing methyl 3-benzyl-4-cyanobenzoate (5.80 g, 23.1 mmol) dissolved in MeOH (50 mL) and H2O (10 mL) at 0 °C was added LiOH·H2O (1.94 g, 46.2 mmol). The resulting mixture was warmed to rt and stirred 3h at which point it was acidified to pH 5~6 with 1M HCl. The product was then extracted with CH2Cl2 (3x50 mL), the combined organic layer was washed with brine, dried over Na2SO4 then concentrated in vacuo. The product was collected and used in the next step without further purification (yellow solid, 6 g).

### Step-4. 3-benzyl-4-cyano-N,N-dimethylbenzamide:

A 250 mL round-bottom flask containing 3-benzyl-4-cyanobenzoic acid (5.80 g, 24.4 mmol) and HATU (18.6 g, 48.9 mmol) dissolved in N,N-dimethylformamide (60 mL) was cooled to 0 °C and dimethylamine hydrochloride (3.99 g, 48.9 mmol) was added portion wise followed by N,N diisopropylethyl amine (21.3 mL, 165 mmol). The resulting mixture was warmed to rt and stirred for 1h at which point it was diluted with water (200 mL) and the product was extracted with CH2Cl2 (5x50 mL). The organic layers were collected and combined then washed with brine, dried over Na2SO4 and concentrated in vacuo. The resulting crude reaction material was purified by silica gel column chromatography (0% to 100% EtOAc/pet. ether) fractions containing product were combined and concentrated to afford the product as a light yellow solid (5.8 g, 89% yield).

ESI-MS m/z Calc'd 264.1, found 265.2 [M+H]

### Step-5: Ethyl 2-[1-[2-cyano-5-(dimethylcarbamoyl)phenyl]-1-phenylpropan-2-yl]-5-methoxy-1-methyl-6-oxopyrimidine-4-carboxylate:

To a -78 °C stirred solution of 3-benzyl-4-cyano-N,N-dimethylbenzamide (1.99 g, 7.54 mmol) dissolved in THF (50 mL) was added KHMDS (7.54 g, 7.55 mmol, 1M in THF) dropwise. The resulting mixture was then allowed to warm to -50 °C and stirred for additional 1h at which point the mixture was cooled to -78 °C and a solution of ethyl 2-(1-bromoethyl)-5-methoxy-1-methyl-6-oxo-1,6-dihydropyrimidine-4-carboxylate (1.72 g, 5.39 mmol) in THF (10 mL) was added dropwise. Upon complete addition the reaction was stirred for 2h during which time it was allowed to warm to rt and then quenched with Water. The resulting solution was extracted with EtOAc (50 mL) and the organic layer was dried over Na₂SO₄ and concentrated in vacuo. The resulting crude material was purified by reverse phase C18 chromatography (30% to 40% Acetonitrile/water (0.1% FA) fractions containing product were combined and concentrated to afford the product as a white solid (2.1g, 77% yield).

ESI-MS m/z: Calc'd 502.2, found 503.2 [M+H]

### Step-6: lithio 2-[1-[2-cyano-5-(dimethylcarbamoyl)phenyl]-1-phenylpropan-2-yl]-5-methoxy-1-methyl-6-oxopyrimidine-4-carboxylate:

To a 40 mL vial containing ethyl 2-[1-[2-cyano-5-(dimethylcarbamoyl)phenyl]-1-phenylpropan-2-yl]-5-methoxy-1-methyl-6-oxopyrimidine-4-carboxylate (1.50 g, 2.98 mmol) dissolved in MeOH (12 mL) and H2O (3 mL) at 0 °C was added LiOH·H2O (0.251 g, 5.97 mmol. The resulting mixture was stirred at rt for 2h and then concentrated in vacuo giving the product as a yellow solid (1.6 g)

ESI-MS m/z: Calc'd 474.2, found 475.2 [M+H]

### Step-7: 2-[1-[2-cyano-5-(dimethylcarbamoyl)phenyl]-1-phenylpropan-2-yl]-5-methoxy-1-methyl-N-(1,2-oxazol-4-yl)-6-oxopyrimidine-4-carboxamide:

To a 100 mL round bottom flask containing lithio 2-[1-[2-cyano-5-(dimethylcarbamoyl)phenyl]-1-phenylpropan-2-yl]-5-methoxy-1-methyl-6-oxopyrimidine-4-carboxylate (1.50 g, 3.12 mmol), isoxazol-4-amine hydrochloride (0.753 g, 6.24 mmol, 2 equiv) and HATU (1.78 mg, 4.68 mmol) dissolved in DMF (15 mL) was added N,N-Diisopropylethylamine (2.72 g, 15.6 mmol) dropwise at 0 °C. The resulting mixture was warmed to rt and stirred for 2h at which point it was diluted with brine and the product was extracted with EtOAc. The organic layers were collected and combined then washed with brine, dried over Na2SO4 and concentrated in vacuo. The resulting crude reaction material was purified by silica gel column chromatography (40% to 100% EtOAc/pet. ether) fractions containing product were combined and concentrated to afford the product as a light yellow solid (0.705 g, 41% yield).

ESI-MS m/z: Calc'd 540.2, found 541.2 [M+H]

Separation with reverse phase C18 chromatography (20% to 55% Acetonitrile/water (0.1% FA)

Peak 1 D1 (early eluting peak, containing two separate enantiomers: isomer 1 and isomer2): 480 mg of a white solid

Peak 2 D2 (late eluting peak, containing two separate enantiomers: isomer 3 and isomer4) :114 mg of a white solid

Further separated by Prep-chiral-HPLC:

D1 (early eluting peak from prior separation): Column: XSelect CSH Prep C18 OBD Column, 5um, 19 *150mm; Mobile Phase A: Water (0.1% FA), Mobile Phase B: Acetonitrile; Flow rate: 25 mL/min; Gradient: 29% B to 56% B in 8 min

Peak 1 (Isomer-1): afforded a white solid (104 mg)

Peak 2 (Isomer-2): afforded a white solid (107 mg)

D2 (late eluting peak from prior separation): Column: XSelect CSH Prep C18 OBD Column, 5um, 19*150mm; Mobile Phase A: Water (0.1% FA), Mobile Phase B: Acetonitrile; Flow rate:25 mL/min; Gradient: 29% B to 56% B in 8 min).

Peak 1 (Isomer-3): afforded a white solid (40 mg)

Peak 2 (Isomer-4): afforded a white solid (44 mg)

Step-9: (2-[1-(2-cyano-5-(dimethylcarbamoyl)phenyl)-1-phenylpropan-2-yl]-5-methoxy-1-methyl-N-(1,2-oxazol-4-yl)-6-oxopyrimidine-4-carboxamide: To a solution of 2-[1-[2-cyano-5-(dimethylcarbamoyl)phenyl]-1-phenylpropan-2-yl]-5-methoxy-1-methyl-N-(1,2-oxazol-4-yl)-6-oxopyrimidine-4-carboxamide (0.104 mg, 0.19 mmol, 1.0 equiv) in DMF (5 mL), lithium bromide (0.334 mg, 3.84 mmol, 20 equiv) was added at RT. The reaction mixture was then heated to 95 °C for 1h. After completion of reaction, the reaction mixture was concentrated and the residue was purified by reverse phase C18 chromatography (D1: 29 to 56 % acetonitrile in H2O (0.1% FA); D2: 35 to 58% acetonitrile in H2O (0.1% FA)). Each isomer from Step 8 was processed in a substantially similar fashion.

Example 17, Isomer-1 : Isolated product as a white solid (0.069 g, 67% yield); >98 % ee

ESI-MS m/z: Calc'd 526.2, found 527.4 [M+H]

¹H NMR (400 MHz, DMSO-d6): δ 11.16 (s, 1H), 10.60 (s, 1H), 9.34 (s, 1H), 8.92 (s, 1H), 8.22 (s, 1H), 7.89 (d, 1H), 7.45 (dd, 1H), 7.26 - 7.13 (m, 4H), 7.08 (t, 1H), 5.00 (d, 1H), 4.37 (dd, 1H), 3.42 (s, 3H), 3.06 (s, 3H), 2.89 (s, 3H), 1.31 (d, 3H).

Example 17, Isomer-2: Isolated product as a white solid (0.076 g, 73% yield); >98% ee

ESI-MS m/z: Calc'd 526.2, found 527.3 [M+H]

¹H NMR (400 MHz, DMSO-d6): δ 11.16 (s, 1H), 10.57 (s, 1H), 9.35 (s, 1H), 8.92 (s, 1H), 8.22 (d, 1H), 7.89 (d, 1H), 7.45 (dd, 1H), 7.26 - 7.13 (m, 4H), 7.10 - 7.06 (m, 1H), 5.00 (d, 1H), 4.38 (dd, 1H), 3.42 (s, 3H), 3.06 (s, 3H), 2.89 (s, 3H), 1.31 (d, 3H).

Example 17, Isomer-3: Isolated product as a white solid (0.024 g, 60% yield); > 98% ee

ESI-MS m/z: Calc'd 526.2, found 527.1 [M+H]

¹H NMR (400 MHz, DMSO-d6): δ 11.17 (s, 1H), 10.61 (s, 1H), 9.30 (s, 1H), 8.87 (s, 1H), 7.92 (d, 1H), 7.73 (d, 2H), 7.67 (d, 1H), 7.41 (t, 2H), 7.28 (t, 1H), 7.18 (dd, 1H), 5.22 (d, 1H), 4.32 (dd, 1H), 3.68 (s, 3H), 2.95 (s, 3H), 2.57 (s, 3H), 1.24 (d, 3H).

Example 17, Isomer-4: Isolated product as a white solid (0.026 g, 60% yield); >98% ee

ESI-MS m/z: Calc'd 526.2, found 527.2 [M+H]

¹H NMR (400 MHz, DMSO-d6): δ 11.17 (s, 1H), 10.61 (s, 1H), 9.30 (s, 1H), 8.87 (s, 1H), 7.92 (d, 1H), 7.73 (d, 2H), 7.67 (d, 1H), 7.41 (t, 2H), 7.28 (t, 1H), 7.18 (dd, 1H), 5.22 (d, 1H), 4.32 (dd, 1H), 3.68 (s, 3H), 2.95 (s, 3H), 2.57 (s, 3H), 1.24 (d, 3H).

### Reference Example 18; Isomer 1, Isomer 2

### (S)-2-(3-fluoro-1,1-diphenylpropan-2-yl)-5-hydroxy-N-(isoxazol-4-yl)-1-methyl-6-oxo-1,6-dihydropyrimidine-4-carboxamide

### (R)-2-(3-fluoro-1,1-diphenylpropan-2-yl)-5-hydroxy-N-(isoxazol-4-yl)-1-methyl-6-oxo-1,6-dihydropyrimidine-4-carboxamide

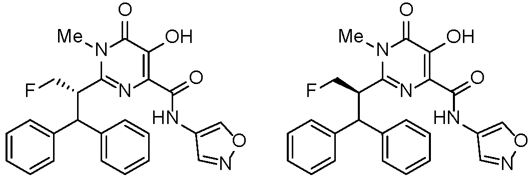

### Step-1: N-benzhydryl-4-methylbenzenesulfonamide:

To a 0 °C stirred solution of benzhydrylamine (25.0 g, 136 mmol) in CH2Cl2 (400 mL) was added p-toluenesulfonyl chloride (33.8 g, 177 mmol) followed by triethylamine (27.6 g, 273 mmol) dropwise over 2 min. This reaction was then allowed to warm to rt and stirred for 1 h at which point it was again cooled to 0 °C and quenched by the addition of ice water. The resulting mixture was extracted with EtOAc (3 x 300 mL), the combined organic layer was washed with water then dried over Na2SO4 and concentrated in vacuo. The crude material was then purified by silica gel chromatography (20% EtOAc/pet. ether) fractions containing product were collected and the solvent was removed in vacuo to give the product as a light yellow solid (45g, 98% yield).

¹H NMR (400 MHz, DMSO-d6): δ 8.72 (d, J = 8.9 Hz, 1H), 7.54 - 7.47 (m, 2H), 7.22 - 7.12 (m, 12H), 5.54 (d, J = 7.9 Hz, 1H), 2.27 (s, 3H)

### Step-2: Ethyl 2-cyano-3,3-diphenylpropanoate:

To a 0 °C stirred solution of N-(diphenylmethyl)-4-methylbenzenesulfonamide (41.0 g, 122 mmol) and ethyl cyanoacetate (17.9 g, 158 mmol) in CHCl3 (500 mL) was added aluminum trichloride (16.2 g, 122 mmol) portion wise. Upon complete addition the reaction was heated to 80 °C and stirred overnight at which point it was cooled to 0 °C and quenched with HCl (conc.) bringing the reaction to pH 5. The resulting mixture was then extracted with EtOAc (3 x 300 mL) and the combined organic layers was washed with water then dried over Na2SO4 and concentrated in vacuo. Product was purified by silica gel chromatography (10% EtOAc/pet. ether) fractions containing product were collected and the solvent was removed in vacuo to give the product as a light yellow solid (30g, 88% yield).

¹H NMR (400 MHz, DMSO-d6): δ 7.55 - 7.49 (m, 2H), 7.42 - 7.19 (m, 8H), 5.26 (d, J = 10.1 Hz, 1H), 4.71 (d, J = 10.1 Hz, 1H), 4.04 - 3.99 (m, 2H), 0.95 (t, J = 7.1 Hz, 3H)

### Step-3: 2-(hydroxymethyl)-3,3-diphenylpropanenitrile:

To a 0 °C stirred solution of ethyl 2-cyano-3,3-diphenylpropanoate (35.0 g, 125 mmol) dissolved in ethanol (500 mL) was added sodium borohydride (23.7 g, 627 mmol) portionwise. Upon complete addition the reaction mixture was warmed to rt and stirred overnight at which point it was cooled to 0°C and quenched with ice water. The resulting mixture was extracted with CH2Cl2 (3 x 300 mL) and the combined organic layers were further washed with water then dried over Na2SO4 and concentrated in vacuo. The crude reaction mixture was purified by silica gel chromatography (20% EtOAc/pet. ether) fractions containing product were collected and the solvent was removed in vacuo to give the product as a white solid (29g, 98% yield)

¹H NMR (400 MHz, DMSO-d6): δ 7.51 - 7.46 (m, 2H), 7.43 - 7.38 (m, 2H), 7.31 (m, 4H), 7.24 - 7.17 (m, 2H), 5.35 (t, J = 5.0 Hz, 1H), 4.33 (d, J = 11.3 Hz, 1H), 3.98 (m, 1H), 3.50 (m, 1H), 3.40 - 3.34 (m, 1H).

### Step 4: 2-(((tert-butyldimethylsilyl)oxy)methyl)-3,3-diphenylpropanenitrile.

To a 0 °C stirred solution of 2-(hydroxymethyl)-3,3-diphenylpropanenitrile (15.0 g, 63 mmol) in CH2Cl2 (400 mL) was added imidazole (25.8 g, 379 mmol) followed by tertbutyldimethylsilyl chloride (19.1 g, 126 mmol) portion wise. Upon complete addition the reaction was warmed to rt and stirred for 4 h at which point it was then diluted with water and the product was extracted with EtOAc (3 x 300 mL). The combined organic layer was washed with water then dried over Na₂SO₄ and concentrated in vacuo. The crude reaction material was purified by silica gel chromatography (10% EtOAc/pet. ether) fractions containing product were collected and the solvent was removed in vacuo to give the product as a white solid (18.8g, 85% yield).

¹H NMR (400 MHz, DMSO-d6): δ 7.54 - 7.49 (m, 2H), 7.43 - 7.38 (m, 2H), 7.37 - 7.28 (m, 4H), 7.26 - 7.19 (m, 2H), 4.32 (d, J = 11.6 Hz, 1H), 4.12 (m, 1H), 3.68 (dd, J = 10.2, 3.4 Hz, 1H), 3.51 (dd, J = 10.2, 5.5 Hz, 1H), 0.88 (s, 9H), 0.01 (d, J = 10.5 Hz, 6H).

### Step-5: 2-(((tert-butyldimethylsilyl)oxy)methyl)-N'-hydroxy-3,3-diphenylpropanimidamide:

To a stirred mixture of 2-(((tert-butyldimethylsilyl)oxy)methyl)-3,3-diphenylpropanenitrile (18.0 g, 51 mmol) in ethanol (100 mL) was added hydroxylamine hydrochloride (5.1 g, 154 mmol) in water (10 mL). The reaction mixture was heated to 80 °C and stirred for 16 hours at which point it was cooled to rt and diluted with water (100 mL). The product was then extracted with EtOAc (2 x 100 mL) and the combined organic layer was dried over anhydrous sodium sulphate then concentrated in vacuo, the resulting crude material was used in the next step without further purification.

### Step-6: dimethyl 2-((((Z)-1-amino-2-(((tert-butyldimethylsilyl)oxy)methyl)-3,3-diphenylpropylidene) amino)oxy)but-2-enedioate:

A solution of 2-(((tert-butyldimethylsilyl)oxy)methyl)-N'-hydroxy-3,3-diphenylpropanimidamide in MeOH (100 mL) was cooled 0 °C. To the cooled solution was added dimethyl acetylenedicarboxylate (29.1 g, 205.0 mmol) drop wise. The resulting mixture was allowed to warm to rt and stirred for 4 hours at which point the solvent was removed in vacuo and the crude material was purified by silica gel chromatography (10% EtOAc/pet. ether) fractions containing product were collected and the solvent was removed in vacuo to give the product as a brown solid (17.1g)

ESI-MS m/z: Calc'd 526.2, found 526.3 [M+H].

¹H NMR (400 MHz, DMSO-d6): δ 7.44 - 7.32 (m, 4H), 7.29 (m, 2H), 7.25 - 7.14 (m, 3H), 7.14 - 7.07 (m, 1H), 6.41 (s, 1H), 6.04 (d, J = 38.2 Hz, 1H), 5.47 (d, J = 29.1 Hz, 1H), 4.24 (dd, J = 22.0, 12.4 Hz, 1H), 3.74 (d, J = 7.8 Hz, 3H), 3.62 (d, J = 20.5 Hz, 3H), 3.49 - 3.43 (m, 1H), 3.37 - 3.19 (m, 2H), 0.80 (d, J = 3.3 Hz, 9H), -0.02 - -0.17 (m, 6H).

### Step-7: Methyl 2-(3-((tert-butyldimethylsilyl)oxy)-1,1-diphenylpropan-2-yl)-5-hydroxy-6-oxo-1,6-dihydropyrimidine-4-carboxylate:

Dimethyl 2-((((Z)-1-amino-2-(((tert-butyldimethylsilyl)oxy)methyl)-3,3-diphenylpropylidene)amino)oxy) but-2-enedioate (18.0 g, 34 mmol) was dissolved in o-xylene (900 mL) and heated to 140 °C for 4 hour. Upon completion the reaction was cooled to rt and then concentrated in vacuo. The resulting crude material was purified by silica gel chromatography (5% MeOH/CH2Cl2) fractions containing product were collected and the solvent was removed in vacuo to give the product as a yellow solid (13g, 77% yield)

ESI-MS m/z: Calc'd 495.2, found 494.2 [M+H]

### Step-8: Methyl 2-(3-((tert-butyldimethylsilyl)oxy)-1,1-diphenylpropan-2-yl)-5-methoxy-1-methyl-6-oxo-1,6-dihydropyrimidine-4-carboxylate:

To a stirred solution of methyl 2-(3-((tert-butyldimethylsilyl)oxy)-1,1-diphenylpropan-2-yl)-5-hydroxy-6-oxo-1,6-dihydropyrimidine-4-carboxylate (2.5 g, 5 mmol) and K2CO3 (1.4g, 10 mmol) in DMF (40 mL) was added iodomethane (1.4g, 10 mmol) dropwise. The resulting mixture was heated to 50 °C and stirred for 6 h at which point it was cooled to rt and diluted with water (400 mL). The product was extracted with EtOAc (3 x 300 mL) and the combined organic layer was washed with water then dried over Na2SO4 and concentrated in vacuo. Crude product was purified by silica gel chromatography (3% MeOH/CH2Cl2) fractions containing product were collected and the solvent was removed in vacuo to give the product as a brown solid (2.3g, 87% yield).

ESI-MS m/z: Calc'd 522.3, found 523.3 [M +H]+

### Step-9: Methyl 2-(3-hydroxy-1,1-diphenylpropan-2-yl)-5-methoxy-1-methyl-6-oxo-1,6-dihydropyrimidine-4-carboxylate:

To a 0 °C stirred solution of methyl 2-(3-((tert-butyldimethylsilyl)oxy)-1,1-diphenylpropan-2-yl)-5-methoxy-1-methyl-6-oxo-1,6-dihydropyrimidine-4-carboxylate (3.2 g, 6 mmol) dissolve in THF (20 mL) was added Tetrabutylammonium fluoride (12.2 mL, 12 mmol) dropwise. The resulting reaction mixture was then warmed to rt and stirred for 6 h at which point it was diluted with water (200 mL) and the product was extracted with EtOAc (3 x 200 mL). The combined organic layer was washed with water then dried over Na2SO4 and concentrated in vacuo. The crude reaction material was purified by silica gel chromatography (20% EtOAc/pet. Ether) fractions containing product were collected and the solvent was removed in vacuo to give the product as a yellow solid (1.6 g, 89% yield).

ESI-MS m/z: Calc'd 408.2, found 409.1 [M+H]

### Step-10: Methyl 2-(3-fluoro-1,1-diphenylpropan-2-yl)-5-methoxy-1-methyl-6-oxo-1,6-dihydropyrimidine-4-carboxylate:

To a 0 °C stirred solution of methyl 2-(3-hydroxy-1,1-diphenylpropan-2-yl)-5-methoxy-1-methyl-6-oxo-1,6-dihydropyrimidine-4-carboxylate (0.6 g, 1.5 mmol) dissolved in CH2Cl2 (10 mL) was added N,N-diethyl-1,1,1-trifluoro-4-sulfanamine (0.474 g, 2.9 mmol) dropwise. The resulting mixture was warmed to rt and stirred for 6 h at which point it was quenched by Ice water and the product was extracted with CH2Cl2 (3 x 300 mL). The combined organic layer was washed with water then dried over Na2SO4 and concentrated in vacuo. The resulting crude material was purified by prep-TLC (30% EtOAc/pet. Ether), the product was isolated as a yellow solid (0.420 g, 70% yield).

ESI-MS m/z: Calc'd 410.2, found 411.1 [M+H].

### Step-11: 2-(3-fluoro-1,1-diphenylpropan-2-yl)-5-methoxy-1-methyl-6-oxo-1,6-dihydropyrimidine-4-carboxylic acid:

To a 0 °C solution of methyl 2-(3-fluoro-1,1-diphenylpropan-2-yl)-5-methoxy-1-methyl-6-oxo-1,6-dihydropyrimidine-4-carboxylate (0.300 g, 0.73 mmol) dissolved in 1,2-dichloroethane (10 mL) was added trimethyltin hydroxide (0.264 g, 1.5 mmol). The resulting mixture was heated to 65 °C and stirred for 3.5 hours then cooled to rt and filtered, washed with EtOAc (2 x 10 mL). The filtrate was concentrated in vacuo giving the desired product which was used in the next step with no further purification (0.240 g, 48% yield).

ESI-MS m/z: Calc'd 396.1, founds 397.3 [M+H]

### Step-12: 2-(3-fluoro-1,1-diphenylpropan-2-yl)-N-(isoxazol-4-yl)-5-methoxy-1-methyl-6-oxo-1,6-dihydropyrimidine-4-carboxamide:

To a 0 °C stirred solution of 2-(3-fluoro-1,1-diphenylpropan-2-yl)-5-methoxy-1-methyl-6-oxo-1,6-dihydropyrimidine-4-carboxylic acid (0.240 g, 0.35 mmol) and 1,2-oxazol-4-amine hydrochloride (0.064 g, 0.53 mmol) in DMF (3 mL) was added HATU (0.471 g, 1.24 mmol) followed by N,N diisopropylethyl amine (0.404 g, 3.12 mmol) dropwise at 0 °C. The resulting mixture was warmed to rt and stirred for 2h at which point it was diluted with brine and the product was extracted with EtOAc. The organic layers were collected and combined then washed with brine, dried over Na₂SO₄ and concentrated in vacuo. The resulting crude material was purified by prep-TLC (5% MeOH/CH₂Cl₂).

ESI-MS m/z: Calc'd 462.2, found 463.3 [M+H]

Enantiomers of this material were separated by Prep-chiral-HPLC:

Column: CHIRAL ART Cellulose-SC, 2*25cm, 5um; Mobile Phase A:Hex: MtBE=1:1(0.5% 2M NH3-MEOH), Mobile Phase B:EtOH--HPLC; Flow rate:20 mL/min; Gradient:20 B to 20 B in 8 min; 220/300 nm; Injection Volume: 0.3 mL; Number Of Runs:16;.

Peak 1 (Isomer-1, early eluting peak): RT 4.28 min; afforded a white solid (52 mg)

Peak 2 (Isomer-2, late eluting peak): RT 5.52 min; afforded a white solid (48 mg)

### Step-13: 2-(3-fluoro-1,1-diphenylpropan-2-yl)-5-hydroxy-N-(isoxazol-4-yl)-1-methyl-6-oxo-1,6-dihydropyrimidine-4-carboxamide:

To a solution of 2-[3-fluoro-1,1-diphenylpropan-2-yl]-5-methoxy-1-methyl-N-(1,2-oxazol-4-yl)-6-oxopyrimidine-4-carboxamide (0.045 mg, 0.10 mmol, 1.00 equiv) in DMF (2.5 mL), lithium bromide (0.299 mg, 3.44 mmol, 15 equiv) was added at RT. The reaction mixture was then heated to 95 °C for 1h. After completion of reaction, the reaction mixture was concentrated and the residue was purified by reverse phase chromatography (Gemini-NX C18 AXAI Packed column, 21.2*150mm 5um; 45% to 78% acetonitrile in water + 0.1% formic acid) to afford the title compound. Both enantiomers were processed by substantially the same procedure.

Example 18, Isomer 1 (derived from the early eluting peak from step 11): Isolated product as a white solid (0.031 g, 71% yield); >98% ee

ESI-MS m/z: Calc'd 448.2, found 449.3 [M+H]

¹H NMR (400 MHz, DMSO-d6): δ 11.39 (s, 1H), 10.69 (s, 1H), 9.37 (s, 1H), 9.01 (s, 1H), 7.75 - 7.67 (m, 2H), 7.39 (t, J = 7.6 Hz, 2H), 7.33 - 7.23 (m, 3H), 7.14 (t, J = 7.6 Hz, 2H), 7.07 - 7.00 (m, 1H), 4.89 (m, 1H), 4.73 (q, J = 11.6 Hz, 2H), 4.67 - 4.48 (m, 1H), 3.49 (s, 3H).

¹⁹F NMR (377 MHz, DMSO): δ -220.31.

Example 18, Isomer 2 (derived from the late eluting peak from step 11): Isolated product as a white solid (0.032 g, 73% yield); >97% ee

ESI-MS m/z: Calc'd 448.2, found 449.3 [M+H]

¹H NMR (400 MHz, DMSO-d6): δ 11.39 (s, 1H), 10.69 (s, 1H), 9.37 (s, 1H), 9.01 (s, 1H), 7.75 - 7.67 (m, 2H), 7.39 (t, J = 7.6 Hz, 2H), 7.33 - 7.23 (m, 3H), 7.14 (t, J = 7.6 Hz, 2H), 7.07 - 7.00 (m, 1H), 4.89 (m, 1H), 4.73 (q, J = 11.6 Hz, 2H), 4.67 - 4.48 (m, 1H), 3.49 (s, 3H).

¹⁹F NMR (377 MHz, DMSO): δ -220.31.

### Reference Example 19

### 2-(1,1-difluoro-2,2-diphenylethyl)-5-hydroxy-N-(isoxazol-4-yl)-1-methyl-6-oxo-1,6-dihydropyrimidine-4-carboxamide

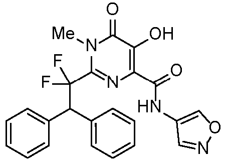

Step 1: methyl 2-(2,2-diphenylethyl)-5-methoxy-1-methyl-6-oxopyrimidine-4-carboxylate (700 mg, 1.85 mmol, 1 equiv) and lithium(I) (dimethylsilyl)(trimethylsilyl) amide (402.37 mg, 2.41 mmol, 1.3 equiv) in tetrahydrofuran (7 mL) at -78°C under argon atmosphere was stirred for 0.5 h at -70°C. To the above mixture was added N-(benzenesulfonyl)-N-fluorobenzenesulfonamide (758.27 mg, 2.41 mmol, 1.3 equiv) dropwise at -78°C. The resulting mixture was stirred for additional 1.5 h from -78 °C to rt. The reaction was quenched with water/ice at 0 °C. The resulting mixture was extracted with ethyl acetate (3 x 30 mL). The combined organic layers were washed with brine (2x30 mL), dried over anhydrous sodium sulfate. After filtration, the filtrate was concentrated under reduced pressure. The residue was purified by silica gel column chromatography, eluted with ethyl acetate/petroleum (1:5) to afford methyl 2-(1-fluoro-2,2-diphenylethyl)-5-methoxy-1-methyl-6-oxopyrimidine-4-carboxylate (400 mg, 54.6%) which was used directly in the following reaction.

ESI-MS m/z = 396.9 [M+H].

Step 2 : methyl 2-(1-fluoro-2,2-diphenylethyl)-5-methoxy-1-methyl-6-oxopyrimidine-4-carboxylate (900.00 mg, 2.27 mmol, 1.00 equiv) and lithium(I) (dimethylsilyl)(trimethylsilyl) amide (759.77 mg, 4.54 mmol, 2 equiv) in tetrahydrofuran (10.00 mL, 123.43 mmol, 54.37 equiv) at -78°C under argon atmosphere was stirred for 0.5 h at -70° C. To the above mixture was added N-(benzenesulfonyl)-N-fluorobenzenesulfonamide (1431.81 mg, 4.54 mmol, 2.00 equiv) dropwise at -78°C. The resulting mixture was stirred for additional 1 h from -78°C to rt. The reaction was quenched with water/ice at 0°C. The resulting mixture was extracted with ethyl acetate (3 x 50 mL). The combined organic layers were washed with brine (2x30 mL), dried over anhydrous sodium sulfate. After filtration, the filtrate was concentrated under reduced pressure. The residue was purified by silica gel column chromatography, eluted with ethyl acetate/petroleum (1:5) to afford methyl 2-(1,1-difluoro-2,2-diphenylethyl)-5-methoxy-1-methyl-6-oxopyrimidine-4-carboxylate (200 mg, 21.26%) as a dark yellow oil.

ESI-MS m/z =415.2 [M+H].

Step 3: To a stirred mixture of methyl 2-(1,1-difluoro-2,2-diphenylethyl)-5-methoxy-1-methyl-6-oxopyrimidine-4-carboxylate (250.00 mg, 0.60 mmol, 1.00 equiv) and trimethyltin hydroxide (1091.92 mg, 6.03 mmol, 10.00 equiv) in 1,2-dichloroethane (6.00 mL). The resulting mixture was stirred for additional 3.5 h at 70 °C. The resulting mixture was filtered, the filter cake was washed with ethyl acetate (2x10 mL). The filtrate was concentrated under reduced pressure. The crude product was used in the next step directly without further purification.

ESI-MS m/z =401.0 [M+H]

Step 4: To a stirred mixture of 2-(1,1-difluoro-2,2-diphenylethyl)-5-methoxy-1-methyl-6-oxopyrimidine-4-carboxylic acid (250.00 mg, 0.62 mmol, 1.00 equiv) and 1,2-oxazol-4-amine hydrochloride (112.90 mg, 0.94 mmol, 1.50 equiv) in N,N dimethyl formamide (3.00 mL) were added HATU (471.20 mg, 1.24 mmol, 2.00 equiv) and N,N diisopropyl ethyl amine (403.50 mg, 3.12 mmol, 5 equiv) dropwise at 0 o C under nitrogen atmosphere. The resulting mixture was stirred for additional 1 h at room temperature. The reaction was quenched with Water/Ice at 0 o C. The resulting mixture was extracted with ethyl acetate (3 x 30 mL). The combined organic layers were washed with water (3x30 mL), dried over anhydrous sodium sulfate. After filtration, the filtrate was concentrated under reduced pressure. The residue was purified by Prep-TLC (ethyl acetate/petroleum 1:1) to afford (2-(1,1-difluoro-2,2-diphenylethyl)-5-methoxy-1-methyl-N-(1,2-oxazol-4-yl)-6-oxopyrimidine-4-carboxamide)(107 mg, 42.8%) as a dark yellow solid.

Step 5: To a stirred mixture of 2-(1,1-difluoro-2,2-diphenylethyl)-5-methoxy-1-methyl -N-(1,2-oxazol-4-yl)-6-oxopyrimidine-4-carboxamide (107.00 mg, 0.23 mmol, 1.00 equiv) and lithium bromide (298.83 mg, 3.44 mmol, 15.00 equiv) in N,N dimethyl formamide (4.00 mL). The resulting mixture was stirred for additional 1 h at 95 o C. The mixture was allowed to cool down to room temperature. The crude product (107 mg) was purified by Prep-HPLC with the following conditions (Column: Gemini-NX C18 AXAI Packed, 21.2*150mm 5um; Mobile Phase A: Water(0.05%FA), Mobile Phase B: ACN; Flow rate:25 mL/min; Gradient:52 B to 70 B in 8 min; 254/220 nm; RT1:6.93;) to afford Example 19 (2-(1,1-difluoro-2,2-diphenylethyl)-5-hydroxy-1-methyl-N-(1,2-oxazol-4-yl)-6-oxopyrimidine-4-carboxamide)(58.6 mg, 54.76%) as a off-white solid.

ESI-MS m/z = 453.0[M+H]

¹H NMR (400 MHz, DMSO-d6) δ 11.61 (s, 1H),10.27 (s, 1H), 9.31 (s, 1H), 8.91 (s, 1H), 7.53 (d, J = 7.6 Hz, 4H), 7.31 (dd, J = 8.3, 6.9 Hz, 4H), 7.24 - 7.18 (m, 2H), 5.90 (t, J = 18.9 Hz, 1H) ,3.62 (t, J = 2.2 Hz, 3H).

### Reference Example 20; Isomer 1, Isomer 2

### (S)-5-hydroxy-N-(isoxazol-4-yl)-1-methyl-6-oxo-2-(1,1,1-trifluoro-3,3-diphenylpropan-2-yl)-1,6-dihydropyrimidine-4-carboxamide

### (R)-5-hydroxy-N-(isoxazol-4-yl)-1-methyl-6-oxo-2-(1,1,1-trifluoro-3,3-diphenylpropan-2-yl)-1,6-dihydropyrimidine-4-carboxamide

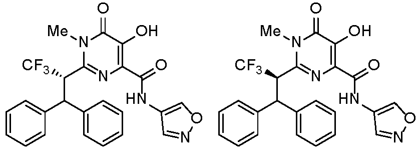

Scheme B, steps 6 and 7. Lithium 5-methoxy-1-methyl-6-oxo-2-(1,1,1-trifluoro-3,3-diphenylpropan-2-yl)-1,6-dihydro pyrimidine-4-carboxylate: To a stirred solution of methyl 5-methoxy-1-methyl-6-oxo-2-(1,1,1-trifluoro-3,3-diphenylpropan-2-yl)-1,6-dihydropyrimidine-4-carboxylate (0.160 g, 0.36 mmol) in 1:1:1 MeOH: THF: Water (4.8 mL) was added lithium hydroxide monohydrate (0.043 g, 1.05 mmol) at RT. The reaction mixture was heated and stirred at 50 °C for 8 hours. After completion of reaction, the reaction mixture was concentrated to dryness. The residue was co-distilled with toluene (2 x 10 mL). The solid was dried over high vacuum to afford the title compound (0.18 g, crude) which was used in the next step without further purification. LCMS: m/z = 433.35 [M+1]

To a stirred solution of lithium 5-methoxy-1-methyl-6-oxo-2-(1,1,1-trifluoro-3,3-diphenylpropan-2-yl)-1,6-dihydropyrimidine-4-carboxylate (0.180 g, 0.41 mmol) in DMF (2 mL) was added HATU (0.624 g, 1.64 mmol) and isoxazol-4-amine (0.136 g, 1.61 mmol) at RT. The reaction mixture was stirred for 1 hour and then DIPEA (0.319 g, 2.46mmol) was added. The reaction mixture was stirred for 30 minutes. After completion of reaction, the reaction mixture was diluted with cold water (15 mL) and extracted with ethyl acetate (3 x 15 mL). The combined organic layers were washed with brine (10 mL), dried over anhydrous sodium sulphate and concentrated. The residue was purified by silica gel column chromatography to afford the title compound (0.15 g, 82% yield) as a racemic mixture.

¹H NMR (400 MHz, CDCl₃): δ 3.65 (s, 3H), 4.09 (s, 3H), 4.68-4.72 (m, 1H), 4.95 (d, J = 12.4 Hz, 1H), 7.17-7.25 (m, 4H), 7.34 (d, J = 7.2 Hz, 1H), 7.42 (t, J = 7.2 Hz, 2H), 7.50 (d, J = 7.6 Hz, 2H), 8.54 (s, 1H),9.07 (s, 1H), 9.23 (s, 1H). LCMS: m/z = 499.3 [M+1]. Amide NH not observed.

The racemic title compound was resolved by preparative chiral SFC (CHIRALPAK AD-H; 25% (50:50 MeOH:IPA) in liquid CO2 + 0.1% DEA to furnish the enantiomeric intermediates. Isomer 1 (early-eluting enantiomer: tR = 3.99 minutes); and isomer 2 (late-eluting enantiomer tR = 4.53 minutes).

Scheme B, step 8. 5-Hydroxy-N-(isoxazol-4-yl)-1-methyl-6-oxo-2-(1,1,1-trifluoro-3,3-diphenyl propan-2-yl)-1,6-dihydropyrimidine-4-carboxamide: To a solution of N-(isoxazol-4-yl)-5-methoxy-1-methyl-6-oxo-2-(1,1,1-trifluoro-3,3-diphenylpropan-2-yl)-1,6-dihydropyrimidine-4-carboxamide (0.058 g, 0.12 mmol) in DMF (1.0 mL) was added lithium bromide (0.07 g, 0.81 mmol) at RT and the reaction mixture was heated and stirred at 80 °C for 48 hours. After completion of reaction, the reaction mixture was directly purified by reverse phase chromatography (C18 column; acetonitrile in water + 0.1% formic acid) to afford the title compound (0.025 g, 44% yield). Both stereoisomers were prepared in a similar fashion.

Example 20, isomer 1 (derived from the early-eluting enantiomer in the previous step): ¹H NMR (400 MHz, DMSO-d6): δ 3.72 (s, 3H), 5.39-5.45 (m, 2H), 7.02 (t, J = 7.6 Hz, 1H), 7.16 (t, J = 7.6 Hz, 2H), 7.24 (t, J = 7.6 Hz, 1H), 7.36 (t, J = 7.6 Hz, 2H), 7.48 (d, J = 7.6 Hz, 2H), 7.82 (d, J = 7.6 Hz, 2H), 9.03 (s, 1H), 9.38 (s, 1H), 10.55 (s, 1H), 11.61 (s, 1H). LCMS: m/z = 485.2 [M+1].

Example 20, isomer 2 (derived from the late-eluting enantiomer in the previous step): ¹H NMR (400 MHz, DMSO-d6): δ 3.72 (s, 3H), 5.37-5.45 (m, 2H), 7.0 (t, J = 7.6 Hz, 1H), 7.16 (t, J = 7.6 Hz, 2H), 7.24 (t, J = 7.6 Hz, 1H), 7.36 (t, J = 7.6 Hz, 2H), 7.48 (d, J = 7.2 Hz, 2H), 7.82 (d, J = 7.6 Hz, 2H), 9.03 (s, 1H), 9.37 (s, 1H), 10.55 (s, 1H), 11.61 (s, 1H). LCMS: m/z = 485.2 [M+1].

### Example 21; Isomer 1, Isomer 2

### (S)-2-(3,3-bis(2-cyanophenyl)-1,1,1-trifluoropropan-2-yl)-5-hydroxy-N-(isoxazol-4-yl)-1-methyl-6-oxo-1,6-dihydropyrimidine-4-carboxamide

### (R)-2-(3,3-bis(2-cyanophenyl)-1,1,1-trifluoropropan-2-yl)-5-hydroxy-N-(isoxazol-4-yl)-1-methyl-6-oxo-1,6-dihydropyrimidine-4-carboxamide

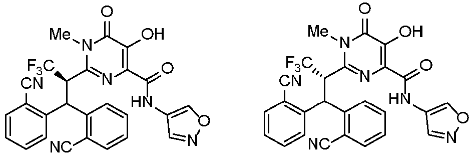

### Step-1: Ethyl 5-methoxy-1,2-dimethyl-6-oxo-1,6-dihydropyrimidine-4-carboxylate:

A mixture of ethyl 2-Chloro-5-methoxy-1-methyl-6-oxo-1,6-dihydropyrimidine-4-carboxylate (30 g, 121.95 mmol), trimethylboroxine (40.77 mL, 50% w/w in THF, 146.34 mmol), cesium carbonate (119.2 g, 365.8 mmol) in dioxane (300 mL) was purged for 10 minutes with argon gas. To the reaction mixture, S-Phos Palladium G3 precatalyst (4.75 g, 6.1 mmol) was added and purging was continued for another 10 minutes. The reaction mixture was heated in a sealed tube at 90°C for 45 minutes. After completion of the reaction (monitored by TLC), water (300 mL) was added to the reaction mixture and extracted with DCM (2 x 600 mL). The combined organic layer was washed with brine (250 mL), dried over anhydrous sodium sulphate and concentrated under vacuum. The crude product was purified by column chromatography to afford pure title compound (21 g, 76%) as light yellow solid.

LCMS: m/z 227.2 [M+H].

¹H NMR (400 MHz, DMSO-d6): δ 1.28 (t, J = 6.8 Hz, 3H), 2.45 (s, 3H), 3.45 (s, 3H), 3.78 (s, 3H), 4.29 (q, J = 7.2 Hz, 2H).

### Step-2: Ethyl 2-(bromomethyl)-5-methoxy-1-methyl-6-oxo-1,6-dihydropyrimidine-4-carboxylate:

A mixture of ethyl 5-methoxy-1,2-dimethyl-6-oxo-1,6-dihydropyrimidine-4-carboxylate (21 g, 92.9 mmol), N-bromosuccinimide (18.19 g, 102.2 mmol) and AIBN (1.52 g, 9.29 mmol) in 1,2 dichloroethane (210 mL) was heated at 90°C for 3 hours under argon gas atmosphere. The reaction progress was monitored by TLC. The reaction mixture was cooled to room temperature (once dibromo product observed on TLC), water (200 mL) was added. The reaction mixture was extracted with dichloromethane (2 x 300 mL). The combined organic layer was washed with brine (150 mL), dried over anhydrous sodium sulphate and concentrated under vacuum. The crude product was purified by column chromatography to afford pure title compound (16.5 g, 58%).

LCMS: m/z 305.0 [M+H] and 307.0 [M++2].

¹H NMR (400 MHz, DMSO-d6): δ 1.29 (t, J = 7.2 Hz, 3H), 3.55 (s, 3H), 3.86 (s, 3H), 4.31 (q, J = 7.2 Hz, 2H), 4.65 (s, 2H).

### Step-3: Ethyl 2-(2,2-bis(2-cyanophenyl) ethyl)-5-methoxy-1-methyl-6-oxo-1,6-dihydropyrimidine-4-carboxylate:

The solution of 2,2'-methylenedibenzonitrile (7.52 g, 34.5 mmol) in THF (35 mL) was cooled to 0°C under nitrogen atmosphere. 1M LiHMDS in THF (34.5 mL, 34.5 mmol) was added dropwise at 0°C for 10 minutes and reaction mixture was stirred for another 10 minutes. Ethyl 2-(bromomethyl)-5-methoxy-1-methyl-6-oxo-1,6-dihydropyrimidine-4-carboxylate (7 g, 23.02 mmol) in THF (105 mL) was added dropwise at 0°C for 10 minutes and reaction mixture was stirred at 0°C for another 15 minutes. After completion of reaction (confirmed by TLC), 10% aqueous solution of ammonium chloride (150 mL) was added and reaction mixture was extracted with ethyl acetate (2 x 500 mL). The combined organic layer was washed with brine (500 mL), dried over anhydrous sodium sulphate and concentrated under vacuum. The crude product was purified by RP column chromatography using acetonitrile and 0.1% formic acid in water to give pure title compound (1.6 g, 15%).

LCMS: m/z 443.39 [M+H].

¹H NMR (400 MHz, DMSO): δ1.20 (t, J = 7.2 Hz, 3H), 3.53 (s, 3H), 3.68 (d, J =7.6 Hz, 2H), 3.75 (s, 3H), 4.17 (q, J = 7.2 Hz, 2H), 5.38 (t, J = 7.2 Hz, 1H), 7.44 (t, J = 6.8 Hz, 2H), 7.56 (d, J = 8.0 Hz, 2H), 7.67 (t, J = 6.4 Hz, 2H), 7.82 (d, J = 7.6 Hz, 2H).

### Step-4: Ethyl 2-(3,3-bis(2-cyanophenyl)-1,1,1-trifluoropropan-2-yl)-5-methoxy-1-methyl-6-oxo-1,6-dihydropyrimidine-4-carboxylate:

The solution of ethyl 2-(2,2-bis(2-cyanophenyl) ethyl)-5-methoxy-1-methyl-6-oxo-1,6-dihydropyrimidine-4-carboxylate (1.6 g, 3.61 mmol) in THF (16 mL) was cooled to 0°C under nitrogen atmosphere. 1M LiHMDS in THF (5.42 mL, 5.42 mmol) was added dropwise at -78°C and reaction mixture was stirred at -78°C for 30 minutes. Tognis-II reagent (2.47 g, 4.69 mmol) was added portion-wise at -78°C for 5 minutes and stirring was continued at -78°C for 30 minutes. After completion of reaction (confirmed by TLC), 10% aqueous solution of ammonium chloride (50 mL) was added and reaction mixture was extracted with ethyl acetate (2 x 50 mL). The combined organic layer was washed with brine (50 mL), dried over anhydrous sodium sulphate and concentrated under vacuum. The crude product was purified by RP column chromatography using acetonitrile and 0.1% formic acid in water to give pure title compound (0.45 g, 37%).

LCMS: m/z 511.7 [M+H].

¹H NMR (400 MHz, DMSO): δ 1.33 (t, J = 6.8 Hz, 3H), 3.33 (s, 3H), 3.77 (s, 3H), 4.30 (q, J = 6.0 Hz, 2H), 5.56 (m, 1H), 5.87 (d, J = 11.2 Hz, 1H), 7.38 (t, J = 7.6 Hz, 1H), 7.52 (t, J = 6.8 Hz, 1H), 7.68-7.73 (m, 3H), 7.90 (t, J = 6.8 Hz, 2H), 8.05 (d, J = 8.0 Hz, 1H).

### Step-5: 2-(3,3-bis(2-cyanophenyl)-1,1,1-trifluoropropan-2-yl)-N-(isoxazol-4-yl)-5-methoxy-1-methyl-6-oxo-1,6-dihydropyrimidine-4-carboxamide:

A solution of ethyl 2-(3,3-bis(2-cyanophenyl)-1,1,1-trifluoropropan-2-yl)-5-methoxy-1-methyl-6-oxo-1,6-dihydropyrimidine-4-carboxylate (0.45 g, 0.88 mmol), isooxazole amine (0.089 g, 1.05 mmol) in Toluene (4.5 mL) was cooled to 0 °C. Trimethyl aluminum solution (0.9 mL, 2M in Toluene, 1.76 mmol) was added at 0 °C. The reaction mixture was heated and stirred at 80°C for 1 hour under microwave irradiation. After completion of reaction (confirmed by TLC), the reaction mixture was loaded on RP gold column and purified using acetonitrile and 0.1% formic acid in water to give pure title compound (0.09 g, 18%).

LCMS: (method J): RT 3.927 min; m/z 549.0 [M+H].

### Chiral HPLC Method:

The diastereomers of title compound was resolved by Chiral SFC [D1: (CHIRALPAK IC(250*21)mm, 5u; MeOH: IPA (50:50) in Hexanes + 0.1% DEA)] and [D2: (CHIRALPAK IC(250*21)mm, 5u; MeOH: IPA (50:50) in Hexanes + 0.1% DEA)] to furnish the enantiopure compounds.

Isomer-1_LCMS: (first eluting isomer) m/z 549.4 [M+H].

Isomer-2_LCMS: (second eluting isomer) m/z 549.4 [M+H].

Chiral HPLC: FR-1 (Isomer-1): RT=4.07; FR-2 (Isomer-2): RT=4.57

### Step-6: 2-(3,3-bis(2-cyanophenyl)-1,1,1-trifluoropropan-2-yl)-5-hydroxy-N-(isoxazol-4-yl)-1-methyl-6-oxo-1,6-dihydropyrimidine-4-carboxamide:

To a solution of 2-(3,3-bis(2-cyanophenyl)-1,1,1-trifluoropropan-2-yl)-N-(isoxazol-4-yl)-5-methoxy-1-methyl-6-oxo-1,6-dihydropyrimidine-4-carboxamide (0.035 g, 0.063 mmol) in DMF (0.3 mL), lithium bromide (0.055 g, 0.633 mmol) was added at room temperature. The reaction mixture was heated and stirred at 130°C for 1 hour under microwave irradiation. After completion of reaction (confirmed by TLC), the reaction mixture was loaded on RP gold column and purified using acetonitrile and 0.1% formic acid in water to give pure title compound (0.008 g, 26%).

Isomer-1_LCMS: (derived from early eluting isomer of precursor) m/z 535.41 [M+H].

Isomer-2_LCMS: ((derived from late eluting isomer of precursor) m/z 535.41 [M+H].

Isomer-1: ¹H NMR (400 MHz, DMSO-d6): δ 3.69 (s, 3H), 5.60-5.76 (m, 1H), 6.14 (d, J = 11.2 Hz, 1H), 7.42 (t, J = 7.6 Hz, 1H), 7.55 (d, J = 7.2 Hz, 1H), 7.73-7.80 (m, 3H), 7.96 (d, J = 7.6 Hz, 1H), 8.05 (d, J = 8.0 Hz, 1H), 8.31 (d, J = 7.6 Hz, 1H), 8.72 (s, 1H), 9.30 (s, 1H), 9.88 (s, 1H), 11.19 (s, 1H).

Isomer-2: ¹H NMR (400 MHz, DMSO-d6): δ 3.70 (s, 3H), 5.60-5.77 (m, 1H), 6.15 (d, J = 11.2 Hz, 1H), 7.43 (t, J = 7.6 Hz, 1H), 7.56 (d, J = 7.2 Hz, 1H), 7.74-7.81 (m, 3H), 7.97 (d, J = 7.6 Hz, 1H), 8.06 (d, J = 8.0 Hz, 1H), 8.32 (d, J = 8.0 Hz, 1H), 8.73 (s, 1H), 9.31 (s, 1H), 9.91 (s, 1H), 11.20 (s, 1H).

HPLC: FR-1 (Isomer-1, 8 mg): RT = 5.21 (97%); FR-2 (Isomer-2, 8 mg): RT = 5.20 (99%).

### hTREX1 Biochemical Assay

Compound potency was assessed through a fluorescence assay measuring degradation of a custom dsDNA substrate possessing a fluorophore-quencher pair on opposing strands. Degradation of the dsDNA liberates free fluorophore to produce a fluorescent signal. Specifically, 7.5 µL of N-terminally His-Tev tagged full length human TREX1 (expressed in E. coli and purified in house) in reaction buffer (50 mM Tris, 150 mM NaCl, 2 mM DTT, 0.1 mg/mL BSA, 0.01% (w/v) Tween-20, 5 mM MgCl2, pH 7.4) was added to a 384-well Black ProxiPlate Plus (PerkinElmer) which already contained compound (150 nL) at varying concentrations as a 10 point dose-response in DMSO. The plate was incubated at 25°C for 4 hours. Reactions were initiated by adding 7.5 µL of dsDNA substrate (Strand A: 5' TEX615/GCT AGG CAG 3'; Strand B: 5' CTG CCT AGC/IAbRQSp (Integrated DNA Technologies)) in reaction buffer. Final concentrations were 4 pM TREX1, 60 nM dsDNA substrate in reaction buffer with 1.0% DMSO (v/v). After 18 hours at 25°C, reactions were quenched by the addition of 2 µL of 500 mM EDTA. Final concentrations in the quenched reaction were 3.5 pM TREX1, 53 nM DNA and 59 mM EDTA in a volume of 17 µL. After a 5-minute incubation at room temperature, plates were read in an EnVision plate reader (PerkinElmer) measuring fluorescence at 615 nm following excitation w/ 570 nm light. IC50 values were calculated by comparing the measured fluorescence at 615 nm relative to control wells pre-quenched w/ EDTA (100% inhibition) and no inhibitor (0% inhibition) controls as using non-linear least square four parameter fits and either Genedata or GraphPad Prism (GraphPad Software, Inc.).

### mTREX1 Biochemical Assay

Compound potency was assessed through a fluorescence assay measuring degradation of a custom dsDNA substrate possessing a fluorophore-quencher pair on opposing strands. Degradation of the dsDNA liberates free fluorophore to produce a fluorescent signal. Specifically, 7.5 µL of N-terminally His-Tev tagged full length mouse TREX1 (expressed in E. coli and purified in house) in reaction buffer (50 mM Tris, 150 mM NaCl, 2 mM DTT, 0.1 mg/mL BSA, 0.01% (w/v) Tween-20, 5 mM MgCl2, pH 7.4) was added to a 384-well Black ProxiPlate Plus (PerkinElmer) which already contained compound (150 nL) at varying concentrations as a 10 point dose-response in DMSO. The plate was incubated at 25°C for 4 hours. Reactions were initiated by adding 7.5 µL of dsDNA substrate (Strand A: 5' TEX615/GCT AGG CAG 3'; Strand B: 5' CTG CCT AGC/IAbRQSp (Integrated DNA Technologies)) in reaction buffer. Final concentrations were 6 pM TREX1, 60 nM dsDNA substrate in reaction buffer with 1.0% DMSO (v/v). After 18 hours at 25°C, reactions were quenched by the addition of 2 µL of 500 mM EDTA. Final concentrations in the quenched reaction were 5.3 pM TREX1, 53 nM DNA and 59 mM EDTA in a volume of 17 µL. After a 5-minute incubation at room temperature, plates were read in an EnVision plate reader (PerkinElmer) measuring fluorescence at 615 nm following excitation w/ 570 nm light. IC50 values were calculated by comparing the measured fluorescence at 615 nm relative to control wells pre-quenched w/ EDTA (100% inhibition) and no inhibitor (0% inhibition) controls as using non-linear least square four parameter fits and either Genedata or GraphPad Prism (GraphPad Software, Inc.).

### hTREX2 Biochemical Assay

Compound potency was assessed through a fluorescence assay measuring degradation of a custom dsDNA substrate possessing a fluorophore-quencher pair on opposing strands. Degradation of the dsDNA liberates free fluorophore to produce a fluorescent signal. Specifically, 7.5 µL of N-terminally His-Tev tagged human TREX2 (residues M44-A279, expressed in E. coli and purified in house) in reaction buffer (50 mM Tris, 150 mM NaCl, 2 mM DTT, 0.1 mg/mL BSA, 0.01% (w/v) Tween-20, 5 mM MgCl2, pH 7.4) was added to a 384-well Black ProxiPlate Plus (PerkinElmer) which already contained compound (150 nL) at varying concentrations as a 10 point dose-response in DMSO. The plate was incubated at 25°C for 4 hours. Reactions were initiated by adding 7.5 µL of dsDNA substrate (Strand A: 5' TEX615/GCT AGG CAG 3'; Strand B: 5' CTG CCT AGC/IAbRQSp (IDT)) in reaction buffer. Final concentrations were 50 pM TREX2, 60 nM dsDNA substrate in reaction buffer with 1.0% DMSO (v/v). After 18 hours at 25°C, reactions were quenched by the addition of 2 µL of 500 mM EDTA. Final concentrations in the quenched reaction mixture were 44 pM TREX2, 53 nM DNA and 59 mM EDTA in a volume of 17 µL. After a 5-minute incubation at room temperature, plates were read in an EnVision plate reader (PerkinElmer) measuring fluorescence at 615 nm following excitation w/ 570 nm light. IC50 values were calculated by comparing the measured fluorescence at 615 nm relative to control wells pre-quenched w/ stop buffer (100% inhibition) and no inhibitor (0% inhibition) controls as using non-linear least square four parameter fits and either Genedata or GraphPad Prism (GraphPad Software, Inc.).

**Table 7. hTREX1 and mTREX1 biochemical IC50 data, and biochemical selectivity for hTREX1 vs hTREX2.**

| hTREX1 IC₅₀ : A = <0.001 µM; B = 0.001 to 0.01 µM; C = 0.01 to 0.1 µM; D = >0.1 µM. mTREX1 IC₅₀ : A = <0.001 µM; B = 0.001 to 0.01 µM; C = >0.01 µM. hTREX1 vs hTREX2 selectivity : A = <25 fold selective for hTREX1, B = 25 to 50 fold selective for hTREX1, C = >50 fold selective for hTREX1 | | | |
|---|---|---|---|
| | **hTREX1 IC50** | **mTREX1 IC50** | **hTREX1 vs hTREX2 selectivity** |
| Example A* | D | C | _{A} |
| Example B* | D | C | A |
| Example 1 Isomer 1* | D | C | A |
| Example 1 Isomer 2* | B | C | A |
| Example 2 Isomer 1 | B | C | A |
| Example 2 Isomer 2 | C | C | A |
| Example 2 Isomer 3 | A | B | B |
| Example 2 Isomer 4 | D | C | A |
| Example 3 Isomer 1 | B | C | B |
| Example 3 Isomer 2 | D | C | A |
| Example 3 Isomer 3 | D | C | A |
| Example 3 Isomer 4 | A | B | B |
| Example 4 Isomer 1 | B | C | C |
| Example 4 Isomer 2 | A | A | C |
| Example 5 Isomer 1 | A | B | B |
| Example 5 Isomer 2 | B | C | |
| Example 6 Isomer 1 | B | C | C |
| Example 6 Isomer 2 | D | C | |
| Example 6 Isomer 3 | D | C | A |
| Example 6 Isomer 4 | A | B | B |
| Example 7 Isomer 1 | A | C | C |
| Example 7 Isomer 2 | D | C | A |
| Example 7 Isomer 3 | D | C | |
| Example 7 Isomer 4 | A | B | B |
| Example 8 Isomer 1 | D | C | |
| Example 8 Isomer 2 | A | B | C |
| Example 8 Isomer 3 | B | C | C |
| Example 8 Isomer 4 | C | C | B |
| Example 9 Isomer 1 | B | C | C |
| Example 9 Isomer 2 | D | C | B |
| Example 9 Isomer 3 | D | C | |
| Example 9 Isomer 4 | A | A | B |
| Example 10 Isomer 1 | A | A | C |
| Example 10 Isomer 2 | C | C | B |
| Example 10 Isomer 3 | A | B | C |
| Example 10 Isomer 4 | A | B | C |
| Example 11 Isomer 1 | D | C | |
| Example 11 Isomer 2 | B | C | C |
| Example 11 Isomer 3 | A | A | B |
| Example 11 Isomer 4 | B | B | A |
| Example 12 Isomer 1 | B | C | C |
| Example 12 Isomer 2 | D | C | |
| Example 12 Isomer 3 | A | A | B |
| Example 12 Isomer 4 | C | C | A |
| Example 13 Isomer 1 | A | A | C |
| Example 13 Isomer 2 | A | B | C |
| Example 13 Isomer 3 | C | C | B |
| Example 13 Isomer 4 | D | C | |
| Example 14 Isomer 1 | D | C | A |
| Example 14 Isomer 2 | B | C | C |
| Example 14 Isomer 3 | D | C | |
| Example 14 Isomer 4 | B | C | B |
| Example 15 Isomer 1 | A | A | C |
| Example 15 Isomer 2 | D | C | A |
| Example 16 Isomer 1 | B | C | |
| Example 16 Isomer 2 | D | C | |
| Example 16 Isomer 3 | D | C | A |
| Example 16 Isomer 4 | A | A | B |
| Example 17 Isomer 1 | C | C | A |
| Example 17 Isomer 2 | D | C | |
| Example 17 Isomer 3 | A | B | B |
| Example 17 Isomer 4 | C | C | |
| Example 18 Isomer 1* | C | C | A |
| Example 18 Isomer 2* | D | C | |
| Example 19* | C | C | B |
| Example 20 Isomer 1* | C | C | A |
| Example 20 Isomer 2* | D | C | A |
| Example 21 Isomer 1 | A | B | C |
| Example 21 Isomer 2 | C | C | C |

| | | | |
|---|---|---|---|
| *The * indicates that the example is a reference example* | | | |

### hTREX1 HCT116 Cell Assay

HCT116 dual cells (Invivogen, San Diego, CA, USA) are derived from the human HCT116 colorectal carcinoma cell line. Cells have been selected for the stable integration of SEAP and Luciferase reporter genes, which expression is under the control of 5 tandem response elements for NF-KB/AP1 and STAT1/STAT2, respectively. The cell line was used to monitor Type I interferon induction and subsequent signaling by measuring the activity of the Lucia luciferase secreted in the culture medium.

HCT116 cells were plated in 96-well plate(s) at 40,000 cells/well in 100uL DMEM supplemented with 10% FBS and 25mM Hepes (pH 7.2 - 7.5). After overnight settling, cells were treated with TREX1i for 4h (maximum DMSO fraction was 0.1%) before 1ug/mL pBR322/BstNI restriction digest (New England Biolabs, Ipswich, MA, USA) was transfected with Lipofectamine LTX (ThermoFisher, Grand Island, NY, USA), according to product manual recommendations. Briefly, Lipofectamine LTX (0.35uL/well) was diluted in OptiMEM (5uL/well). pBR322/BstNI (100ng/well) was diluted in OptiMEM (5uL/well) before Plus reagent (0.1uL/100ng DNA) was added. After 5min incubation at room temperature, the DNA mixture was mixed dropwise with the diluted Lipofectamine LTX. After an additional 10 min incubation, the transfection mix (10uL/well) was added to the cells. Cells were maintained at 37C for 48h before monitoring the Lucia Luciferase activity from the cell culture medium.

**Table 8. hTREX1 HCT116 Cell Assay EC50 data.**

| EC₅₀ : A = <0.01 µM; B = 0.01 to 0.1 µM; C = >0.1 µM. | | | | |
|---|---|---|---|---|
| | hTREX1 HCT116 IC50 | | | hTREX1 HCT116 IC50 |
| Example 1 Isomer 1* | C | | Example 9 Isomer 4 | B |
| Example 1 Isomer 2* | C | | Example 10 Isomer 1 | B |
| Example 2 Isomer 1 | C | | Example 10 Isomer 2 | C |
| Example 2 Isomer 2 | C | | Example 10 Isomer 3 | C |
| Example 2 Isomer 3 | B | | Example 10 Isomer 4 | B |
| Example 3 Isomer 1 | B | | Example 11 Isomer 2 | C |
| Example 3 Isomer 3 | C | | Example 11 Isomer 3 | A |
| Example 3 Isomer 4 | B | | Example 11 Isomer 4 | C |
| Example 4 Isomer 1 | C | | Example 12 Isomer 1 | C |
| Example 4 Isomer 2 | A | | Example 12 Isomer 3 | A |
| Example 5 Isomer 1 | A | | Example 12 Isomer 4 | C |
| Example 5 Isomer 2 | C | | Example 13 Isomer 1 | B |
| Example 6 Isomer 1 | C | | Example 13 Isomer 2 | B |
| Example 6 Isomer 3 | C | | Example 13 Isomer 3 | C |
| Example 6 Isomer 4 | B | | Example 14 Isomer 2 | C |
| Example 7 Isomer 1 | C | | Example 14 Isomer 4 | C |
| Example 7 Isomer 4 | B | | Example 15 Isomer 1 | B |
| Example 8 Isomer 2 | B | | Example 16 Isomer 1 | C |
| Example 8 Isomer 3 | C | | Example 16 Isomer 4 | B |
| Example 8 Isomer 4 | C | | Example 17 Isomer 3 | B |
| Example 9 Isomer 1 | C | | Example 20 Isomer 1* | C |
| Example 9 Isomer 2 | C | | Example 21 Isomer 1 | A |

| | | | | |
|---|---|---|---|---|
| *The * indicates that the example is a reference example* | | | | |

### TREX1 kinetics assays

Compound binding kinetics were assessed using a pair of TR-FRET assays which measure the proportion of protein bound to a biotinylated TREX1 inhibitor ("probe").

### Association

N-terminally His-Tev tagged full length human TREX1 (expressed in E. coli and purified in house), complexed with Eu-W1024-anti-6xHis ("Eu"; PerkinElmer) in reaction buffer (50 mM Tris, 150 mM NaCl, 2 mM DTT, 0.1 mg/mL BSA, 0.01% (w/v) Tween-20, 5 mM MgCl2, pH 7.4) was combined with an equal volume of test compound in reaction buffer and incubated at 25°C. Concentrations at this stage were 1 nM TREX1/Eu complex, and four concentrations of compound (diluted from 10 mM stocks in 100% DMSO). At defined time points, 18 µL of this mixture was withdrawn and combined with 2 µL probe to a final concentration of 1 µM probe. After incubating for 30 seconds, 18 µL was withdrawn and combined with 2 µL streptavidin-allophycocyanin ("SA-APC"; PerkinElmer) to a final concentration of 1.5 µM SA-APC. Fifteen µL of this mixture was then immediately transferred to a 384-well Black ProxiPlate Plus (PerkinElmer) and read in an EnVision plate reader (PerkinElmer) measuring fluorescence at 615 nm and 665 nm following excitation w/ 337 nm laser light. Final concentrations were 0.8 nM TREX1/Eu complex, 0.9 µM probe, and 1.5 µM SA-APC.

### Dissociation

N-terminally His-Tev tagged full length human TREX1 (expressed in E. coli and purified in house), complexed with Eu-W1024-anti-6xHis ("Eu"; PerkinElmer) in reaction buffer (50 mM Tris, 150 mM NaCl, 2 mM DTT, 0.1 mg/mL BSA, 0.01% (w/v) Tween-20, 5 mM MgCl2, pH 7.4) was combined with an equal volume of test compound in reaction buffer. Concentrations at this stage were 100 nM TREX1/Eu complex and 100 nM test compound (diluted from 10 mM stocks in 100% DMSO). Following an equilibration period of at least an hour at 25°C, this mixture was diluted 100-fold into reaction buffer containing 1 µM probe, and incubated at 25°C. At defined time points, 36 µL of the reaction mixture was withdrawn and combined with 4 µL streptavidin-allophycocyanin ("SA-APC"; PerkinElmer) to a final concentration of 1.5 µM SA-APC. Fifteen µL of this mixture was then immediately transferred to duplicate wells of a 384-well Black ProxiPlate Plus (PerkinElmer) and read in an EnVision plate reader (PerkinElmer) measuring fluorescence at 615 nm and 665 nm following excitation w/ 337 nm laser light.

### Data analysis

The TR-FRET signal, a ratio of 665 nm/615 nm emitted light, was converted to fraction enzyme bound to test compound by normalizing to low signal (no enzyme or test compound) and high signal (no test compound) controls. Data from both association and dissociation experiments were fitted globally using Kintek Explorer software, which computes the rate constants directly.

### Kintek Explorer citations:

Johnson, K. A., Simpson, Z. B., and Blom, T. (2009) Global Kinetic Explorer: A new computer program for dynamic simulation and fitting of kinetic data. Analytical Biochemistry 387, 20-29. http://dx.doi.org/10.1016/j.ab.2008.12.024

Johnson, K. A., Simpson, Z. B., and Blom, T. (2009) FitSpace Explorer: An algorithm to evaluate multi-dimensional parameter space in fitting kinetic data. Analytical Biochemistry 387,30-41. http://dx.doi.org/10.1016/j.ab.2008.12.025

**Table 9. Kinetic data for hTREX1.**

| | **kon (nM⁻¹ mi**n⁻**¹)** | **koff (min⁻¹)** | **KD (nM)** | **residence time (mins)** |
|---|---|---|---|---|
| Example 1 Isomer 2* | 0.00302 | 0.01150 | 3.8 | 87 |
| Example 2 Isomer 3 | 0.00954 | 0.00329 | 0.345 | 304 |
| Example 3 Isomer 4 | 0.00985 | 0.00216 | 0.219 | 463 |
| Example 4 Isomer 2 | 0.05440 | 0.00230 | 0.042 | 435 |
| Example 6 Isomer 4 | 0.01040 | 0.00202 | 0.194 | 495 |
| Example 8 Isomer 2 | 0.00693 | 0.00250 | 0.361 | 400 |
| Example 9 Isomer 4 | 0.01070 | 0.00124 | 0.116 | 806 |
| Example 10 Isomer 1 | 0.02690 | 0.00189 | 0.070 | 529 |
| Example 11 | 0.01540 | 0.00115 | 0.075 | 870 |
| Isomer 3 | | | | |
| Example 15 Isomer 1 | 0.01560 | 0.00286 | 0.183 | 350 |

While we have described a number of embodiments, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of example.

Unless otherwise defined, all technical and scientific terms used herein are accorded the meaning commonly known to one with ordinary skill in the art.

## Claims

1. A compound having the Formula I: 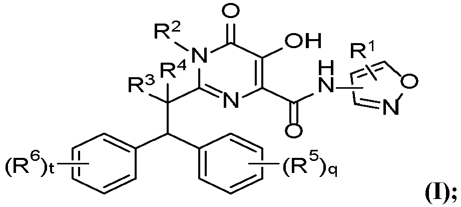 or a pharmaceutically acceptable salt thereof, wherein:
R¹ is halo, hydrogen, (C₁-C₄)alkyl, or halo(C₁-C₄)alkyl;
R² is hydrogen, (C₁-C₄)alkyl, halo(C₁-C₄)alkyl, -(C₁-C₄)alkylOR^{a}, -(C₁-C₄)alkylSR^{a}, - (C₁-C₄)alkylNR^{a}R^{b},
R^{a} and R^{d} are each independently selected from hydrogen, (C₁-C₄)alkyl, halo(C₁-C₄)alkyl, COOR^{e}, and -C(O)NR^{c}R^{d};
R³ and R⁴ are each independently hydrogen, halo, (C₁-C₄)alkyl, or halo(C₁-C₄)alkyl, wherein when R³ is halo then R⁴ is halo and wherein when R³ is hydrogen then R⁴ is (C₁-C₄)alkyl or halo(C₁-C₄)alkyl;
R⁵ and R⁶ are each independently (C₁-C₄)alkyl, halo(C₁-C₄)alkyl, halo, cyano, or - C(O)NR^{c}R^{d};
R^{c} and R^{d} are each independently hydrogen or (C₁-C₄)alkyl;
t is 1, 2, or 3; and
q is 0, 1, 2, or 3.

2. The compound of Claim 1, wherein the compound is of the Formula **II**: 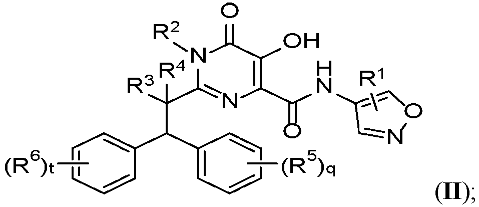 or a pharmaceutically acceptable salt thereof.

3. The compound of Claim 1 or 2, or a pharmaceutically acceptable salt thereof, wherein R¹ is H.

4. The compound of any one of Claims 1 to 3, or a pharmaceutically acceptable salt thereof, wherein R² is (C₁-C₄)alkyl.

5. The compound of any one of Claims 1 to 4, or a pharmaceutically acceptable salt thereof, wherein R³ is halo.

6. The compound of any one of Claims 1 to 4, or a pharmaceutically acceptable salt thereof, wherein R³ is hydrogen.

7. The compound of any one of Claims 1 to 6, or a pharmaceutically acceptable salt thereof, wherein R⁴ is halo.

8. The compound of any one of Claims 1 to 6, or a pharmaceutically acceptable salt thereof, wherein R⁴ is (C₁-C₄)alkyl or halo(C₁-C₄)alkyl.

9. The compound of any one of Claims 1 to 6, or a pharmaceutically acceptable salt thereof, wherein R⁴ is (C₁-C₄)alkyl.

10. The compound of any one of Claims 1 to 9, or a pharmaceutically acceptable salt thereof, wherein R⁵ is halo, cyano, or -C(O)NR^{a}R^{b}.

11. The compound of any one of Claims 1 to 10, or a pharmaceutically acceptable salt thereof, wherein R⁶ is halo, cyano, or -C(O)NR^{a}R^{b}.

12. The compound of any one of Claims 1 to 11, or a pharmaceutically acceptable salt thereof, wherein R⁶ is halo or cyano.

13. The compound of Claim 1, wherein the compound is selected from: 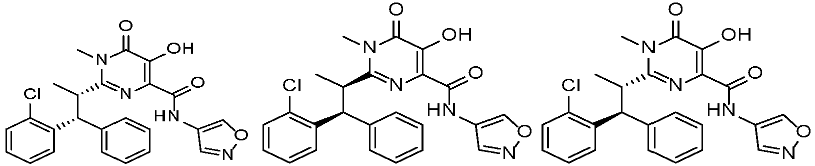 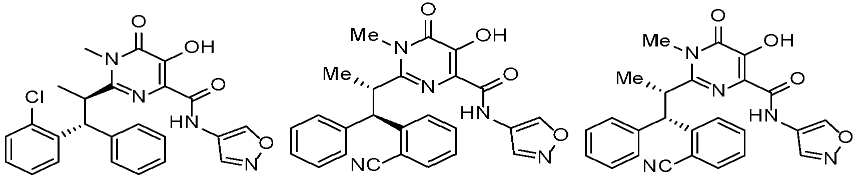 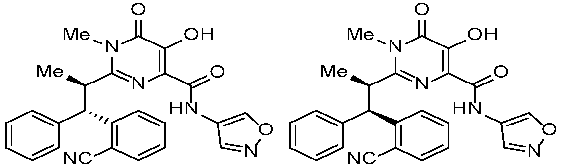 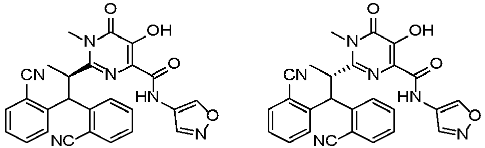 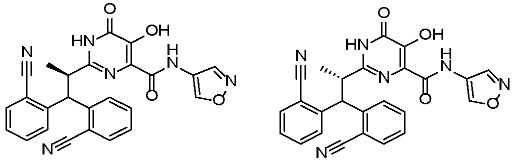 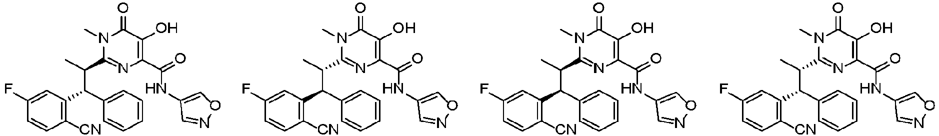 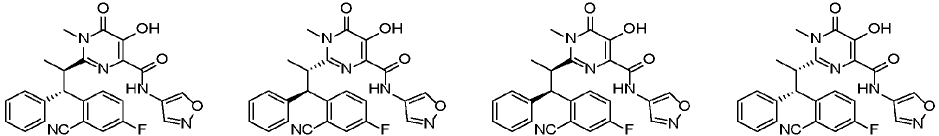 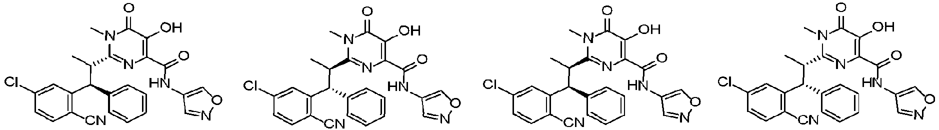 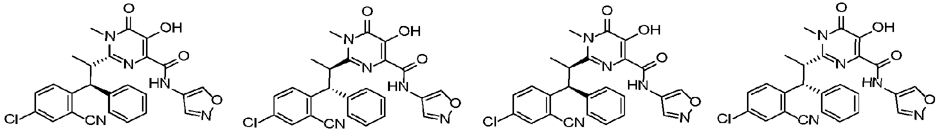 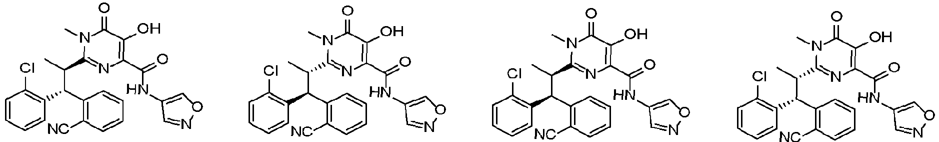 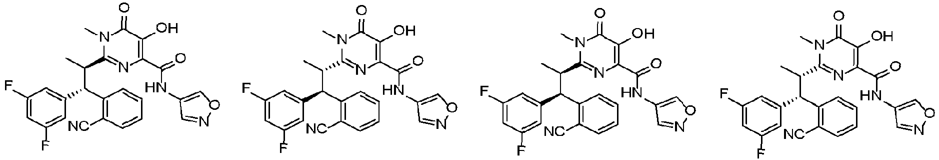 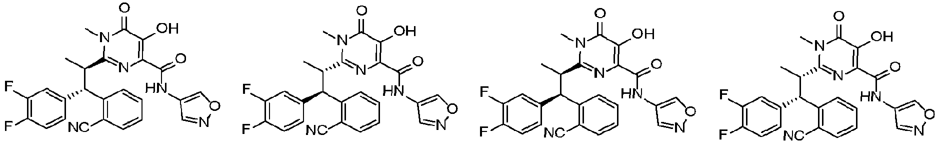 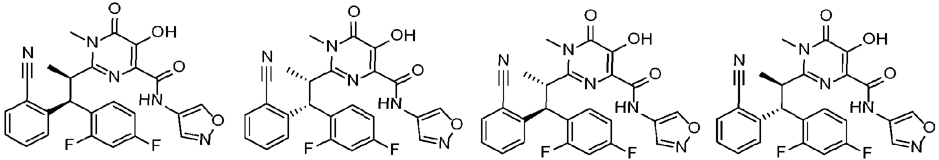 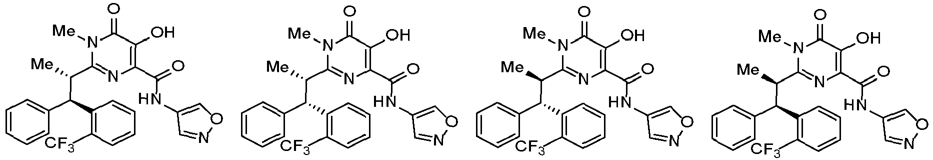 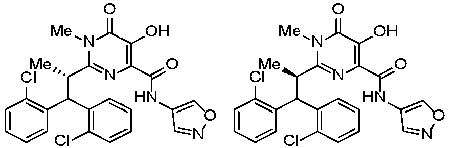 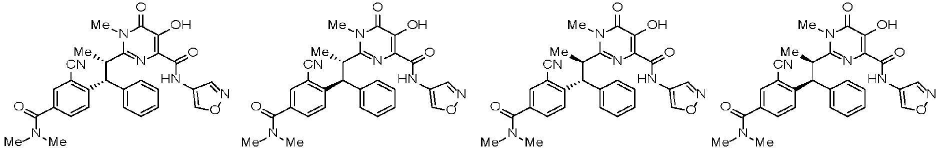 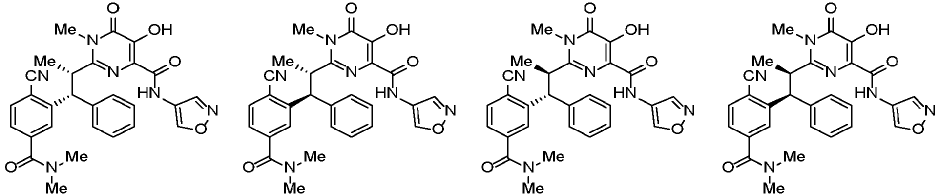 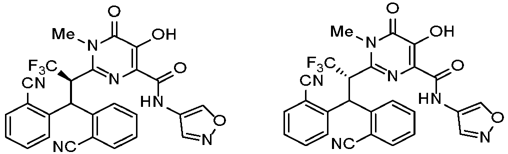 or a pharmaceutically acceptable salt of any one of the foregoing.

14. A pharmaceutical composition comprising the compound of any one of Claims 1 to 13, or a pharmaceutically acceptable salt thereof; and a pharmaceutically acceptable carrier.

15. A compound of any one of Claims 1-13, or a pharmaceutically acceptable salt thereof, or the composition according to Claim 14, for use in treating cancer.

## Patentansprüche

1. Verbindung, aufweisend die Formel I: 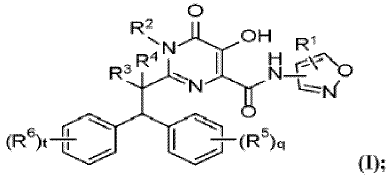 oder ein pharmazeutisch unbedenkliches Salz davon, wobei:
R¹ Halogen, Wasserstoff, (C₁-C₄)-Alkyl oder Halogen (C₁-C₄)-Alkyl ist;
R² Wasserstoff, (C₁-C₄)Alkyl, Halogen- (C₁-C₄)-Alkyl, -(C₁-C₄)-AlkylOR^{a}, - (C₁-C₄)-AlkylSR^{a}, - (C₁-C₄)-AlkylNR^{a}R^{b} ist,
R^{a} und R^{b} jeweils unabhängig voneinander ausgewählt sind aus Wasserstoff, (C₁-C₄)-Alkyl, Halogen- (C₁-C₄)-Alkyl, COOR^{c} und -C(O)NR^{c}R^{d};
R³ und R⁴ jeweils unabhängig voneinander Wasserstoff, Halogen, (C₁-C₄)-Alkyl oder Halogen- (C₁-C₄)-Alkyl sind, wobei, wenn R³ Halogen ist, R⁴ Halogen ist, und wobei, wenn R³ Wasserstoff ist, R⁴ (C₁-C₄)-Alkyl oder Halogen-(C₁-C₄)-Alkyl ist;
R⁵ und R⁶ jeweils unabhängig voneinander (C₁-C₄)-Alkyl, Halogen- (C₁-C₄)-Alkyl, Halogen, Cyano oder -C(O)NR^{c}R^{d} sind;
R^{c} und R^{d} jeweils unabhängig voneinander Wasserstoff oder (C₁-C₄)-Alkyl sind;
t 1, 2 oder 3 ist; und
q 0, 1, 2 oder 3 ist.

2. Verbindung nach Anspruch 1, wobei die Verbindung die Formel **II** aufweist: 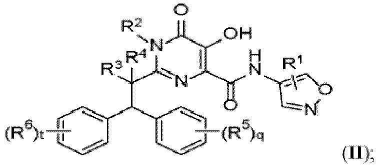 oder ein pharmazeutisch unbedenkliches Salz davon.

3. Verbindung nach Anspruch 1 oder 2 oder ein pharmazeutisch unbedenkliches Salz davon, wobei R¹ H ist.

4. Verbindung nach einem der Ansprüche 1 bis 3 oder ein pharmazeutisch unbedenkliches Salz davon, wobei R² (C₁-C₄)-Alkyl ist.

5. Verbindung nach einem der Ansprüche 1 bis 4 oder ein pharmazeutisch unbedenkliches Salz davon, wobei R³ Halogen ist.

6. Verbindung nach einem der Ansprüche 1 bis 4 oder ein pharmazeutisch unbedenkliches Salz davon, wobei R³ Wasserstoff ist.

7. Verbindung nach einem der Ansprüche 1 bis 6 oder ein pharmazeutisch unbedenkliches Salz davon, wobei R⁴ Halogen ist.

8. Verbindung nach einem der Ansprüche 1 bis 6 oder ein pharmazeutisch unbedenkliches Salz davon, wobei R⁴ (C₁-C₄)-Alkyl oder Halogen- (C₁-C₄)-Alkyl ist.

9. Verbindung nach einem der Ansprüche 1 bis 6 oder ein pharmazeutisch unbedenkliches Salz davon, wobei R⁴ (C₁-C₄)-Alkyl ist.

10. Verbindung nach einem der Ansprüche 1 bis 9 oder ein pharmazeutisch unbedenkliches Salz davon, wobei R⁵ Halogen, Cyano oder -C(O)NR^{a}R^{b} ist.

11. Verbindung nach einem der Ansprüche 1 bis 10 oder ein pharmazeutisch unbedenkliches Salz davon, wobei R⁶ Halogen, Cyano oder -C(O)NR^{a}R^{b} ist.

12. Verbindung nach einem der Ansprüche 1 bis 11 oder ein pharmazeutisch unbedenkliches Salz davon, wobei R⁶ Halogen oder Cyano ist.

13. Verbindung nach Anspruch 1, wobei die Verbindung ausgewählt ist aus: 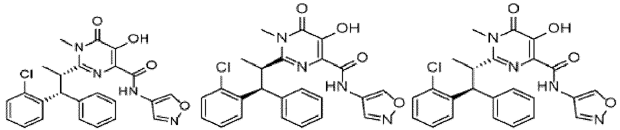 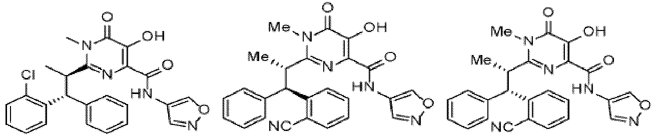 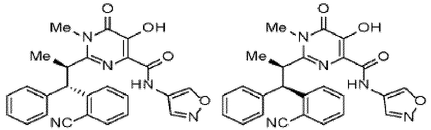 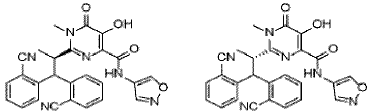 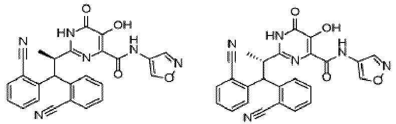 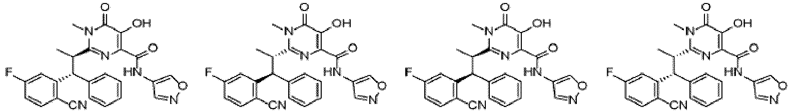 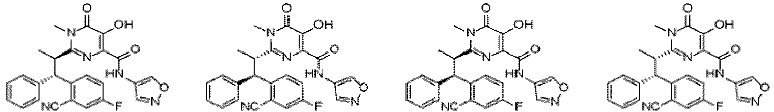 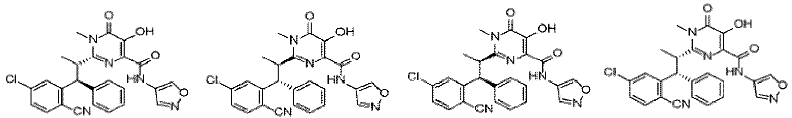 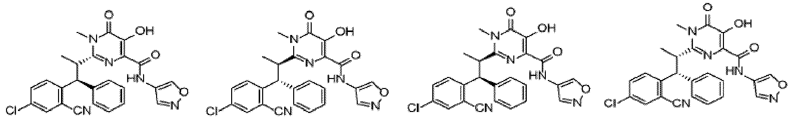 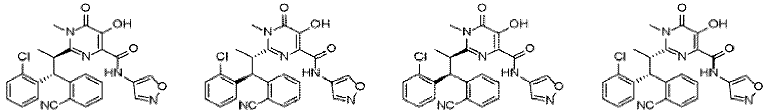 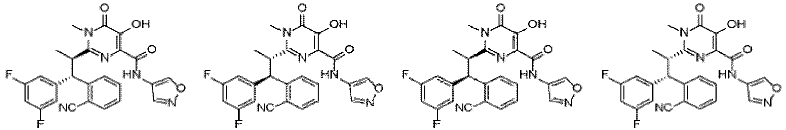 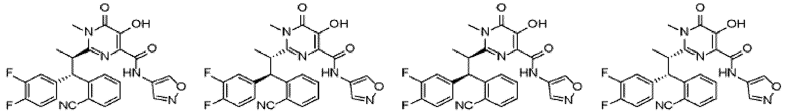 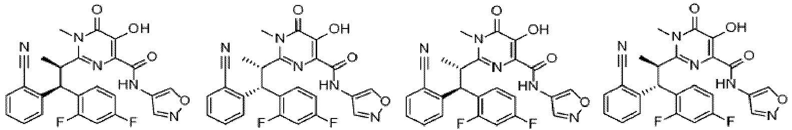 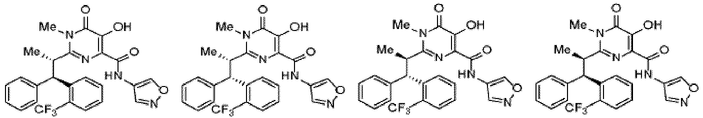 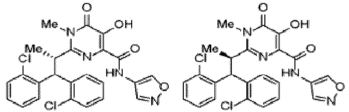 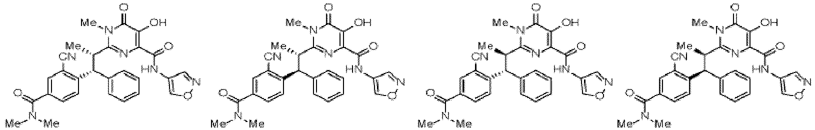 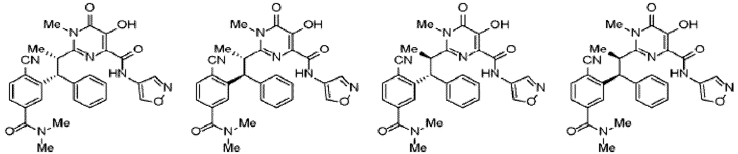 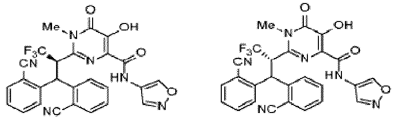 oder einem pharmazeutisch unbedenklichen Salz eines der Vorgenannten.

14. Pharmazeutische Zusammensetzung, umfassend die Verbindung nach einem der Ansprüche 1 bis 13 oder ein pharmazeutisch unbedenkliches Salz davon und einen pharmazeutisch unbedenklichen Träger.

15. Verbindung nach einem der Ansprüche 1 bis 13 oder ein pharmazeutisch unbedenkliches Salz davon oder die Zusammensetzung nach Anspruch 14 zur Verwendung bei der Behandlung von Krebs.

## Revendications

1. Composé ayant la formule I : 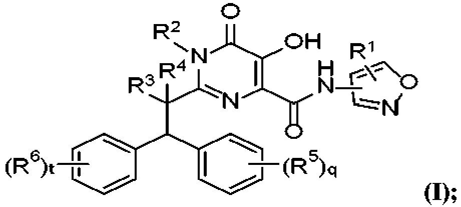 ou un sel pharmaceutiquement acceptable de celui-ci, dans lequel :
R¹ est halogéno, hydrogène, alkyle en C₁-C₄ ou halogénoalkyle en C₁-C₄ ;
R² est hydrogène, alkyle en C₁-C₄, halogénoalkyle en C₁-C₄, -(alkyl en C₁-C₄)OR³, -(alkyl en C₁-C₄) SR^{a}, -(alkyl en C₁-C₄)NR^{a}R^{b},
R^{a} et R^{b} sont chacun indépendamment choisis parmi hydrogène, alkyle en C₁-C₄, halogénoalkyle en C₁-C₄, COOR^{c} et -C(O)NR^{c}R^{d} ;
R³ et R⁴ sont chacun indépendamment hydrogène, halogéno, alkyle en C₁-C₄ ou halogénoalkyle en C₁-C₄, où lorsque R³ est halogéno alors R⁴ est halogéno et où lorsque R³ est hydrogène alors R⁴ est alkyle en C₁-C₄ ou un halogénoalkyle en C₁-C₄ ;
R⁵ et R⁶ sont chacun indépendamment alkyle en C₁-C₄, haloalkyle en C₁-C₄, halogéno, cyano ou -C(O)NR^{c}R^{d} ;
R^{c} et R^{d} sont chacun indépendamment hydrogène ou alkyle en C₁-C₄ ;
t est 1, 2 ou 3 ; et
q est O, 1, 2 ou 3.

2. Composé selon la revendication 1, dans lequel le composé répond à la formule II : 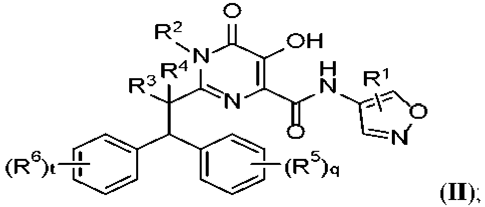 ou un sel pharmaceutiquement acceptable de celui-ci.

3. Composé selon la revendication 1 ou 2, ou sel pharmaceutiquement acceptable de celui-ci, dans lequel R¹ est H.

4. Composé selon l'une quelconque des revendications 1 à 3, ou sel pharmaceutiquement acceptable de celui-ci, dans lequel R² est alkyle en C₁-C₄.

5. Composé selon l'une quelconque des revendications 1 à 4, ou sel pharmaceutiquement acceptable de celui-ci, dans lequel R³ est halogéno.

6. Composé selon l'une quelconque des revendications 1 à 4, ou sel pharmaceutiquement acceptable de celui-ci, dans lequel R³ est hydrogène.

7. Composé selon l'une quelconque des revendications 1 à 6, ou sel pharmaceutiquement acceptable de celui-ci, dans lequel R⁴ est halogéno.

8. Composé selon l'une quelconque des revendications 1 à 6, ou sel pharmaceutiquement acceptable de celui-ci, dans lequel R⁴ est alkyle en C₁-C₄ ou halogénoalkyle en C₁-C₄.

9. Composé selon l'une quelconque des revendications 1 à 6, ou sel pharmaceutiquement acceptable de celui-ci, dans lequel R⁴ est alkyle en C₁-C₄.

10. Composé selon l'une quelconque des revendications 1 à 9, ou sel pharmaceutiquement acceptable de celui-ci, dans lequel R⁵ est halogéno, cyano ou -C(O)NR^{a}R^{b}.

11. Composé selon l'une quelconque des revendications 1 à 10, ou sel pharmaceutiquement acceptable de celui-ci, dans lequel R⁶ est halogéno, cyano ou -C(O)NR^{a}R^{b}.

12. Composé selon l'une quelconque des revendications 1 à 11, ou sel pharmaceutiquement acceptable de celui-ci, dans lequel R⁶ est halogéno ou cyano.

13. Composé selon la revendication 1, dans lequel le composé est choisi parmi : 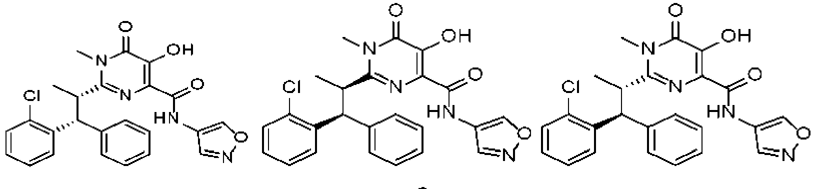 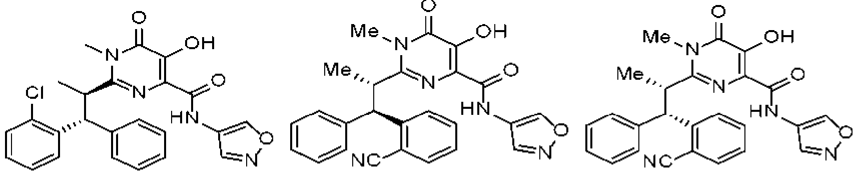 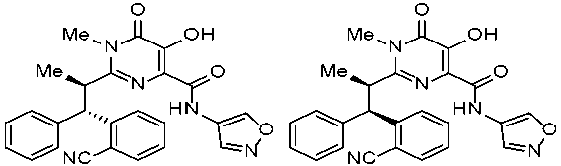 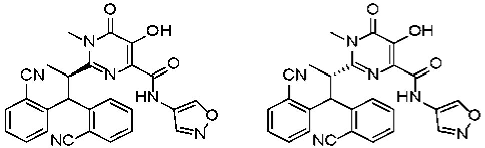 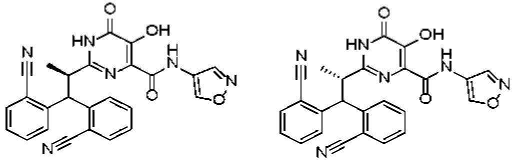 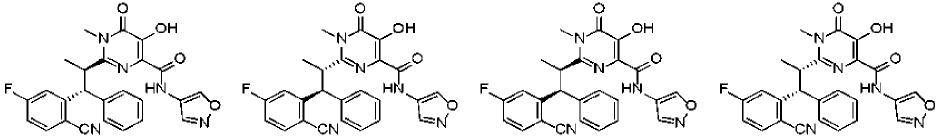 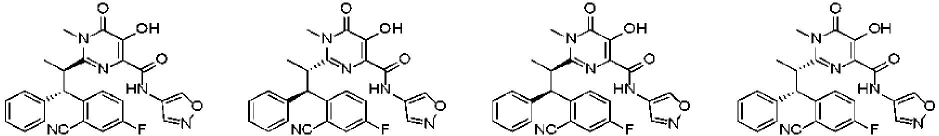 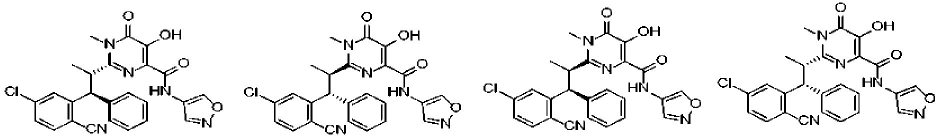 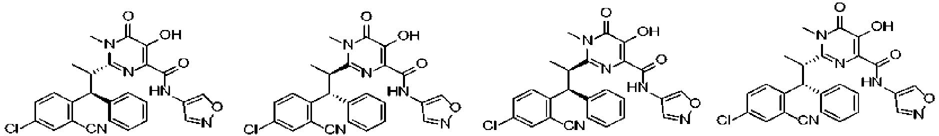 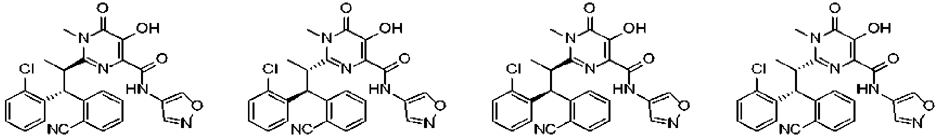 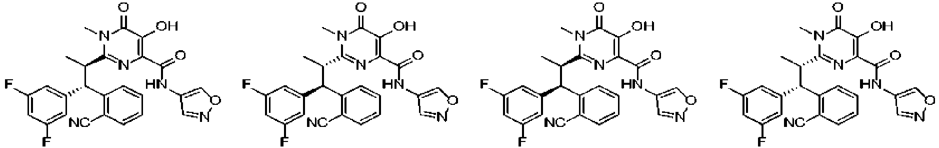 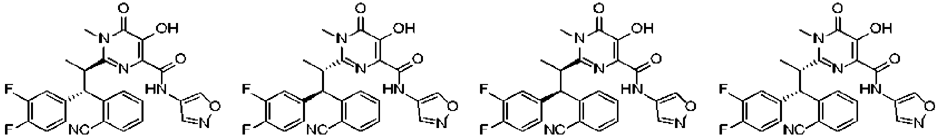 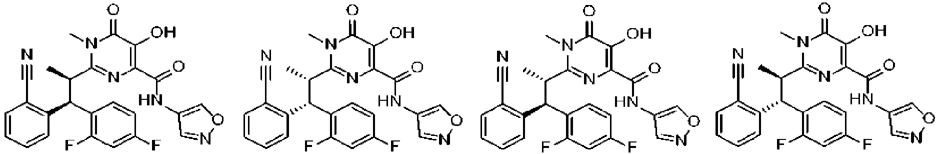 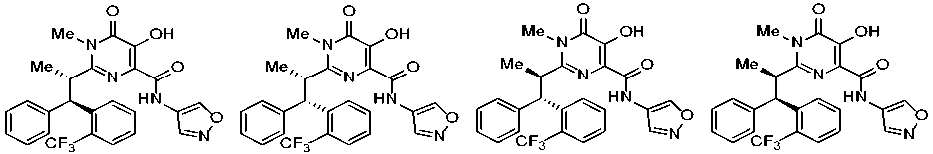 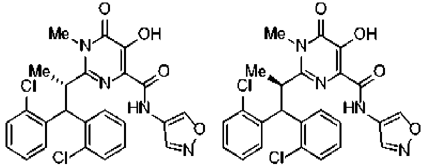 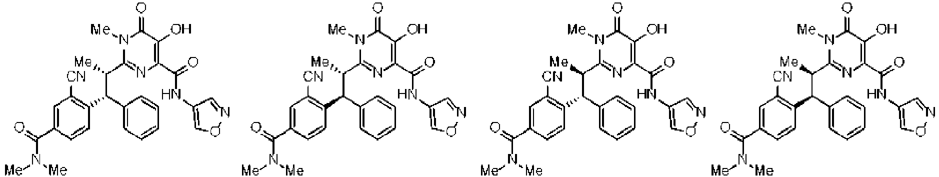 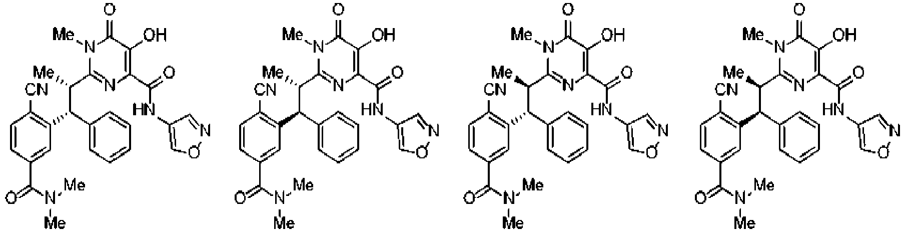 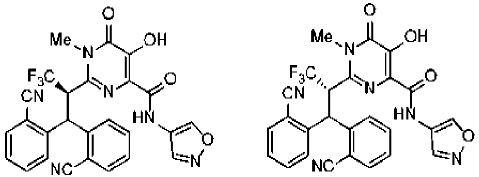 ou un sel pharmaceutiquement acceptable de l'un quelconque des composés précédents.

14. Composition pharmaceutique comprenant le composé selon l'une quelconque des revendications 1 à 13, ou un sel pharmaceutiquement acceptable de celui-ci ; et un support pharmaceutiquement acceptable.

15. Composé selon l'une quelconque des revendications 1 à 13, ou sel pharmaceutiquement acceptable de celui-ci, ou composition selon la revendication 14, destiné à être utilisé dans le traitement du cancer.
